# FASCICULE DE BREVET EUROPEEN

(11) **EP 0 770 132 B1**
(45) Date de publication et mention de la délivrance du brevet: **08.09.2010**
(21) Numéro de dépôt: 95924396.5
(22) Date de dépôt: 04.07.1995
(51) Int. Cl.: C12N 15/52, C12N 15/76, C07K 7/06, C12N 1/21, A61K 38/08, C07C 229/36, C07C 229/42, C07C 321/28, C07D 295/135

(54) **NOUVELLES STREPTOGRAMINES**
NEUE STREPTOGRAMINE
NEW STREPTOGRAMINES

(30) Priorité: 08.07.1994 FR 9408478
(43) Date de publication de la demande: 02.05.1997
(60) Demande divisionnaire: 10154211.6
(73) Titulaire: Aventis Pharma S.A., 92160 Antony (FR)
(72) Inventeur: BLANC, Véronique, F-75012 Paris (FR); THIBAUT, Denis, F-75013 Paris (FR); BAMAS-JACQUES, Nathalie, F-75015 Paris (FR); BLANCHE, Francis, F-75019 Paris (FR); CROUZET, Joel, F-92330 Sceaux (FR); BARRIERE, Jean-Claude, F-91440 Bures-sur-Yvette (FR); DEBUSSCHE, Laurent, F-91200 Athis-Mons (FR); FAMECHON, Alain, F-91510 Janville-sur-Juine (FR); PARIS, Jean-Marc, F-77360 Vaires-sur-Marne (FR); DUTRUC-ROSSET, Gilles, F-75012 Paris (FR)
(74) Mandataire: Becker, Philippe
(86) Numéro de dépôt international: PCT/FR1995/000889
(87) Numéro de publication internationale: WO 1996/001901

(56) Documents cités:
- EP-A- 0 133 096
- WO-A-93/20182
- WO-A-94/08014
- US-A- 4 156 734
- J. AM. CHEM. SOC. (1990), 112(13), 5212-19 CODEN: JACSAT;ISSN: 0002-7863, BRESLOW, RONALD ET AL 'Artificial transaminases linking pyridoxamine to binding cavities: controlling the geometry'
- HELV. CHIM. ACTA (1974), 57(8), 2406-20 CODEN: HCACAV, ZELLER, E. ALBERT ET AL 'Ophidian L-amino acid oxidases. 6. Interaction of ophidian L-amino acid oxidase with its substrates and inhibitors. Role of molecular geometry and electron distribution'

## Description

La présente invention se rapporte principalement à de nouveaux composés apparentés aux streptogramines du groupe B.

Les streptogramines forment un groupe homogène d'antibiotiques constitués d'une association de deux types de molécules chimiquement différentes; d'une part des macrolactones polyinsaturées (composants du groupe A), et d'autre part des depsipeptides (composants du groupe B). Ce groupe comprend de nombreux antibiotiques connus sous différents noms en fonction de leur origine, dont les pristinamycines, les mikamycines, les virginiamycines (Cocito 1979,1983).

Les composants A et B ont une activité antibactérienne synergique qui peut atteindre 100 fois celle des composants séparés et qui, contrairement à celle de chaque composant, est bactéricide (Cocito 1979). Cette activité est plus particulièrement efficace contre les bactéries Gram-positifs, comme les staphylocoques et les streptocoques (Cocito 1979, Videau 1982). Les composants A et B inhibent la synthèse protéique en se fixant à la sous-unité 50S du ribosome (Cocito 1979 ; Di Giambattista et al. 1989).

La connaissance des voies de biosynthèse de chacun des composants reste partielle à ce jour, bien que des études, précédentes, présentées dans la demande de brevet PCT/FR93/00923 (ou WO94/08014), aient permis d'identifier plusieurs protéines et les gènes de structure correspondants, impliqués dans la biosynthèse des deux types de composants (WO 93/20182).

Dans le processus de biosynthèse des streptogramines du groupe B, deux parties peuvent être distinguées:
1) Biosynthèse des précurseurs, ou de leurs analogues, du macrocycle: acide 3-hydroxypicolinique, acide L-2-aminobutyrique, 4-diméthylamino-L-phénylalanine, acide-L-pipécolique, L-phénylglycine.
2) Formation du macrocycle à partir des précurseurs cités ci-dessus, de la L-thréonine et de la L-proline, ou de leurs analogues, avec éventuellement modification(s) subséquente(s), de type N-méthylation peptidique, épimérisation, hydroxylation et oxydation.

La demande de brevet PCT/FR9300923 ou WO94/08014), a notamment pour objet les enzymes catalysant l'incorporation des précurseurs dans la chaîne peptidique des streptogramines B en cours d'élongation ainsi que leurs gènes de structure. Ces résultats ont permis de mettre en évidence le caractère de synthèse peptidique non ribosomale des composants de type B.

Le brevet US 4,156,734 décrit des compositions contenant un groupe aryle substitué par une alanine azo, et un acide arylhydrizino proprionique, notamment le 3-methylaminophenylalanine pour traiter l'hypertension.

La présente invention concerne plus particulièrement de nouveaux composés apparentés aux streptogramines du groupe B et plus précisément des nouveaux composés de la famille des pristinamycines I (figures 1 et 2), désignés ci-après par PI, ou de la famille des virginiamycines S (figure 3).

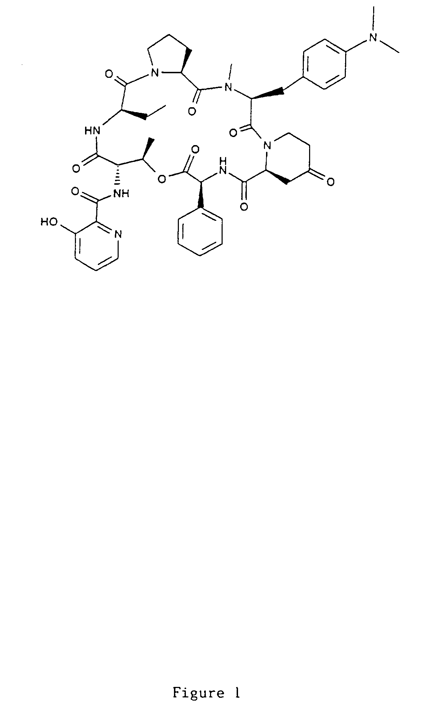

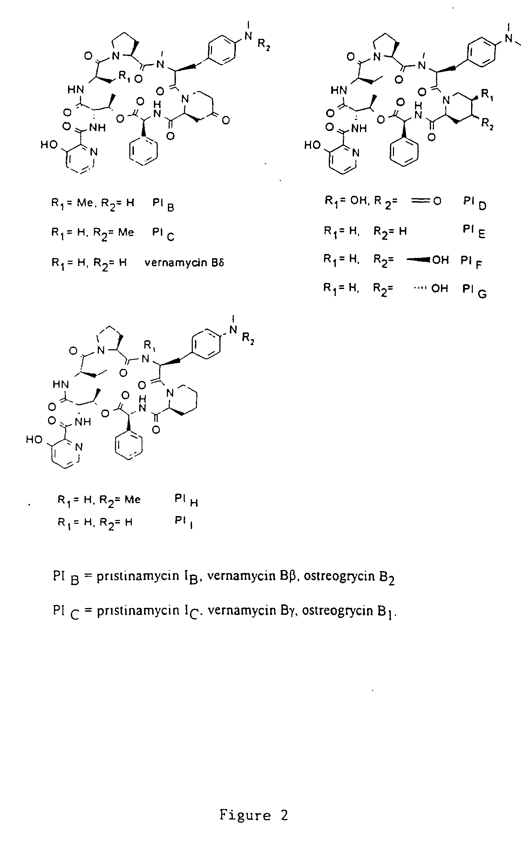

Le constituant majoritaire des pristinamycines I (PI) est la PI_{A} (figure 1) qui représente environ 94 % des PI, les environ 6 % restant étant représentés par des constituants minoritaires du depsipeptide (PI_{B} à PI_{I}) dont les structures sont représentées en figure 2. La PI résulte essentiellement de la condensation d'acides aminés dont certains sont indispensables pour la synthèse protéique (thréonine et proline) et dont d'autres sont originaux et considérés eux-mêmes comme des métabolites secondaires (acide L-2-aminobutyrique, 4-diméthylamino-L-phénylalanine (DMPAPA), acide L-pipécolique et L-phénylglycine pour la PI_{A}) ainsi que d'un précurseur aromatique, l'acide 3-hydroxypicolinique.

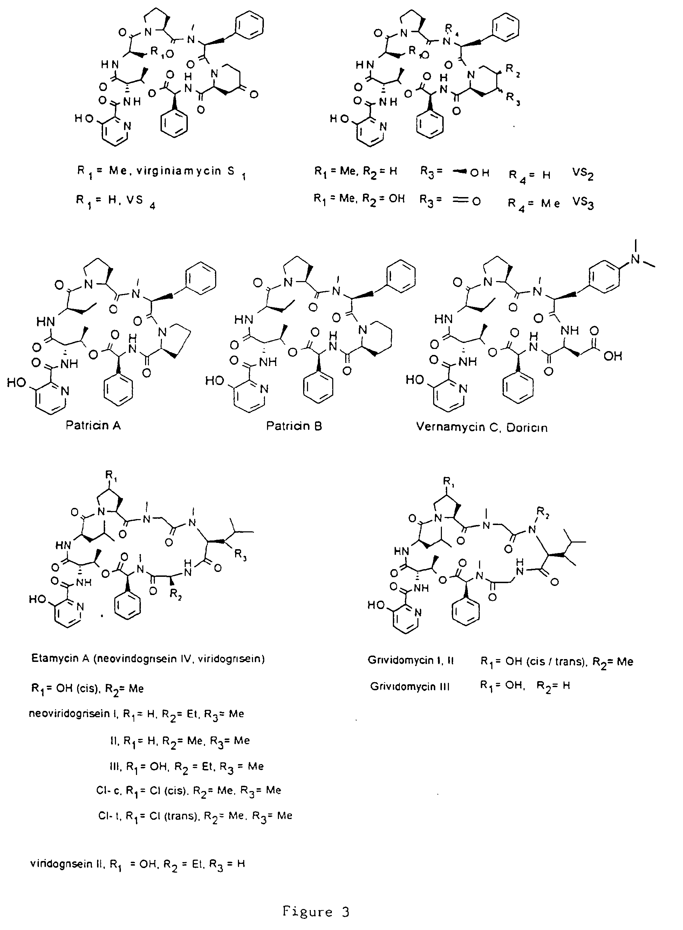

En ce qui concerne les dérivés de virginiamycines S, ils résultent de la condensation des mêmes acides que pour la PI à l'exception de la DMPAPA qui est remplacée par une phénylalanine (voir figure 3).

La production de ces différents composés par biosynthèse nécessite donc la synthèse préalable, par la souche productrice, des précurseurs originaux identifiés ci-dessus.

La présente invention résulte précisément d'un procédé de préparation original de streptogramines qui met en oeuvre, à titre de souche productrice de streptogramines, une souche de microorganisme mutée de manière à altérer la biosynthèse des précurseurs de streptogramines du groupe B. Selon ce procédé, ladite souche mutante est cultivée dans un milieu complémenté par un précurseur original, différent du précurseur dont la biosynthèse est altérée, De manière inattendue, il s'en suit une production de nouveaux composés apparentés aux streptogramines du groupe B, intéressants sur le plan thérapeutique.

Plus précisément, la présente invention se rapporte à de nouveaux composés représentés par la formule générale I: 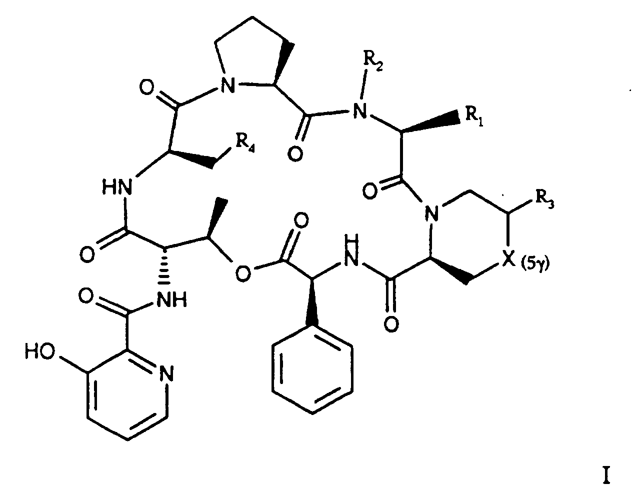 dans laquelle :
- R₂ et R₄ représentent, indépendamment l'un de l'autre, un atome d'hydrogène ou un groupement méthyle,
- R₃ représente un atome d'hydrogène ou un groupement hydroxyle,
- X représente un groupement CO, CHOH ou CH₂ et
- R₁ représente: 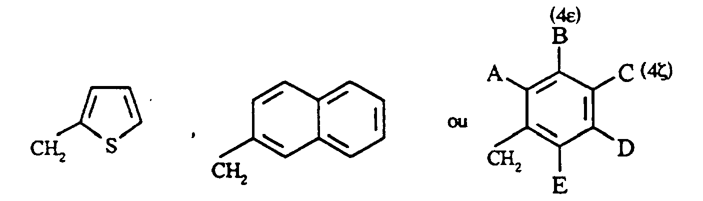
avec
- pour les dérivés méta :
   A, C, D et E représentant un atome d'hydrogène et
   B pouvant représenter:
   - un halogène et de préférence un atome de fluor,
   - un groupement monoalkylamino ou dialkylamino avec alkyle représentant de préférence un groupement méthyle ou éthyle,
   - un groupement éther. Il s'agit plus particulièrement d'un groupement OR avec R choisi de préférence parmi les groupements méthyle, éthyle, trifluorométhyle et allyle,
   - un groupement thioéther représenté de préférence par un groupement alkylthio avec de préférence allyle représentant un groupement méthyle,
   - un groupement alkyle en C₁ à C₃ ou
   - un groupement trihalogénométhyle et de préférence le trifluorométhyle.
- pour les dérivés para :
   A, B, D et E représentant un atome d'hydrogène et
   C pouvant représenter:
   - un halogène,
   - un groupement NR₁R₂ avec R₁ et R₂ représentant indépendamment l'un de l'autre un groupement choisi parmi
      - l'hydrogène,
      - un groupement alkyle en C₁ à C₄ linéaire ou ramifié avec lorsque l'un des substituants R₁ ou R₂ représente un groupement méthyle, l'autre représente obligatoirement un groupement éthyle,
      - un groupement alkyl-cycloalkylméthyle avec un cycloalkyle en C₃ à C₄
      - un groupement cycloalkyle en C₃ à C₄ éventuellement substitué,
      - un groupement alcényle en C₃ à C₄ linéaire ou ramifié avec lorsque l'un des substituants, R₁ ou R₂, représente un groupement alcényle, l'autre est différent d'un groupement méthyle ou d'un cycloalkyle en C3 à C6,
   - un groupement N-pyrrolidinyle substitué ou non,
   - un groupement éther, il s'agit de préférence d'un groupement OR avec R choisi de préférence parmi les groupements méthyle, éthyle éventuellement substitué par un atome de chlore, trifluorométhyle et alcényle .
   - un groupement thioéther représenté de préférence par un groupement alkylthio avec de préférence alkyle représentant un groupement alkyle en C₁ à C_{3.}
   - un groupement acyle ou alcoxycarbonyle et plus particulièrement un groupement COR avec R représentant de préférence un groupement alkyle en C₁ à C₃ ou un groupement alkoxy en C₁ à C_{3.}
   - un groupement alkyle en C₁ à C₆, linéaire ou ramifié, et de préférence choisi parmi les groupements méthyle, isopropyle et tert-butyle,
   - un groupement alkylthiométhyl et plus préférentiellement un groupement CH2SR avec R représentant de préférence un groupement alkyle en C₁ à C₃
   - un groupement aryle et de préférence un phényle ou
   - un groupement trihalogénométhyle et de préférence le trifluorométhyle.
- pour les dérivés disubstitués méta-para :
   A, D et E représentant un atome d'hydrogène et
   B pouvant représenter:
   - un halogène et de préférence un atome de fluor,
   - un groupement monoalkylamino ou dialkylamino avec alkyle représentant de préférence un groupement méthyle ou éthyle,
   - un groupement éther et de préférence un groupement OR avec R choisi de préférence parmi les groupements méthyle, éthyle et trifluorométhyle,
   - un groupement thioéther et de préférence alkylthio avec alkyle représentant préférentiellement un groupement éthyle, ou
   - un groupement alkyle en C₁ à C₃ et
   C pouvant représenter:
   - un halogène et de préférence un atome de fluor,
   - un groupement amino, monoalkylamino ou dialkylamino avec alkyle représentant de préférence un groupement méthyle à la condition que B soit différent d'un atome de brome ou de chlore, ou un groupement allyle substitué ou non,
   - un groupement éther et de préférence un groupement OR avec R choisi de préférence parmi les groupements méthyle, éthyle et trifluorométhyle,
   - un groupement thioéther et de préférence un groupement alkylthio avec de préférence alkyle représentant un groupement méthyle,
   - un groupement alkyle en C₁ à C₆ ou
   - un groupement trihalogénométhyle et de préférence le trifluorométhyle et
- pour les dérivés disubstitués ortho-para :
   B, E et D représentant un atome d'hydrogène et A et C, un groupement méthyle.

A titre de composés préférés, on peut plus particulièrement citer:
la 4ζ-méthylthio-dés(4ζ-diméthylamino) pristinamycine I_{A},
la 4ζ-méthylthio-dés(4ζ-diméthylamino) pristinamycine I_{H},
la 5γ-hydroxy 4ζ-méthylthio-dés(4ζ-diméthylamino) pristinamycine I_{H},
la 4ζ-méthyl-dés(4ζ-diméthylamino) pristinamycine I_{A},
la 4ζ-méthyl-dés(4ζ-diméthylamino) pristinamycine I_{H},
la 4ζ-méthoxy-dés(4ζ-diméthylamino) pristinamycine I_{A},
la 4ζ-méthoxycarbonyl-dés(4ζ-diméthylamino) pristinamycine I_{A},
la 4ζ-chloro-dés(4ζ-dméthylamino) pristinamycine I_{A},
la 4ζ-bromo-dés(4ζ-diméthylamino) pristinamycine I_{A},
la 4ζ-bromo-dés(4ζ-diméthylamino) pristinamycine I_{H},
la 4ζ-iodo-dés(4ζ-diméthylamino) pristinamycine I_{A},
la 4ζ-iodo-dés(4ζ-diméthylamino) pristinamycine I_{H},
la 4ζ-trifluorométhyl-dés(4ζ-diméthylamino) pristinamycine I_{A},
la 4ζ-trifluorométhyl-dés(4ζ-diméthylamino) pristinamycine I_{H},
la 4ζ-tert-butyl-dés(4ζ-diméthylamino) pristinamycine I_{A},
la 4ζ-isopropyl-dés(4ζ-diméthylamino) pristinamycine I_{A},
la 4ζ-isopropyl-dés(4ζ-diméthylamino) pristinamycine I_{E}.
la 4ε-méthylamino-dés(4ζ-diméthylamino) pristinamycine I_{A},
la 4ε-méthoxy-dés(4ζ-diméthylamino) pristinamycine I_{A}
la 4ε-méthoxy-dés(4ζ-diméthylamino) pristinamycine I_{H},
la 4ε-fluoro 4ζ-méthyl-dés(4ζ-diméthylamino) pristinamycine I_{A}
la 4ζ-amino-dés(4ζ-diméthylamino) pristinamycine I_{A}
la 4ζ-éthylamino-dés(4ζ-diméthylamino) pristinamycine I_{A}
la 4ζ-diéthylamino-dés(4ζ-diméthylamino) pristinamycine I_{A}
la 4ζ-allylamino-dés(4ζ-diméthylamino) pristinamycine I_{A}
la 4ζ-diallylamino-dés(4ζ-diméthylamino) pristinamycine I_{A}
la 4ζ-allyl éthylamino-dés(4ζ-diméthylamino) pristinamycine I_{A}
la 4ζ-éthyl propylamino-dés(4ζ-diméthylamino) pristinamycine I_{A}
la 4ζ-éthyl isopropylamino-dés(4ζ-diméthylamino) pristinamycine I_{A}
la 4ζ-éthyl méthylcyclopropylamino-dés(4ζ-diméthylamino) pristinamycine I_{A}
la 4ζ-(1-pyrrolidinyl) -dés(4ζ-diméthylamino) pristinamycine I_{A}
la 4ζ-trifluorométhoxy-dés(4ζ-diméthylamino) pristinamycine I_{A}
la 4ζ-allyloxy-dés(4ζ-diméthylamino) pristinamycine I_{A}
la 4ζ-éthoxy-dés(4ζ-diméthylamino) pristinamycine I_{A}
la 4ζ-éthylthio-dés(4ζ-diméthylamino) pristinamycine I_{A}
la 4ζ-méthylthiométhyl-dés(4ζ-diméthylamino) pristinamycine I_{A}
la 4ζ-(2-chloroéthoxy)-dés(4ζ-diméthylamino) pristinamycine I_{A}
la 4ζ-acétyl-dés(4ζ-diméthylamino) pristinamycine I_{A}
la 4ζ-éthyl-dés(4ζ-diméthylamino) pristinamycine I_{A}
la 4ζ-éthyl-dés(4ζ-diméthylamino) pristinamycine I_{H}
la 4ε-diméthylamino-dés(4ζ-diméthylamino) pristinamycine I_{A}
la 4ε-méthylthio-dés(4ζ-diméthylamino) pristinamycine I_{A}
la 4ε-éthoxy-dés(4ζ-diméthylamino) pristinamycine I_{A}

Un procédé notamment utile pour préparer les composés de formule générale I, est décrit ci-après.

Plus précisément, ce procédé pour préparer des streptogramines est **caractérisé en ce qu**'il met en oeuvre une souche d'un microorganisme producteur de streptogramines, possèdant au moins une modification génétique affectant la biosynthèse d'un précurseur des streptogramines du groupe B et en ce que ladite souche mutante est cultivée dans un milieu de culture adéquat et complémenté avec au moins un précurseur original, autre que celui dont la biosynthèse est altérée et en ce que l'on récupère lesdites streptogramines.

Les souches mises en oeuvre dans le cadre de la présente invention sont donc des souches productrices de streptogramines, mutées. La ou lesdites modifications génétiques peuvent être localisées soit au niveau d'un des gènes impliqués dans la biosynthèse desdits précurseurs soit en dehors de la région codante, par exemple dans les régions responsables de l'expression et/ou de la régulation transcriptionnelle ou post-transcriptionnelle desdits gènes ou dans une région appartenant au transcript contenant lesdit gènes.

Selon un mode particulier de l'invention, les souches mutantes possèdent une ou plusieurs modifications génétiques au niveau d'au moins un de leurs gènes impliqués dans la biosynthèse des précurseurs des streptogramines du groupe B.

Cette ou ces modifications génétiques altèrent l'expression dudit gène c'est à dire rendent ce gène, et le cas échéant un autre des gènes impliqués dans la biosynthèse des précurseurs, partiellement ou totalement incapable de coder pour l'enzyme naturelle impliquée dans la biosynthèse d'au moins un précurseur. L'incapacité desdits gènes à coder pour les protéines naturelles peut se manifester soit par la production d'une protéine inactive en raison de modifications structurales ou conformationnelles, soit par l'absence de production, soit par la production d'une protéine ayant une activité enzymatique altérée, ou encore par la production de la protéine naturelle à un niveau atténué ou selon un mode de régulation désiré. L'ensemble de ces manifestations possibles se traduit par une altération voire un bloquage au niveau de la synthèse d'au moins l'un des précurseurs des streptogramines du groupe B.

Les gènes, susceptibles d'être mutés dans le cadre de la présente invention, sont de préférence les gènes impliqués dans la biosynthèse des précurseurs suivants: acide L-2-aminobutyrique, 4-diméthylamino-L-phénylalanine (DMPAPA), acide L-pipécolique, L-phénylglycine et/ou l'acide 3-hydroxypicolinique (3-HPA).

Il s'agit plus préférentiellement des gènes papA, papM, papB (SEQ ID N°3), papC (SEQ ID N°2), hpaA (SEQ ID N°8), snbE (SEQ ID N°6) et pipA (SEQ ID N°5) décrits ci-après.

En ce qui concerne les gènes papA et papM, ils ont déjà été décrits dans la demande de brevet PCT/FR93/0923. Ils sont présents sur le cosmide pIBV2. Le gène papA semble correspondre à un gène de biosynthèse de la 4-amino-L-phénylalanine à partir du chorismate. La 4-amino-L-phénylalanine est ensuite diméthylée par le produit du gène papM. une N-méthyltransférase, pour former la 4-diméthylamino-L-phénylalanine, DMPAPA, qui est ensuite incorporée dans la pristinamycine I_{A}. Ces deux gènes interviennent donc plus particulièrement au niveau de la synthèse du précurseur dit DMPAPA.

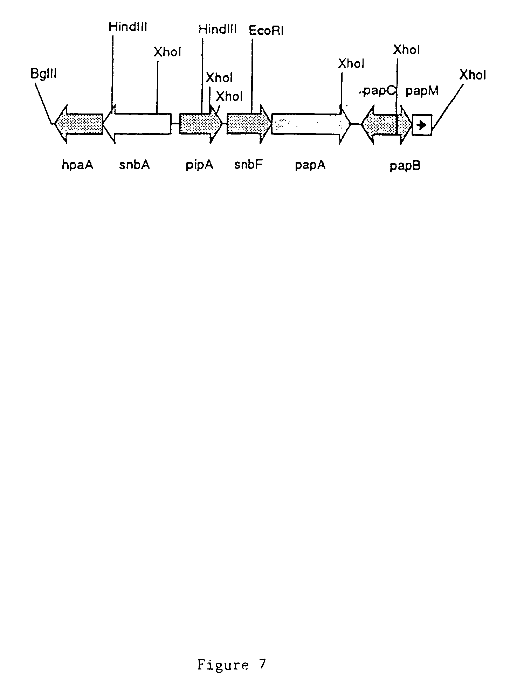

En ce qui concerne les autres gènes papB, papC, pipA, snbF et hpaA, ils ont été identifiés et caractérisés dans le cadre de la présente invention. Ils sont regroupés avec les gènes snbA, papA, et papM sur une région chromosomique d'environ 10 kb (figure 7).

Les homologies de séquences mises en évidence pour les protéines PapB et PapC, montrent que ces protéines sont également impliquées dans la biosynthèse du précurseur DMPAPA, conjointement avec les protéines PapA et PapM. Les deux nouveaux gènes correspondants, papB et papC, ont été isolés et identifiés par sous-clonages effectués à partir du cosmide pIBV2, décrit dans la demande de brevet PCT/FR93/0923 et d'un plasmide pVRC900, dérivé de pIBV2 par une délétion HindIII, également décrit dans la demande de brevet PCT/FR93/00923 (ou WO94/08014).

La comparaison de la protéine codée par le gène papC, avec les séquences protéiques contenues dans la banque Genpro fait apparaître une homologie de 27 % avec la région impliquée dans l'activité préphénate déhydrogénase des protéines bifonctionnelles TyrA d' E. coli (Hudson et Davidson, 1984) et d' Erwinia herbicola (EMBL data library, 1991). Cette région de TyrA catalyse l'aromatisation du préphénate en 4-hydroxyphénylpyruvate dans la biosynthèse de la tyrosine. Une aromatisation similaire à partir du 4-déoxy 4-amino préphénate conduisant au 4-amino phénylpyruvate intervient très vraisemblablement dans la synthèse de la DMPAPA. Elle serait catalysée par la protéine PapC (SEQ ID n°2).

Quant à la protéine PapB, elle possède une homologie de 24 à 30 % avec la région impliquée dans l'activité chorismate mutase des protéines bifonctionnelles TyrA et PheA d' E. coli (Hudson et Davidson, 1984) et de la protéine TyrA d' Erwinia herbicola. Cette région catalyse l'isomérisation du chorismate en préphénate dans la biosynthèse de la tyrosine et de la phénylalanine. La protéine PapB (SEQ ID n°3) intervient vraisemblablement au niveau de l'isomérisation similaire à partir du 4-déoxy 4-amino chorismate conduisant au 4-déoxy 4-aminopréphénate dans la synthèse de la DMPAPA.

En ce qui concerne les gènes pipA, snbF et hpaA, ils ont été localisés dans les régions contenues entre le gène snbA codant pour l'acide 3-hydroxypicolinique AMP ligase, décrite dans la demande de brevet PCT/FR93/0923 et les gènes papA ou snbR. Ils ont précisément été localisés par sous-clonages effectués à partir du plasmide pVRC900 et du cosmide pIBV2, décrits dans la demande de brevet PCT/FR93/0923.

En comparant la protéine codée par le gène hpaA et les séquences protéiques contenues dans la banque Genpro, il a été mis en évidence une homologie de 30 à 40 % avec un groupe de protéines probablement impliquées (Thorson et al., 1993) dans la transamination d'intermédiaires de biosynthèse de différents antibiotiques (DnrJ, EryC1, TylB, StrS, PrgL). La synthèse du précurseur 3-HPA qui semble dériver de la lysine par une autre voie que la cyclodéamination (voir exemples 1-2 et 2-1), nécessite vraisemblablement une étape de transamination susceptible d'être catalysée par le produit de ce gène appelé hpaA (SEQ ID n°8). Les résultats de mutation réalisée dans ce gène montrent par ailleurs sans équivoque l'implication de ce gène dans la synthèse du précurseur 3-HPA.

La comparaison du produit codé par le gène dit pipA avec les séquences protéiques contenues dans la banque Genpro fait apparaître une homologie de 30 % avec l'ornithine cyclodéaminase d'Agrobacterium tumefasciens (Schindier et al., 1989). Cette enzyme intervient dans la dernière étape du catabolisme de l'octopine; elle convertit la L-omithine en L-proline par cyclo-déamination. Des auteurs ont montré par incorporation de lysine marquée, que l'acide 4-oxopipécolique et l'acide 3-hydroxypicolinique, retrouvés aussi bien dans la PI_{A} que dans la virginiamycine S1, dérivaient de la lysine (Molinero et al., 1989; Reed et al., 1989). Une réaction de cyclodéamination de la lysine similaire à celle décrite pour l'ornithine conduirait à la formation d' acide pipécolique. En tenant compte de cette hypothèse ce produit a été appelé PipA (SEQ ID n° 5). Les résultats de mutation dans le gène pipA, présentés dans les exemples ci-après, montrent l'implication du gène pipA dans la seule synthèse de l'acide pipécolique. On note en particulier que cette mutation n'affecte pas la biosynthèse de l'acide 3-hydroxypicolinique, qui dérive aussi de la lysine et dont l'acide pipécolique aurait pu être un précurseur.

Enfin, en comparant le produit du gène dit snbF avec les séquences protéiques contenues dans la banque Genpro, il a été noté une homologie de 30 à 40 % avec plusieurs hydroxylases de type cytochrome P450, impliquées dans la biosynthèse de métabolites secondaires (Omer et al., 1990. Trower et al., 1992). Plusieurs hydroxylations sont envisageables dans la biosynthèse des précurseurs de la pristinamycine I, notamment au niveau de la biosynthèse du 3-HPA (hydroxylation en 3 de l'acide picolinique) et de l'acide 4-oxopipécolique (hydroxylation en 4 de l'acide pipécolique). La protéine correspondante a été appelée SnbF (SEQ ID n°6).

Les résultats de mutation dans le gène pipA, avec des effets polaires sur l'expression du gène snbF, montrent l'implication du gène snbF dans l'hydroxylation du résidu acide pipécolique des streptogramines du groupe B. C'est ainsi que l'expression du gène snbF est altérée par le biais d'une modification génétique au niveau du gène pipA.

Préférentiellement, la ou les modifications génétiques rendent ledit gène partiellement ou totalement incapable de coder pour la protéine naturelle.

Par modification génétique, on doit entendre plus particulièrement toute suppression, substitution, délétion, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore en exposant lesdits microorganismes à un traitement au moyen d'agents mutagènes. Par agents mutagènes, on peut citer par exemple les agents physiques tels que les rayonnements énergétiques (rayons X, γ, ultra violet, etc..), ou les agents chimiques capables de réagir avec différents groupements fonctionnels des bases de l'ADN, et par exemple les agents alkylants [éthylméthane sulfonate (EMS), N-méthyl-N'-nitro-N-nitrosoguanidine, N-nitroquinoléine-1-oxyde (NQO)], les agents bialkylants, les agents intercalants, etc... Par délétion, on entend toute suppression d'une partie ou de la totalité du gène considéré. Il peut s'agir notamment d'une partie de la région codant pour lesdites protéines, et/ou de tout ou partie de la région promotrice de la transcription, de la traduction ou encore du transcript.

Les modifications génétiques peuvent également être obtenues par disruption génique, par exemple selon le protocole initialement décrit par Rothstein [Meth. Enzymol. 101 (1983) 202] ou avantageusement par double recombinaison homologue. Dans ce cas, l'intégralité de la séquence codante sera préférentiellement perturbée pour permettre le cas échéant, le remplacement, par recombinaison homologue, de la séquence génomique sauvage par une séquence non fonctionnelle ou mutante.

Selon un autre mode de réalisation, les modifications génétiques peuvent consister à placer le ou les gènes codant pour lesdites protéines sous contrôle d'un promoteur régulé.

Les souches de micororganismes mutantes décrites peuvent être obtenues à partir de tout microorganisme producteur de streptogramines (Cf. tableau V). Selon un mode de réalisation particulier de l'invention, il s'agit d'une souche dérivant de S. pristinaespiralis et plus particulièrement de S. pristinaespiralis SP92.

A titre de souche mutante préférée dans le cadre de la présente invention, on peut plus particulièrement citer la souche SP92::pVRC508, mutée dans la biosynthèse du précurseur DMPAPA par disruption par simple crossing-over du gène papA ou encore plus préférentiellement la souche SP212, mutée dans la biosynthèse du précurseur DMPAPA par disruption du gène papA par double recombinaison homologue. Ces souches ne produisent plus de PI sauf lorsqu'elles sont complémentées par le précurseur DMPAPA. De manière inattendue, lorsqu'un précurseur original, différent de la DMPAPA et capable après, le cas échéant, métabolisation, d'être incorporé par la PI synthétase III (protéine SnbD responsable de l'incorporation des résidus L-proline et DMPAPA) est ajouté au milieu de production, ces deux souches deviennent capables de produire de nouvelles pristinamycines I ou virginiamycines ou de produire majoritairement un composant normalement minoritaire de la PI, notamment la PI_{B} (figure 2).

Dans le cadre de la présente invention, deux autres souches mutantes ont été préparées. Il s'agit respectivement de la souche SP92pipA::Ωam^{R} disruptée dans le gène pipA par recombinaison homologue et la souche SP92hpaA::Ωam^{R} disruptée dans le gène hpaA. La souche SP92pipA::Ωam^{R} d'une part, ne produit plus de PI dans les conditions standards de fermentation et d'autre part, en présence d'acide L-pipécolique, permet la forte production d'un composant initialement minoritaire des composants B des streptogramines dans lesquels l'acide 4-oxopipécolique est remplacé par l'acide L-pipécolique. Quant à la souche S. pristinaespiralis SP92hpaA:: Ωam^{R}, elle ne produit plus de PI dans les conditions standards de fermentation mais est capable de produire de nouvelles streptogramines du groupe B, en présence de précurseurs originaux.

En complémentant le milieu de culture de souches mutantes selon l'invention, avec au moins un précurseur original, il s'avère possible d'orienter la biosynthèse soit vers de nouvelles streptogramines, soit vers une forme minoritaire d'entre elles, ou encore de privilégier la formation d'une d'entre elles.

Les précurseurs mis en oeuvre dans le cadre de la présente invention peuvent être des dérivés ou analogues d'acides aminés et plus particulièrement de la phénylalanine ainsi que d'acides organiques et notamment d'acides alpha-cétocarboxyliques et plus particulièrement des dérivés d'acide phénylpyruvique.

Bien entendu, le précurseur original est tel qu'il pourvoit à l'altération voire le blocage, induit selon l'invention, au niveau de la biosynhèse d'un des précurseurs naturels des streptogramines du groupe B et conduit à la synthèse de streptogramines. Selon un mode particulier de l'invention, ce précurseur original est choisi de manière à être apparenté au précurseur dont la biosynthèse est altérée. Ainsi dans le cas particulier de mutant bloqué dans la biosynthèse du DMPAPA, le précurseur original est de préférence un dérivé de phénylalanine.

A titre de précurseurs convenant à l'invention, on peut notamment citer les suivants:
Phénylalanine, 4-diméthylaminophénylalanine, 4-méthylaminophénylalanine, 4-aminophénylalanine, 4-diéthylaminophénylalanine, 4-éthylaminophénylalanine, 4-méthylthiophénylalanine, 4-méthylphénylalanine, 4-méthoxyphénylalanine, 4-trifluorométhoxyphénylalanine, 4-méthoxycarbonylphénylalanine, 4-chlorophénylalanine, 4-bromophénylalanine, 4-iodophénylalanine, 4-trifluorométhylphénylalanine, 4-tert-butylphénylalanine, 4-isopropylphénylalanine, 3-méthylaminophénylalanine, 3-méthoxyphénylalanine, 3-méthylthiophénylalanine, 3-fluoro 4-méthylphénylalanine, acide L-pipécolique, acide 4-tert-butylphénylpyruvique, acide 4-méthylaminophénylpyruvique, 2-naphtylphénylalanine, 4-fluorophénylalanine, 3-fluorophénylalanine, 3-éthoxyphénylalanine, 2,4-diméthylphénylalanine, 3,4-diméthylphénylalanine, 3-méthylphénylalanine, 4-phénylphénylalanine, 4-butylphénylalanine, 2-thiényl-3-alanine, 3-trifluorométhylphénylalanine, hydroxyphénylalanine, 3-éthylaminophénylalanine 4- allylaminophénylalanine, 4-diallylaminophénylalanine, 4- allyl éthylaminophénylalanine, 4- éthyl propylaminophénylalanine, 4- éthyl isopropylaminophénylalanine, 4- éthyl méthylcyclopropylaminophénylalanine, 4-(1-pyrrolidinyl) phénylalanine, 4-O-allyltyrasine, 4-O-éthyltyrosine, 4-éthylthiophénylalanine, 4-éthylthiométhylphénylalanine, 4-O-(2-chloroéthyl) tyrosine, 4-acétyl phénylalanine, 4-éthyl phénylalanine, 3-diméthylaminophénylalanine, 3-éthoxy phénylalanine, 3-fluoro4-méthylphénylalanine et 4-aminométhylphénylalanine.

Parmi ces précurseurs, les 4-trifluorométhoxyphénylalanine, 3-méthylaminophénylalanine, 3-méthylthiophénylalanine, 3-fluoro4-méthylphénylalanine, l'acide 4-méthylaminophénylpyruvique, 3-éthoxyphénylalanine, 4- allylaminophénylalanine, 4- diallylaminophénylalanine, 4-allyl éthylaminophénylalanine, 4- éthyl propylaminophénylalanine, 4- éthyl isopropylaminophénylalanine, 4-éthylméthyl-cyclopropylaminophénylalanine, 4-(1-pyrrolidinyl) phénylalanine, 4-éthylthiométhylphénylalanine, 4-O-(2-chloroéthyl) tyrosine, 3-diméthylaminophénylalanine et 3-éthylaminophénylalanine sont nouveaux et ont été préparés et caractérisés dans le cadre de la présente invention. Ils se révèlent particulièrement utiles pour préparer des streptogramines selon l'invention.

Le procédé revendiqué s'avère particulièrement intéressant pour préparer de nouvelles streptogramines du groupe B ou encore pour privilégier la formation de certaines d'entre elles. A ce titre il est tout particulièrement utile pour préparer la PIB.

Les associations d'un composant des streptogramines du groupe A et d'un composé de formule générale I, selon l'invention, constituent des compositions particulièrement intéressantes sur le plan thérapeutique. Elles sont notamment employées pour les traitements d'affections dues à des bactéries Gram-positifs (du genre Staphylocoques, Streptocoques, Pneumocoques, Entérocoques) et Gram-négatifs (du genre Haemophilus, Gonocoques, Méningocoques). C'est ainsi que les composés selon l'invention synergisent l'action antibactérienne de la pristinamycine IIB sur Staphylococcus aureus IP8203 in vivo chez la souris, à des doses principalement comprises entre 30mg/kg et 100mg/kg par voie orale, lorsqu'ils sont associés dans des proportions PI /PII de l'ordre de 30/70.

La présente invention s'étend à toute composition pharmaceutique contenant au moins un composé de formule générale I en association ou non avec une streptogramine du groupe A.

Les exemples figurant ci-après sont présentés à titre illustratif et non limitatif de la présente invention.

### LISTE DES FIGURES.

Figure 1 : Structure de la pristinamycine I_{A}.
Figure 2 : Structure des composants minoritaires de la pristinamycine I.
Figure 3 : Autres exemples de structures de composants B des streptogramines.

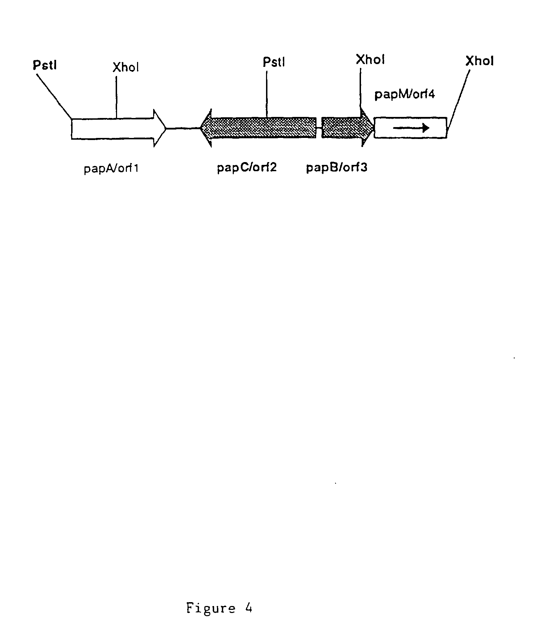

Figure 4 : Représentation de la région PstI-XhoI de 2,9 kb.

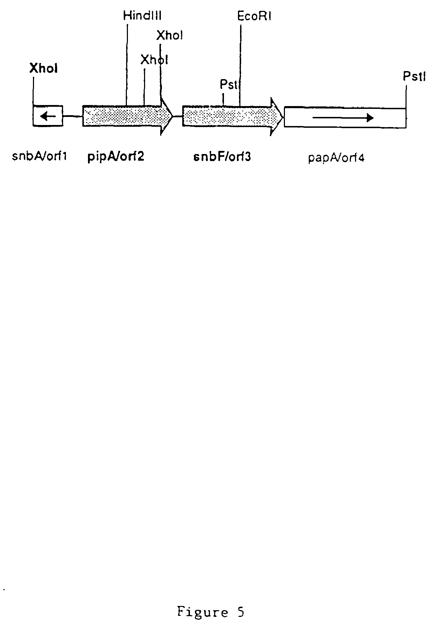

Figure 5 : Représentation de la région XhoI-PstI de 4,5 kb.

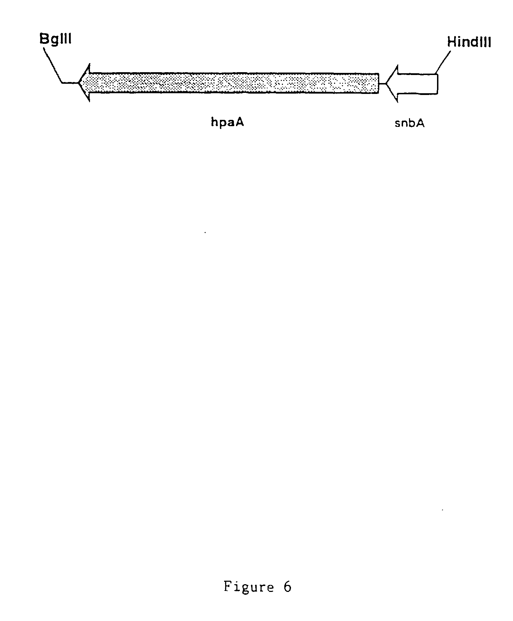

Figure 6 : Représentation de la région HindIII-BglII de 1,6 kb.
Figure 7 : Représentation de la région BglII-XhoI d'environ 10 kb.

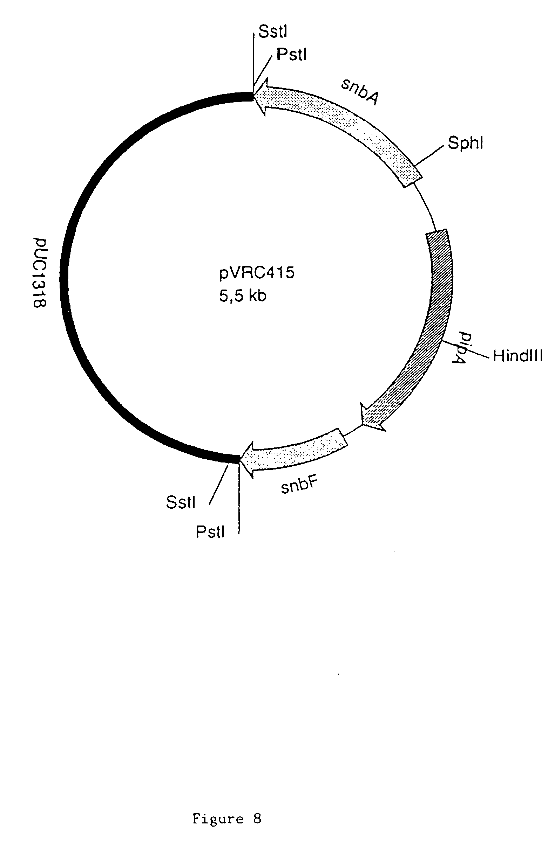

Figure 8 : Représentation du plasmide pVRC415.

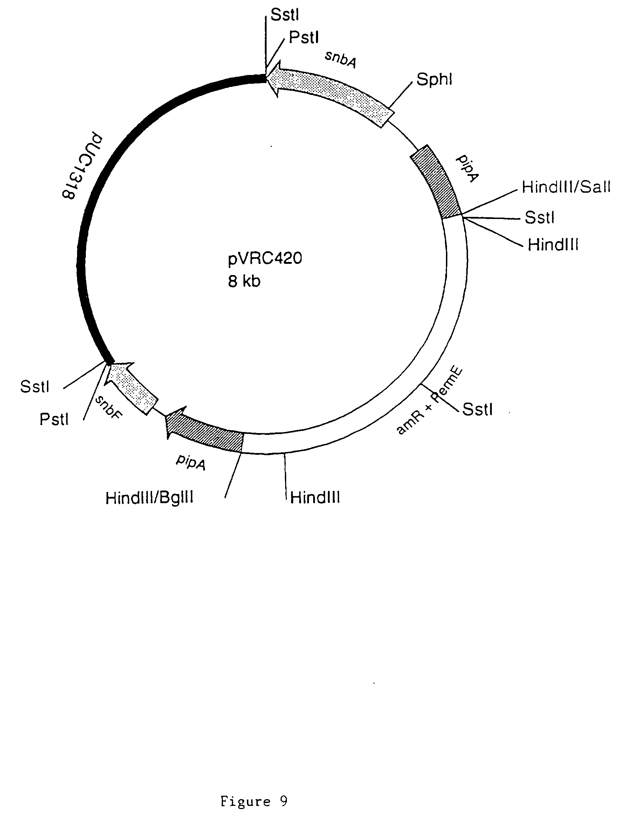

Figure 9 : Représentation du plasmide pVRC420.

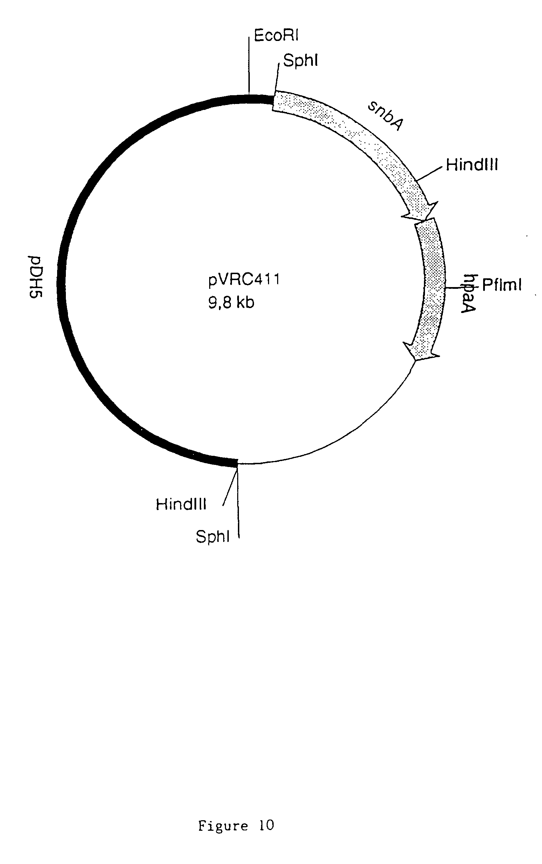

Figure 10 : Représentation du plasmide pVRC411.

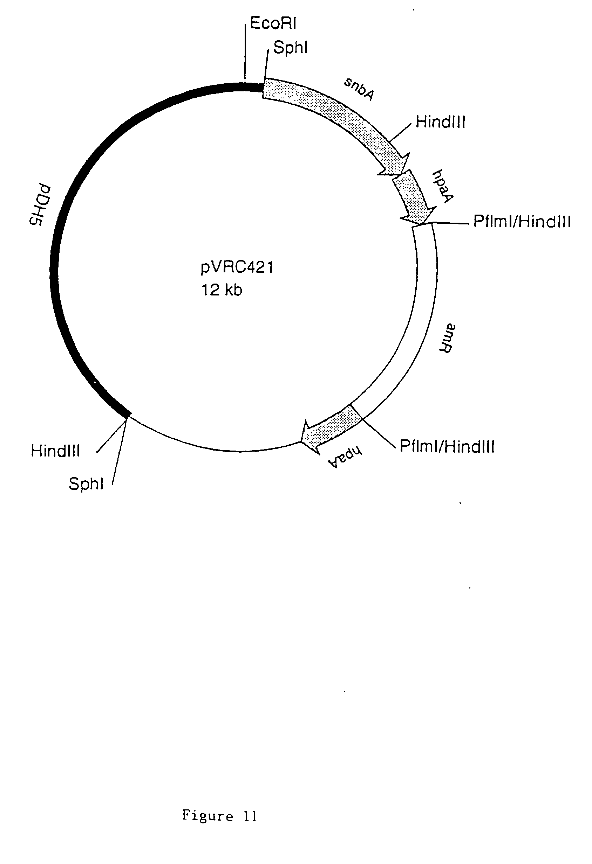

Figure 11 : Représentation du plasmide pVRC421.

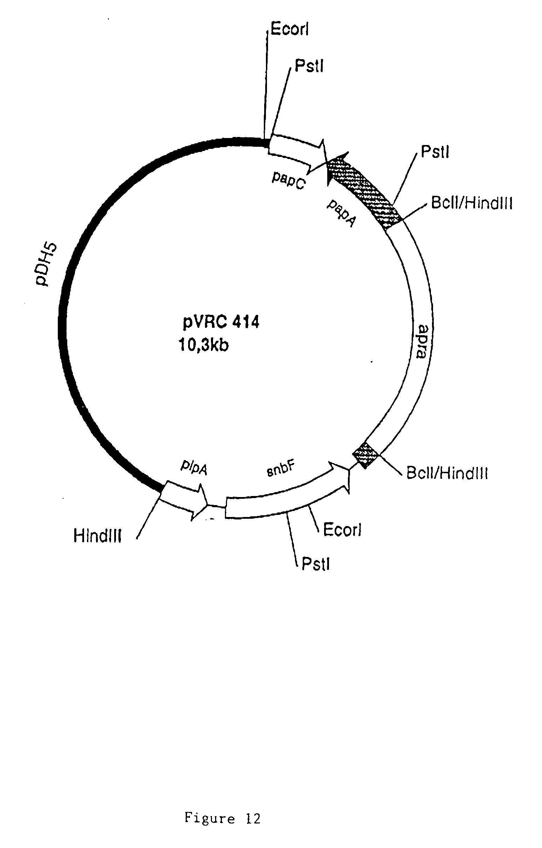

Figure 12: Représentation du plasmide pVRC414.

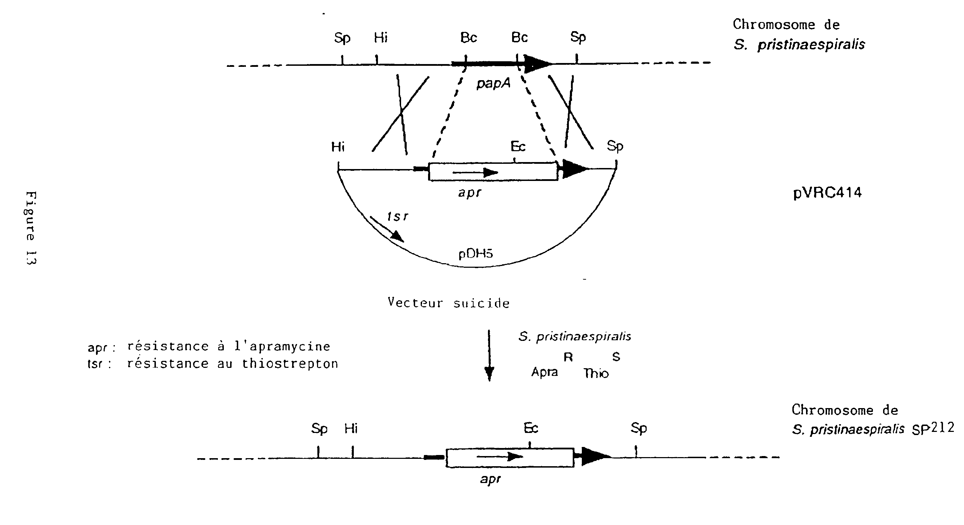

Figure 13: Stratégie de construction de SP212.

### EXEMPLE 1: Séquençage et identification de gènes impliqués dans la biosynthèse de la pristinamycine I et de ses précurseurs.

Identification par séquençage des gènes situés en aval et en amont du gène codant pour l'enzyme PapA décrite dans le brevet PCT/FR93/0923, ainsi que d'un gène situé en aval du gène codant pour l'enzyme SnbA, également décrite dans le brevet PCT/FR93/0923.

Cet exemple décrit comment à partir du cosmide pIBV2, décrit dans le brevet PCT/FR93/0923, et contenant les gènes de structure des enzymes PapA et PapM, intervenant dans la synthèse du précurseur 4-diméthylamino-L-phénylalanine (DMPAPA) de la pristinamycine I et le gène de structure de l'enzyme SnbA responsable de l'activation du précurseur aromatique de la pristinamycine I, l'acide 3-hydroxypicolinique (3-HPA), il s'est avéré possible d'identifier par séquençage autour de ces gènes et étude des mutants correspondants, d'autres gènes impliqués dans la biosynthèse du précurseur DMPAPA, ou dans la biosynthèse d'autres précurseurs de la pristinamycine I.

Dans ce but, des sous-clonages ont été effectués à partir du cosmide pIBV2, et du plasmide pVRC900, dérivé de pIBV2 par une délétion HindIII. et également décrit dans le brevet PCT/FR93/00923 (ou WO94/08014).

Cet exemple illustre comment les séquences nucléotidiques de fragments situés en aval et en amont des gènes papA et snbA de S. pristinaespiralis peuvent être obtenues.

Les techniques de clonage des fragments d'ADN d'intérêt dans les vecteurs M13mp18/19 (Messing et al., 1981) sont les techniques classiques de clonage dans Escherichia coli et sont décrites dans Maniatis et al. (1989).

### 1-1 Séquençage et analyse de la région en aval du gène papA.

Pour séquencer cette région, contenue entre les gènes papA et papM. les fragments PstI-PstI de 1,5 kb, PstI-XhoI de 0,7 kb et XhoI-XhoI de 0,7 kb ont été sous-clonés dans les vecteurs M13mp18 et M13mp19 à partir du plasmide pVRC900. Les sites de clonages ont été traversés par séquençage sur ADN double brin, en utilisant les plasmides pVRC900 et pVRC409 décrits dans le brevet PCT/FR93/00923 (ou WO94/08014). Les clonages ont été réalisés comme suit. Environ 2 µg du plasmide pVRC900 ont été coupés par les enzymes de restriction PstI et/ou XhoI (New England Biolabs) dans les conditions préconisées par le fournisseur. Les fragments de restrictions ainsi obtenus ont été séparés sur gel d'agarose 0,8% et les fragments d'intérêt, PstI-PstI de 1,5 kb, PstI-XhoI de 0,7 kb et XhoI-XhoI de 0,7 kb ont été isolés et purifiés par Geneclean (Bio101, La Jolla, Californie). Pour chaque clonage, environ 10 ng de M13mp19 et/ou M13mp18 coupés par PstI et/ou XhoI, ont été ligaturés avec 100 ng du fragment à cloner, dans les conditions décrites par Maniatis et al. 1989. Après transformation de la souche TG1 (K12, Δ(*lac-pro*) *sup*E *thi hsd* ΔS F *tra*D36 *pro*A⁺B⁺ *lac*Iq *lac*Z ΔM15; Gibson, 1984) et sélection des plages de lyse sur milieu LB + X-gal + IPTG, selon la technique décrite par Maniatis et al. (1989), les phages portant les fragments souhaités ont été isolés. Les différents inserts ont été séquencés par la méthode de réaction de terminaison de chaîne en utilisant comme amorce de synthèse le primer universel ou des oligonucléotides synthétiques et complémentaires d'une séquence de 20 nucléotides de l'insert à séquencer. Les réactions ont été faites en utilisant des didéoxynucléotides fluorescents (PRISM Ready Reaction DyeDeoxy Terminator Cycle Sequencing Kit-Applied Biosystem) et analysées sur un séquenseur d'ADN de type Applied Biosystems Model 373A. Le recouvrement entre ces différents inserts a permis d'établir la séquence nucléotidique totale présente entre les gènes papA et papM (SEQ ID N°1).

A partir de cette séquence nucléotidique, il est possible de déterminer les phases ouvertes de lecture et d'identifier ainsi des gènes impliqués dans la biosynthèse de la PI ou de ses précurseurs chez S. pristinaespiralis ainsi que les polypeptides codés par ces gènes.

Nous avons recherché la présence de phases ouvertes de lecture au sein du fragment PstI-XhoI de 2,9 kb contenant la séquence nucléotidique entre les gènes papA et papM, en utilisant le fait que l'ADN des Streptomyces présente un haut pourcentage en bases G et C ainsi qu'un fort biais dans l'usage des codons qui composent les phases codantes (Bibb et al. 1984). La méthode de Staden et Mc Lachlan (1982) permet de calculer la probabilité des phases codantes en fonction de l'usage des codons de gènes de Streptomyces déjà séquencés et rassemblés dans un fichier contenant 19673 codons obtenu à partir du serveur informatique BISANCE (Dessen et al. 1990).

Cette méthode a permis de caractériser au sein du fragment PstI-XhoI de 2,9 kb, quatre phases ouvertes de lecture fortement probables qui sont représentées dans le tableau suivant (TABLEAU I). Elles sont nommées phases 1 à 4 d'après leur position à partir du site PstI. Pour chacune, on a indiqué leur longueur en bases et leur position au sein du fragment (le site PstI étant situé à la position 1); pour les phases ouvertes de lecture 2 et 3, a été également indiquée la taille en acides aminés du polypeptide codé. Les phases 1, 3 et 4 sont codées par le même brin et la phase 2 par le brin complémentaire (figure 4). Les phases 1 et 4 correspondent respectivement à la région C-terminale de la protéine PapA et N-terminale de la protéine PapM précédemment identifiées et décrites dans le brevet PCT/FR93/00923 (ou WO94/08014).

**TABLEAU I**

| **Numéro de la phase et/ou nom du gène** | **Position** | **Nombre de nucléotides** | **Nombre d'acides aminés** |
|---|---|---|---|
| 1 (PapA) | 1-684 | 684 | - |
| 2 (PapC)(inv) | 949-1836 | 888 | 296 |
| 3 (PapB) | 1873-2259 | 387 | 129 |
| 4 (PapM) | 2259-2887 | 629 | - |

La comparaison du produit de la phase 2 (TABLEAU I) avec les séquences protéiques contenues dans la banque Genpro fait apparaître une homologie de 27 % avec la région impliquée dans l'activité préphénate déhydrogénase des protéines bifonctionnelles TyrA d' E. coli (Hudson et Davidson, 1984) et d' Erwinia herbicola (EMBL data library, 1991). Cette région de TyrA catalyse l'aromatisation du préphénate en 4-hydroxyphénylpyruvate dans la biosynthèse de la tyrosine. Une aromatisation similaire à partir du 4-déoxy 4-amino préphénate conduisant au 4-amino phénylpyruvate intervient très vraisemblablement dans la synthèse de la DMPAPA. Cette réaction serait catalysée par le produit de la phase 2, appelé PapC (SEQ ID n°2).

La comparaison du produit de la phase 3 (TABLEAU I) avec les séquences protéiques contenues dans la banque Genpro fait apparaître une homologie de 24 à 30 % avec la région impliquée dans l'activité chorismate mutase des protéines bifonctionnelles TyrA et PheA d' E. coli (Hudson et Davidson, 1984) et de la protéine TyrA d' Erwinia herbicola . Cette région catalyse l' isomérisation du chorismate en préphénate dans la biosynthèse de la tyrosine et de la phénylalanine. Une isomérisation similaire à partir du 4-déoxy 4-amino chorismate conduisant au 4-déoxy 4-aminopréphénate intervient très vraisemblablement dans la synthèse de la DMPAPA. Cette réaction serait catalysée par le produit de la phase 3, appelé PapB (SEQ ID n°3).

Dans le cas de TyrA et PheA les activités chorismate mutase et préphénate déhydratase ou préphénate déhydrogénase sont catalysées par la même protéine. Chez S. pristinaespiralis, les activités enzymatiques chorismate mutase et préphénate déhydrogénase sont catalysées par deux protéines séparées, PapB et PapC respectivement.

Les homologies de séquences mises en évidence pour les protéines PapB et PapC, montrent que ces deux protéines sont impliquées dans la biosynthèse du dérivé aromatique DMPAPA, conjointement avec les protéines PapA et PapM. De même que pour papA la disruption des gènes papB et papC doit conduire à la construction de souches de S. pristinaespiralis incapables de produire de la PI mais susceptibles de produire en présence de précurseurs originaux de nouvelles PI modifiées au niveau du résidu DMPAPA.

### 1-2. Séquençage et analyse de la région en amont du gène papA.

Cette région est contenue entre le gène snbA codant pour l'acide 3-hydroxypicolinique-AMP ligase, décrite dans le brevet PCT/FR93/00923 et le gène papA.

Les clonages ont été réalisés comme décrit dans l'exemple 1-1, à partir du plasmide pVRC900 et du cosmide pIBV2 décrits dans le brevet PCT/FR93/00923. Les fragments XhoI-XhoI de 1,3 kb, XhoI-XboI de 0,2 kb, XhoI-XhoI de 3,3 kb, HindIII-PstI de 1,1 kb et PstI-PstI de 2,2 kb ont été sous-clonés dans les vecteurs M13mp18 et M13mp19. Ces différents clonages ont permis de traverser tous les sites de clonage. Les différents inserts ont été séquencés comme décrit en 1-1, en utilisant comme amorce de synthèse le primer universel ou des oligonucléotides synthétiques, complémentaires d'une séquence de 20 nucléotides de l'insert à séquencer.

Le recouvrement entre ces différents inserts a permis d'établir la séquence nucléotidique totale présente entre les gènes snbA et papA (SEQ ID n° 4).

A partir de cette séquence nucléotidique, il est possible de déterminer les phases ouvertes de lecture et d'identifier des gènes impliqués dans la biosynthèse des précurseurs de la PI de S. pristinaespiralis ansi que les polypeptides codés par ces gènes.

Nous avons recherché la présence de phases ouvertes de lecture au sein du fragment XhoI-PstI de 4,5 kb contenant la séquence nucléotidique entre les gènes snbA et papA. comme décrit dans l'exemple 1.1. Cette méthode a permi de caractériser au sein du fragment XhoI-PstI de 4,5 kb, quatre phases ouvertes de lecture fortement probables qui sont représentées dans le tableau suivant (TABLEAU II). Elles sont nommées phases 1 à 4 d'après leur position à partir du site XhoI. Pour chacune, on a indiqué leur longueur en bases et leur position au sein du fragment (le site XhoI étant situé à la position 1); pour les phases ouvertes de lecture 2 et 3, on a également indiqué la taille en acides aminés du polypeptide codé. Les phases 2, 3 et 4 sont codées par le même brin et la phase 1 par le brin complémentaire (figure 5). Les phases 1 et 4 correspondent respectivement aux régions N-terminales des protéines SnbA et PapA précédemment identifiées et décrites dans le brevet PCT/FR93/00923 (ou WO94/08014).

**TABLEAU II**

| **Numéro de la phase et/ou nom du gène** | **Position** | **nombre de nucléotides** | **nombre d'acides aminés** |
|---|---|---|---|
| 1 (SnbA) (inv) | 1-329 | 329 | - |
| 2 (PipA) | 607-1671 | 1065 | 355 |
| 3 (SnbF) | 1800-2993 | 1194 | 398 |
| 4 (PAPA) | 3018-4496 | 1479 | - |

La comparaison du produit de la phase 2 (TABLEAU II) avec les séquences protéiques contenues dans la banque Genpro fait apparaître une homologie de 30 % avec l'ornithine cyclodéaminase d' Agrobacterium tumefasciens (Schindler et al., 1989). Cette enzyme intervient dans la dernière étape du catabolisme de l'octopine; elle convertit la L-omithine en L-proline par cyclo-déamination. Des auteurs ont montré par incorporation de lysine marquée, que l'acide 4-oxopipécolique et 1' acide 3-hydroxypicolinique, retrouvés aussi bien dans la PI_{A} que dans la virginiamycine S1, dérivaient de la lysine (Molinero et al., 1989; Reed et al., 1989). Une réaction de cyclodéamination de la lysine similaire à celle décrite pour 1' ornithine conduirait à la formation d' acide pipécolique. En tenant compte de cette hypothèse le produit de la phase 2 a été appelé PipA (SEQ ID n° 5). Les résultats de mutation dans le gène pipA, présentés en 2-1, montrent l'implication du gène pipA dans la seule synthèse de 1' acide pipécolique car cette mutation n' affecte pas la biosynthèse de l'acide 3-hydroxypicolinique, qui dérive aussi de la lysine et dont l'acide pipécolique aurait pu être un précurseur.

La comparaison du produit de la phase 3 (TABLEAU II) avec les séquences protéiques contenues dans la banque Genpro fait apparaître une homologie de 30 à 40 % avec plusieurs hydroxylases de type cytochrome P450 impliquées dans la biosynthèse de métabolites secondaires (Omer et al., 1990. Trower et al., 1992). Plusieurs hydroxylations sont envisageables dans la biosynthèse des précurseurs de la pristinamycine I, notamment au niveau de la biosynthèse du 3-HPA (hydroxylation en 3 de l'acide picolinique) et de l'acide 4-oxopipécolique (hydroxylation en 4 de l'acide pipécolique). Les résultats de mutation dans le gène pipA. présentés en 2-1-3, montrent l'implication du produit de la phase 3 dans 1' hydroxylation du résidu acide pipécolique de la PI_{E}. Le gène correspondant a donc été appelé snbF et la protéine correspondante SnbF (SEQ ID n°6).

### 1-3. Séquencage de la région en aval du gène snbA.

Cette région est comprise entre le gène snbA codant pour l'acide 3-hydroxypicolinique-adénylate-ligase et le gène snbR, codant pour une protéine membranaire probablement responsable du transport et de la résistance à la PI, toutes deux décrites dans le brevet PCT/FR93/00923. Les séquences ont été réalisées à partir d'un fragment isolé du cosmide pIBV2, comme décrit dans l'exemple 1-1.

Le fragment HindIII-BglII de 1,6 kb a été sous-cloné dans les vecteurs M13mp18 et M13mp19, à partir du cosmide pIBV2. L' insert a été séquencé comme décrit en 1-1, en utilisant comme amorce de synthèse le primer universel ou des oligonucléotides synthétiques, complémentaires d'une séquence de 20 nucléotides de l'insert à séquencer. A partir de la séquence nucléotidique ainsi obtenue (SEQ ID n°7), il est possible de déterminer les phases ouvertes de lecture et d'identifier des gènes impliqués dans la biosynthèse des précurseurs de la PI de S. pristinaespiralis ainsi que les polypeptides codés par ces gènes. Nous avons recherché la présence de phases ouvertes de lecture au sein du fragment HindIII- BglII de 1,6 kb correspondant à la fin du gène snbA et à sa région aval, comme décrit dans l'exemple 1-1. Une phase ouverte complète codée par le même brin que le gène snbA (figure 6), a été mise en évidence. Par rapport à la position 1 correspondant au site HindIII, cette phase démarre au nucléotide 249, 30 nucléotides après la fin du gène snbA, et se termine au nucléotide 1481. Sa taille est de 1233 nucléotides correspondant à une protéine de 411 acides aminés.

La comparaison du produit de cette phase ouverte avec les séquences protéiques contenues dans la banque Genpro fait apparaître une homologie de 30 à 40 % avec un groupe de protéines probablement impliquées (Thorson et al., 1993) dans la transamination d'intermédiaires de biosynthèse de différents antibiotiques (DnrJ, EryC1, TylB, StrS, PrgL ). La synthèse du précurseur 3-HPA qui semble dériver de la lysine par une autre voie que la cyclodéamination (voir exemples 1-2 et 2-1), pourrait nécessiter une étape de transamination susceptible d'être catalysée par le produit de cette phase 3, appelé HpaA (SEQ ID n°8). Les résultats de mutation dans ce gène, présentés en 2-2, montrent sans équivoque l'implication de ce gène dans la synthèse du précurseur 3-HPA et confirment notre hypothèse.

Les gènes papB, papC, pipA, snbF et hpaA décrits dans la présente invention sont regroupés avec les gènes snbA, papA, et papM sur une région chromosomique de environ 10 kb (figure 7). Ceci confirme la présence d'un cluster de gènes impliqués dans la biosynthèse de la P I et de ses précurseurs. L'étude des régions en amont et en aval de ce cluster devrait permettre d'identifier les autres gènes de biosynthèse des précurseurs de la PI, notamment de la L-phénylglycine et de l'acide L-2-aminobutyrique.

### EXEMPLE 2: Construction de souches recombinées par disruption des gènes identifiés.

Cet exemple illustre comment il est possible de mettre en évidence l'implication des gènes décrits dans l'exemple 1, dans la biosynthèse des précurseurs des pristinamycines, ainsi que de construire des souches de S. pristinaespiralis capables de produire de nouvelles pristinamycines. Ces souches sont obtenues en disruptant les gènes impliqués dans la biosynthèse du résidu que l'on veut substituer et les nouvelles pristinamycines sont produites en complémentant ces mutants par des précurseurs originaux.

La souche SP92::pVRC508 utilisée dans la présente invention pour produire de nouveaux dérivés de PI en remplaçant le précurseur DMPAPA par d'autres molécules, est décrite dans le brevet PCT/FR93/00923 (WO94/08014). Elle résulte de la disruption par simple crossing-over du gène papA impliqué dans la biosynthèse du précurseur de la DMPAPA et supposé intervenir dans une étape précoce concernant la transamination du chorismate. Cette disruption a un caractère polaire puisque, dans ce mutant, l'expression du gène papM (PCT/FR93/00923 ou WO 94/108014), situé 1, 5 kb en aval du gène papA et impliqué dans la double méthylation de la 4-amino-L-phénylalanine en DMPAPA, est très réduite. En effet le dosage de l'activité de l'enzyme de méthylation SAM-dépendante de la 4-amino-L-phénylalanine (PAPA) en DMPAPA indique pour le mutant SP92::pVRC508 une activité inférieure à 5% de 1' activité de la souche sauvage.

Dans la présente invention cette souche SP92::pVRC508 permet dans des conditions de fermentation et de complémentation adéquates de produire de nouvelles pristinamycines, modifiées au niveau du résidu DMPAPA, comme il sera présenté dans 1' exemple 3. Des mutants de même phénotype peuvent être obtenus par disruption des gènes papB ou papC décrits dans la présente invention.

D'une manière similaire, un autre type de souche de S. pristinaespiralis,disruptée dans le gène papA et possédant le même phénotype que la souche SP92:pVRC508, a été obtenue par disruption par double crossing over du gène papA. Cette construction a été réalisée à partir d'un fragment SphI-HindIII de 4,6kb, isolé du cosmide pIBV2 et contenant la région 3' du gène pipA, les gènes snbF et papA en entier ainsi que la partie 3' du gène papC. Ce fragment a été cloné dans le vecteur suicide pDH5, capable de se répliquer seulement chez E. coli, mais portant un marqueur de résistance s'exprimant chez Streptomyces (le gène de résistance au thiostrepton ou au nohiheptide, tsr). Ce vecteur pDH5 a été développé par Wohlebben et al. (1991 Nucleic Acid. Res. 19, 727-731). Une délétion BclI-BclI de 1,1 kb a ensuite été réalisée dans le gène papA et un fragment HindIII-HindIII de 2,2 kb portant le gène amR (résistance à la généticine et à l'apramycine) a été introduit après remplissage des extrémités cohésives. Le vecteur recombinant a été appelé pVRC414 et est présenté en figure 12. Après transformation de la souche productrice de pristinamycines par le plasmide pVRC414 des transformants résistants à la généticine et sensibles au thiostrepton ont été isolés et analysés. Ces clones résultent d'une double recombinaison homologue entre les régions d'ADN de S. pristinaespiralis du plasmide pVRC414 et la région chromosomique de S. pristinaespiralis correspondante telle que décrite en figure 13. Un de ces clones a été appelé SP212. Son phénotype est identique à celui de la souche SP92::pVRC508 en ce qui concerne la non production de PI et sa capacité à produire de nouveaux antibiotiques en présence de précurseurs originaux. Avantageusement, ce type de souche, obtenue par double crossing over, présente une meilleure stabilité comparativement aux souches obtenues par simple crossing-over.

### 2-1. Construction d'un mutant de S.pristinaespiralis SP92 disrupté dans le gène pipA.

Cet exemple illustre comment il est possible par disruption du gène pipA de construire une souche de S.pristinaespiralis SP92 qui ne produit plus de PI dans les conditions standards de fermentation et qui est capable de produire de nouvelles pristinamycines, modifiées au niveau du résidu acide 4-oxopipécolique de la PIA, lorsque des précurseurs originaux sont ajoutés à la fermentation.

Sa construction a été réalisée à l'aide d'un vecteur suicide se répliquant chez E.coli seulement, le vecteur pUC1318. Ce vecteur ne porte pas de marqueur de résistance s'exprimant chez Streptomyces. Sa présence dans le génome de Streptomyces ne peut être détectée que par hybridation sur colonies.

### 2-1-1. Construction du plasmide pVRC420:

Cet exemple illustre comment il est possible de construire un plasmide ne se répliquant pas chez S.pristinaespiralis SP92, qui peut être utilisé pour disrupter par double recombinaison homologue le gène pipA.

Le plasmide pVRC420 a été construit pour réaliser le mutant chromosomique SP92 disrupté dans le gène pipA à partir du cosmide pIBV2 décrit dans le brevet PCT/FR93/0923. Le cosmide pIBV2 a été coupé par l'enzyme de restriction PstI et après séparation des fragments ainsi générés par électrophorèse sur gel d'agarose à 0,8 %, un fragment PstI-PstI de 2,8 kb, contenant le début des gènes snbA et snbF et la totalité du gène pipA a été isolé et purifié par Geneclean (Bio101, La Jolla, Californie). 50 ng du vecteur pUC1318 linéarisés par une digestion PstI, ont été ligués avec 200 ng du fragment de 2,8 kb, comme décrit dans l'exemple 1. Un clone portant le fragment souhaité a été isolé après transformation de la souche TG1 et sélection sur milieu LB + ampicilline 150 µg/ml + X-gal + IPTG. Le plasmide recombinant a été nommé pVRC415 (figure 8). Une cassette contenant le gène am^{R}, codant pour la résistance à l'apramycine ou à la généticine (Kuhstoss et al., 1991) a été alors introduite au site unique HindIII du plasmide pVRC415, ce site étant situé 530 pb en aval du démarrage du gène pipA. Cette construction a été réalisée comme suit. Un fragment d'ADN de 2,5 kb contenant le gène am^{R}, le promoteur PermE (Bibb et al.,1985) ainsi que les 158 premiers acides aminés du gène de résistance à l'érythromycine, ermE a été isolé par une double digestion SalI-BglII à partir d'un plasmide dérivé des plasmides pIJ4026 (plasmide porteur du gène ermE sous contrôle du promoteur PermE) et pHP45Ωam^{R}. Après remplissage des extrémités cohésives 5' sortantes SalI et BglII par l'enzyme Klenow selon le protocole décrit par Maniatis et al. 1989, le fragment contenant le gène am^{R} a été cloné au site HindIII du plasmide pVRC415, dont les extrémités cohésives 5' sortantes avaient été également remplies par 1' enzyme Klenow, comme décrit précédemment. Le plasmide recombinant ainsi obtenu a été appelé pVRC420. Sa carte de restriction est présentée figure 9.

### 2-1-2. Isolement du mutant SP92pipA::Ωam^{R}, disrupté dans le gène pipA par recombinaison homologue.

Cet exemple illustre comment le mutant de S.pristinaespiralis SP92 disrupté dans le gène pipA a été construit.

Ce mutant a été isolé par transformation de la souche SP92 avec le plasmide suicide pVRC420.

La préparation des protoplastes, leur transformation ainsi que l'extraction d'ADN total des souches recombinées ont été réalisées comme décrit par Hopwood et al. (1985).

La souche SP92 a été cultivée en milieu YEME (Hopwood et al., 1985), 34% de sucrose, 5 mM MgCl₂, glycine 0,25% pendant 40 heures à 30°C. Le mycélium a été protoplastisé en présence de lysozyme et 5 X 1 µg de pVRC420 ont été utilisés pour la transformation (par la méthode utilisant le PEG) des protoplastes. Après une nuit de régénération des protoplastes sur milieu R2YE (D. Hopwood et al. 1985), les recombinants ont été sélectionnés par étalement de 3 ml de milieu SNA (D. Hopwood et al. 1985) contenant 1500 µg/ml de généticine.

Sur les 5 transformations réalisées 100 clones résistants à la généticine ont été isolés. Ces recombinants résultent de l'intégration par simple ou double recombinaison homologue entre le gène pipA porté par le chromosome de la souche SP92 et les parties du gène pipA contenues dans le fragment de 5,3 kb porté par le plasmide suicide pVRC420. Pour sélectionner les recombinants obtenus par double crossing-over (c'est à dire ne contenant pas dans leur génome la partie pUC1318 du plasmide pVRC420), des hybridations sur colonies de 90 clones ont été réalisées avec comme sonde le pUC19 marqué au [α-³²P]dCTP, comme décrit dans Maniatis et al. (1989). 10 clones résistants à la généticine mais n' hybridant pas le vecteur pUC19 ont été sélectionnés. Les spores des recombinants ont été isolées par étalement et croissance sur milieu HT7 + 10 µg/ml de généticine, et réétalées sur le même milieu pour obtenir des colonies isolées. Afin de vérifier la position de l'intégration du plasmide pVRC420, différents Southerns de l'ADN total de plusieurs clones recombinants, purifiés comme décrit par Hopwood et al. 1985, ont été réalisés et hybridés avec le fragment PstI-PstI de 2,8 kb utilisé comme sonde après marquage au [α-³²P]dCTP. Les résultats confirment que ces recombinants ont été obtenus par double crossing-over entre le vecteur pVRC420 et le chromosome de la souche SP92, aboutissant au remplacement du fragment PstI-PstI de 2,8 kb, contenant le gène pipA par un fragment PstI-PstI de 5,3 kb contenant le gène pipA disrupté par l'introduction du gène am^{R}. Un de ces mutants a été nommé SP92pipA::Ωam^{R}.

### 2-1-3. Production de pristinamycines par le mutant SP92pipA::Ωam^{R}.

Cet exemple illustre comment il est déterminé que le mutant de S.pristinaespiralis SP92 disrupté dans le gène pipA par l'intégration du plasmide pVR420 d'une part ne produit plus de PI dans les conditions standards de fermentation et d'autre part permet la forte production d'une forme minoritaire des composants B des streptogramines dans lesquels l'acide 4-oxopipécolique est remplacé par l'acide pipécolique.

Le mutant SP92pipA::Ωam^{R}, ainsi que la souche SP92, en tant que souche témoin, ont été cultivés en milieu de production liquide. La fermentation a été réalisée comme suit : 0,5 ml d'une suspension de spores de la souche précitée sont ajoutés en conditions stériles à 40 ml de milieu inoculum dans un erlen chicané de 300 ml. Le milieu inoculum est constitué par 10 g/l de Corn Steep, 15 g/l de saccharose, 10 g/l de (NH₄)₂SO₄, 1 g/l de K₂HPO₄, 3 g/l de NaCl, 0,2 g/l de MgSO₄-7H₂O et 1,25 g/l de CaCO₃. Le pH est ajusté à 6.9 par de la soude avant l'introduction du carbonate de calcium. Les erlens sont agités pendant 44 h à 27°C sur un agitateur rotatif à la vitesse de 325 rpm. 2,5 ml de la culture précédente âgée de 44 h sont ajoutés stérilement à 30 ml de milieu de production dans un erlen de 300 ml. Le milieu de production est constitué par 25 g/l de farine de soja, 7,5 g/l d'amidon, 22,5 g/l de glucose, 3,5 g/l de levure fourragère, 0,5 g/l de sulfate de zinc et 6 g/l de carbonate de calcium. Le pH est ajusté à 6,0 par de l'acide chlorhydrique avant l'introduction du carbonate de calcium. Les erlens sont agités pendant 24, 28 et 32 heures à 27°C. A chaque temps, 10 g de moût sont pesés dans un erlen lisse , auxquels sont ajoutés 20 ml de phase mobile composée de 34 % d'acétonitrile et 66 % d'une solution de 0,1 M de KH₂PO₄ (ajusté à pH 2,9 par H₃PO₄ concentré) permettant l'extraction des pristinamycines. Après agitation, le tout est centrifugé et les pristinamycines contenues dans le surnageant sont dosées par CLHP en injectant 150 µl du surnageant de centrifugation sur une colonne Nucléosil 5-C8 de 4,6 x 150 mm éluée par un mélange de 40 % d'acétonitrile et de 60 % de tampon phosphate 0,1 M pH 2,9. Les pristinamycines I sont détectées grâce à leur absorbance UV à 206 nm.

Les résultats ont montré que dans les conditions de fermentation réalisées, le mutant SP92pipA::Ωam^{R} n' a pas produit de PI, ceci à 24, 28 et 32 hrs de fermentation, alors que le témoin SP92 a produit pour les 3 temps testés, une quantité standard de PI. Pour les deux souches, la quantité de PII produite est restée la même. Le mutant SP92pipA::Ωam^{R} est bien bloqué dans une étape de biosynthèse de la PI. Des tests complémentaires de fermentation ont été réalisés en ajoutant au bout de 16 heures à la culture en milieu de production, les différents précurseurs de la PI, séparément ou ensemble. Les résultats de ces complémentations ont permis de montrer que lorsqu' on ajoute au milieu de fermentation 100 mg/l d' acide pipécolique et 100 mg/l de DMPAPA, simultanément, le mutant produit un dérivé normalement mineur de la PI, la PI_{E} (dont la quantité produite est inférieure à 5 % chez SP92), à un niveau équivalent à la production de PI_{A} par la souche témoin. Cette production n' a pas lieu si 1' acide pipécolique et le DMPAPA sont ajoutés séparément. La PI_{E} diffère de la PI_{A} (composant majeur de la PI) par l'absence de la fonction cétone en 4 sur l'acide pipécolique. Le fait que la complémentation du mutant SP92pipA::Ωam^{R} ne puisse être réalisée qu' en ajoutant simultanément l'acide pipécolique et la DMPAPA, indique que les gènes papA et probablement papB et papM ont été disruptés par effet polaire de la construction. En effet, tous ces gènes sont situés en aval de pipA, et sont probablement cotranscrits avec pipA. La disruption de ce dernier entraîne donc la disruption des gènes pap et par conséquence l'absence de synthèse de la DMPAPA. Le fait que la complémentation du mutant SP92pipA::Ω am^{R} par l'acide pipécolique, entraîne la production de PI_{E} et non de PI_{A}, conduit à deux conclusions : la première est que la construction du cycle de la PI se fait par incorporation de l'acide pipécolique et non de 1' acide 4-oxopipécolique, et qu' une hydroxylation générant la fonction cétone en 4 a alors lieu ultérieurement. La deuxième est que cette hydroxylation est probablement réalisée par l'enzyme SnbF, dont le gène de structure se situe directement en aval du gène pipA. En effet, la polarité évidente de la disruption du gène pipA, sur les gènes pap, implique probablement un effet polaire sur le gène snbF, situé entre pipA et les gènes pap, qui se traduit par l'inhibition de la fonction d'hydroxylation du résidu acide pipécolique de la PI_{E} en acide 4-hydroxypipécolique retrouvé dans les PI_{F} et PI_{G} (figure 2) puis oxydé en acide 4-oxopipécolique dans la PI_{A}.

La réalisation d'un tel mutant a permis de construire une souche de S. pristinaespiralis incapable de produire de la PI sauf en présence des précurseurs de PI, DMPAPA et acide pipécolique, à partir desquels elle est capable de produire en quantité équivalente à la souche de départ un dérivé de PI normalement minoritaire dans le mélange de pristinamycine. De même en présence de précurseurs originaux ou d'un mélange de précurseurs originaux et des précurseurs normalement présents dans la PI, cette souche pourra produire de nouvelles pristinamycines, modifiées dans l'un ou l'autre ou dans les deux résidus DMPAPA et acide 4-oxopipécolique.

### 2-2. Construction d'un mutant de S.pristinaespiralis SP92 disrupté dans le gène hpaA.

Cet exemple illustre comment il est possible par disruption du gène hpaA de construire une souche de S.pristinaespiralis SP92 qui ne produit plus de PI dans les conditions standards de fermentation et qui est capable de produire de nouvelles pristinamycines, modifiées au niveau du précurseur 3-HPA, lorsque des précurseurs originaux sont ajoutés à la fermentation.

Sa construction a été réalisée à 1' aide d'un plasmide ne se répliquant pas chez S.pristinaespiralis SP92, qui peut être utilisé pour disrupter par double recombinaison homologue le gène hpaA.

### 2-2-1. construction du plasmide suicide pVRC421

La construction du plasmide pVRC421 a été réalisée à l'aide d'un vecteur suicide, capable de se répliquer chez E.coli seulement, mais portant un marqueur de résistance s'exprimant chez Streptomyces, le gène de résistance au thiostrepton ou au nosiheptide, tsr. Ce vecteur, pDH5, a été développé par Hillemann et al. (1991).

Le plasmide pVRC421 a été construit pour réaliser le mutant chromosomique SP92 disrupté dans le gène hpaA, à partir du cosmide pIBV2 décrit dans le brevet PCT/FR93/0923. Le pIBV2 a été digéré par 1' enzyme de restriction SphI et après séparation des fragments ainsi générés par électrophorèse sur gel d'agarose à 0,6 %, un fragment SphI-SphI de 4,8 kb, contenant la totalité du gène hpaA et la quasi totalité du gène snbA a été isolé et purifié par Geneclean comme décrit plus haut. 50 ng du vecteur pDH5 linéarisés par une digestion SphI, ont été ligués avec 200 ng du fragment de 4,8 kb, comme décrit ultérieurement. Un clone portant le fragment souhaité a été isolé après transformation de la souche TG1 et sélection sur milieu LB + ampicilline 150 µg/ml + IPTG + X-gal. Le plasmide recombinant a été nommé pVRC411 (figure 10). Une cassette contenant le gène am^{R} codant pour la résistance à l'apramycine ou à la généticine a été alors introduite au site unique PflmI du plasmide pVRC411, ce site étant situé 610 pb en aval du démarrage du gène hpaA. Cette construction a été réalisée comme suit. Un fragment d'ADN de 2,2 kb contenant le gène am^{R} a été isolé après digestion du plasmide pHP45Ωam^{R} contenant le gène am^{R}, par HindIII. Après remplissage des extrémités cohésives 5' sortantes HindIII par l'enzyme Klenow selon le protocole décrit par Maniatis et al. 1989, le fragment contenant le gène am^{R} a été cloné au site PflmI du plasmide pVRC411, dont les extrémités cohésives 3' sortantes avaient été rendues franches par l'enzyme T4 polymérase, comme décrit dans Maniatis et al. 1989. Le plasmide recombinant ainsi obtenu a été appelé pVRC421. Sa carte de restriction est présentée figure 11.

### 2-2-2. Isolement du mutant SP92hpaA::Ωam^{R}, disrupté dans le gène hpaA par recombinaison homologue.

Cet exemple illustre comment le mutant de S.pristinaespiralis SP92, disrupté dans le gène hpaA a été construit.

Ce mutant a été isolé par transformation de la souche SP92 avec le plasmide suicide pVRC421.

La préparation des protoplastes et leur transformation ont été réalisées comme décrit précédemment.

La souche SP92 a été cultivée en milieu YEME, 34% de sucrose, 5 mM MgCl₂, glycine 0,25% pendant 40 heures à 30°C. Le mycélium a été protoplastisé en présence de lysozyme et 5 X 1 µg de pVRC421 ont été utilisés pour la transformation (par la méthode utilisant le PEG) des protoplastes. Après une nuit de régénération des protoplastes sur milieu R2YE, les recombinants ont été sélectionnés par étalement de 3 ml de milieu SNA contenant 1500 µg/ml de généticine.

Sur les 5 transformations réalisées 600 clones résistants à la généticine ont été isolés. Ces recombinants résultent de l'intégration par simple ou double recombinaison homologue entre le gène hpaA porté par le chromosome de la souche SP92 et le fragment de 6 kb du plasmide suicide pVRC421. Pour sélectionner les recombinants obtenus par double crossing-over (c' est à dire les clones ne contenant plus dans leur génome la partie pDH5 du plasmide pVRC421), les clones ont été repiqués sur milieu HT7 contenant 400 µg/ml de thiostrepton. 6 clones résistants à la généticine mais sensibles au thiostrepton ont été sélectionnés. Les spores des recombinants ont été isolées par étalement et croissance sur milieu HT7 + 10 µg/ml de généticine, et réétalées sur le même milieu pour obtenir des colonies isolées. Afin de vérifier la position de l'intégration du plasmide pVRC421, différents Southerns de l'ADN total des 6 clones recombinants, purifiés comme décrit par Hopwood et al. 1985, ont été réalisés et hybridés avec le fragment SphI-SphI de 4,8 kb utilisé comme sonde après marquage au [α-³²P]dCTP. Les résultats confirment que ces recombinants ont été obtenus par double crossing-over entre le vecteur pVRC421 et le chromosome de la souche SP92, aboutissant au remplacement du fragment SphI-SphI de 4,8 kb, contenant le gène hpaA par un fragment SphI-SphI de 6 kb contenant le gène hpaA disrupté par le gène am^{R}. Un de ces mutants a été nommé SP92hpaA:: Ωam^{R}.

### 2-2-3. Production de pristinamycines par le mutant SP92hpaA::Ωam^{R}.

Cet exemple illustre comment il est déterminé que le mutant de S.pristinaespiralis SP92 disrupté dans le gène hpaA par l'intégration du plasmide pVR421, ne produit plus de PI dans les conditions standards de fermentation.

Le mutant SP92hpaA::Ωam^{R}, ainsi que la souche SP92, en tant que souche témoin, ont été cultivés en milieu de production liquide. La fermentation a été réalisée comme décrit à l'exemple 2-1-3 puis les pristinamycines ont été extraites et dosées comme décrit précédemment. Les résultats ont montré que dans les conditions de fermentation réalisées, le mutant SP92hpaA::Ωam^{R} n' a pas produit de PI, ceci à 24, 28 et 32 hrs de fermentation, alors que le témoin a produit pour les 3 temps testés, une quantité standard de PI. Pour les deux souches, la quantité de PII produite est restée la même. Le mutant SP92hpaA::Ωam^{R} est bien bloqué dans une étape de biosynthèse de la PI. Des tests complémentaires de fermentation ont été réalisés en ajoutant au bout de 16 heures à la culture en milieu de production les différents précurseurs de la PI, séparément ou ensemble. Quand on ajoute au milieu de fermentation 100 mg/l d' acide 3-hydroxypicolinique, le mutant produit alors de la PI_{A} à un niveau équivalent à la production de PI par la souche témoin. Le fait que la complémentation du mutant SP92hpaA::Ωam^{R} ne puisse être réalisée qu' en ajoutant de 1' acide 3-hydroxypicolinique, montre que le gène hpaA est impliqué dans la synthèse de ce précurseur.

La construction de ce mutant a permis de réaliser une souche de S. pristinaespiralis mutée pour sa production de PI, mais qui en présence du précurseur 3-HPA est capable de produire en quantité équivalente à la souche de départ de la PI. De même que dans les exemples précédents, il est envisageable à partir d'un tel mutant et en présence de précurseurs originaux de produire de nouvelles pristinamycines modifiées au niveau du résidu acide 3-hydroxypicolinique.

### EXEMPLE 3: Production de composés de formule générale I par le mutant SP92::pVRC508.

Cet exemple illustre comment le mutant de S. pristinaespiralis SP92 disrupté dans le gène papA par l'intégration du plasmide pVRC508 est capable de synthétiser de nouvelles streptogramines en présence de précurseurs ajoutés dans le milieu de production. Ces précurseurs peuvent être des dérivés d'acides aminés et plus particulièrement de phénylalanine mais aussi d'acides α-cétocarboxyliques et plus particulièrement d'acide phénylpyruvique.

Le mutant SP92::pVRC508 a été cultivé en milieu de production liquide. La fermentation a été réalisée comme suit : 0,5 ml d'une suspension de spores de la souche précitée est ajouté en conditions stériles à 40 ml de milieu inoculum dans un erlen chicané de 300 ml. Le milieu inoculum est constitué par 10 g/l de Corn Steep, 15 g/l de saccharose, 10 g/l de (NH₄)₂SO₄, 1 g/l de K₂HPO₄, 3 g/l de NaCl, 0,2 g/l de MgSO₄-7H₂O et 1,25 g/l de CaCO₃. Le pH est ajusté à 6.9 par de la soude avant l'introduction du carbonate de calcium. Les erlens sont agités pendant 44 h à 27°C sur un agitateur rotatif à la vitesse de 325 rpm. 2.5 ml de la culture précédente âgée de 44 h sont ajoutés stérilement à 30 ml de milieu de production dans un erlen de 300 ml. Le milieu de production est constitué par 25 g/l de farine de soja, 7,5 g/l d'amidon, 22,5 g/l de glucose, 3,5 g/l de levure fourragère, 0,5 g/l de sulfate de zinc et 6 g/l de carbonate de calcium. Le pH est ajusté à 6,0 par de l'acide chlorhydrique avant l'introduction du carbonate de calcium. Les erlens sont agités à 27°C sur un agitateur rotatif à la vitesse de 325 rpm. Au bout de 16h, 1 ml d'une solution d'un des précurseurs listés dans le tableau 3 (généralement 5 ou 10 g/l) est ajouté à la culture. Celle-ci est arrêtée 8 ou 24 h plus tard. Aussitôt le moût est volumé et on lui ajoute 2 volumes de phase mobile composée de 34 % d'acétonitrile et 66 % d'une solution de 0,1 M de KH₂PO₄ (ajusté à pH 2,9 par H₃PO₄ concentré) permettant l'extraction des pristinamycines. Après agitation, le tout est centrifugé et les pristinamycines contenues dans le surnageant sont extraites et purifiées comme décrit dans l'exemple 4. Elles sont également dosées par CLHP en injectant 150 µl du surnageant de centrifugation sur une colonne Nucléosil 5-C8 de 4,6 x 150 mm éluée par un mélange de 40 % d'acétonitrile et de 60 % de tampon phosphate 0,1 M pH 2,9. Les nouvelles pristinamycines I sont détectées grâce à leur absorbance UV à 206 nm et éventuellement grâce à leur émission de fluorescence (filtre 370 nm, excitation à 306 nm).

**TABLEAU III**

| **PRECURSEUR** | **ORIGINE** |
|---|---|
| Phénylalanine | Janssen |
| 4-Diméthylaminophénylalanine | Exemple 33 |
| 4-Méthylaminophénylalanine | Exemple 34-1 |
| 4-Aminophénylalanine | Janssen 22.794.96 |
| 4-Diéthylaminophénylalanine | Exemple 33 |
| 4-Ethylaminophénylalanine | Exemple 33 |
| 4-Méthylthiophénylalanine | Exemple 33 |
| 4-Méthylphénylalanine | J.P.S101-312-4/ Exemple 33 |
| 4-Méthoxyphénylalanine | Janssen 16.975.97 |
| 4-Trifluocométhoxyphénylalanine | Exemple 34-8 |
| 4-Méthoxycarbonylphénylalanine | Exemple 33 |
| 4-Chlorophénylalanine | Janssen 15.728.14 |
| 4-Bromophénylalanine | Janssen 22.779.81 |
| 4-Iodophénylalanine | Bachem F 1675 |
| 4-Trifluorométhylphénylalanine | P.C.R. Inc. 12 445-3 |
| 4-tert-Butylphénylalanine | Exemple 35-1 |
| 4-Isopropylphénylalanine | Exemple36-1 |
| 3-Méthylaminophénylalanine | Exemple35-3 |
| 3-Méthoxyphénylalanine | J.P.S. 101-313-2 |
| 3-Méthylthiophénylalanine | Exemple 34-11 |
| 3-Fluoro 4-Méthylphénylalanine | Exemple 34-5 |
| Acide 4-tert-Butylphénylpyruvique | Exemple 33 |
| Acide 4-Méthylaminophénylpyruvique | Exemple 34-4 |
| 2-Naphtylphénylalanine | Bachem F 1865 |
| 4-Fluorophénylalanine | Bachem F 1535 |

| **PRECURSEUR** | **ORIGINE** |
|---|---|
| 3-Fluorophénylalanine | Bachem F 2135 |
| 3-Ethoxyphénylalanine | Exemple 37-1 |
| 2,4-Diméthylphénylalanine | Exemple 33 |
| 3,4-Diméthylphénylalanine | Exemple 33 |
| 3-Méthylphénylalanine | Exemple 33 |
| 4-Phénylphénylalanine | Exemple 33 |
| 4-Butylphénylalanine | Exemple 36-3 |
| 2-Thiényl-3-Alanine | Aldrich 28.728.8 |
| 3-Trifluorométhylphénylalanine | Exemple 33 |
| 3-Hydroxyphénylalanine | Aldrich T 9.039.5 |
| 3-Ethylaminophénylalanine | Exemple 35-6 |
| 4-Antinométhylphénylalanine | Exemple 33 |
| 4- Allylaminophénylalanine | Exemple 38-2 |
| 4- Diallylaminophénylalanine | Exemple 38-1 |
| 4- Allyl éthylaminophénylalanine | Exemple 39-4 |
| 4- Ethyl propylaminophénylalanine | Exemple 39-6 |
| 4- Ethyl isopropylaminophénylalanine | Exemple 39-1 |
| 4-Ethylméthylcyclopropylaminophénylalanine | Exemple 39-8 |
| 4-(1-pyrrolidinyl) phénylalanine | Exemple 40-1 |
| 4-O-allyltvrosine | Exemple 33 |
| 4-O-Ethyltyrosine | Exemple 33 |
| 4-Ethylthiophénylalanine | Exemple 33 |
| 4-Ethylthiométhylphénylalanine | Exemple 41-1 |
| 4-O-(2-Chloroéthyl) tyrosine | Exemple 42-1 |
| 4-Acétyl phénylalanine | Exemple 33 |
| 4-Ethyl phénylalanine | Exemple 33 |
| 3-Diméthylaminophénylalanine | Exemple 35-10 |

Le tableau suivant (TABLEAU IV) indique les temps de rétention relatifs des nouvelles P I produites en prenant pour référence la PI_{A}. Les temps de rétention absolus ont été déterminés à 25°C dans le système CLHP décrit ci-dessus; ils varient légèrement d'une injection à l'autre d'une part et en fonction de la température d'autre part.

**TABLEAU IV**

| **Précurseur** | **t_{R} (temps de rétention relatif) des nouvelles P I (Neo P I)** | | |
|---|---|---|---|
| | Neo P I_{A} | Neo P I_{H} | Autres neo P I |
| 4-Méthylaminophénylalanine | 0,85 | | |
| 4-Aminophénylalanine | 0.64 | | |
| 4-Méthylthiophénylalanine | 1,93 | 2,73 | 1,63 |
| 4-Méthylphénylalanine | 1,77 | 2,65 | |
| 4-Méthoxyphénylalanine | 1,46 | | |
| 4-Méthoxycarbonylphénylalanine | 1,49 | | |
| 4-Chlorophénylalanine | 2,04 | | |
| 4-Bromophénylalanine | 2,16 | | |
| 4-Iodophénylalanine | 2,42 | | |
| 4-Trifluorométhylphénylalanine | 2,56 | 3,74 | |
| 4-tert-Butylphénylalanine | 3,34 | | |
| 4-Isopropylphénylalanine | 2,80 | | 4,35 |
| 3-Méthylaminophénylalanine | 1,15 | | |
| 3-Méthoxyphénylalanine | 1,49 | 2,04 | |
| 3-Fluoro 4-Méthylphénylalanine | 2,93 | | |
| Acide 4-tert-Butylphényl-pyruvique | 3,34 | | |
| Acide 4-Méthylaminophényl-pyruvique | 0,85 | | |
| 4-Ethylarnino phénylalanine | 0,94 | | |
| 4- Diéthylaminophénylalanine | 0,61 | | |
| 4- Allylaminophénylalanine | 1,83 | | |
| 4- Diallylaminophénylalanine | 2,64 | | |
| 4- Allyl éthylaminophénylalanine | 2,4 | | |
| 4-Ethylpropylaminophénvialanine | 1,06 | | |
| 4-Ethylisopropylaminophénylalanine | 0,89 | | |
| 4-Ethylméthylcyclopropylaminophényl alanine | 1,1 | | |
| 4-(1-pyrrolidinyl) phénylalanine | 2,0 | | |
| 4-O-Trifluorométhyltyrosine | 2,42 | | |
| 4-O-allyltyrosine | 2,62 | | |
| 4-O-Ethyltyrosine | 2,2 | | |
| 4-Ethylthiophénylalanine | 1,96 | | |
| 4-Méthylthiométhylphénylalanine | 1,98 | | |
| 4-O-(2-Chloroéthyl) tyrosine | 2,45 | | |
| 4-Acétyl phénylalanine | 1,61 | | |
| 4-Ethyl phénylalanine | 1,86 | 2,40 | |
| 3-Diméthylaminophénylalanine | 1,49 | | |
| 3-Méthylthiophénylalanine | 1,93 | | |
| 3-O-Ethyltyrosine | 1,78 | | |

La nouvelle P I de t_{R} 4,35 pour la 4-Isopropylphénylalanine correspond à une neo P I_{E} décrite dans l'exemple 14.

La nouvelle P 1 de t_{R} 1,63 pour la 4-Méthylthiophénylalanine correspond à une 5γ-hydroxy neo P I_{H} décrite dans l'exemple 5.

Par ailleurs le mutant SP92::pVRC508 a été fermenté en présence de 4-diméthylamino phénylalanine. Dans ces conditions de complémentation le mutant SP92::pVRC508 produit une quantité de pristinamycines I_{A} équivalente à celle produite par la souche SP92.

### EXEMPLE 4: Préparation de la pristinamycine I_{B} [4ζ-méthylamino-dés(4ζ-diméthylamino) pristinamycine I_{A}] et de la 4ζ-amino-dés(4ζ-diméthylamino) pristinamycine I_{A}

### 4.1: Préparation de la pristinamycine I_{B} [4ζ-méthylamino-dés(4ζ-diméthylamino) pristinamycine I_{A}]

On réalise à l'échelle de 60 erlenmeyers comme décrit dans l'exemple 3 une culture de la souche SP92::pVRC508 en milieu de production avec ajout à 16h de 1 ml d'une solution à 10 g/l dans l'eau de 4-méthylaminophénylalanine (R,S) synthétisée comme à l'exemple 34-1. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la pristinamycine I_{B} sont regroupées et évaporées. Le résidu sec est repris par 6 ml d'un mélange 65% eau et 35% acétonitrile et injecté sur une colonne semi-préparative Nucléosil 7µ C8 10x250 mm (Macherey Nagel) éluée dans un mélange de 65% de tampon phosphate 100 mM pH 2,9 et 35% d'acétonitrile. Les fractions contenant la pristinamycine I_{B} sont regroupées et extraites par un volume de dichlorométhane. La phase organique est lavée à l'eau, séchée sur sulfate de sodium puis évaporée. On obtient 52 ring de pristinamycine I_{B}.

Spectre de R.M.N. ¹ H (400 MHz, CDCl₃, δ en ppm, ref. TMS): 0.71 (dd, J= 16 et 6 Hz, 1H, 5 β₂), 0.92 (t, J= 7.5 Hz, 3H: CH₃ 2 γ), de 1.10 à 1.40 (mt, 2H: 3 β₂ et 3 γ₂), 1.34 (d, J= 7.5 Hz, 3H: CH₃ 1 γ), de 1.50 à 1.85 (mt, 3H: 3 γ₁ et CH₂ 2 β), 2.03 (mt, 1H, 3 β₁), 2.22 (mt, 1H, 5 δ₂), 2.33 (d large, J= 16 Hz, 1H: 5 δ₁), 2.40 (d, J= 16 Hz, 1H: 5 β₁), 2.82 (mt, 1H: 5 ε₂), 2.81 (s, 3H: 4 NCH₃ en para du phényle), 2.90 (dd, J= 12 et 4 Hz, 1H: 4 β₂), 3.29 (s, 3H: 4 NCH₃), de 3.20 à 3.45 et 3.60 (2 mts, 1H chacun: CH₂ 3 δ), 3.40 (t, J= 12 Hz, 1H: 4 β₁), 4.57 (dd, J= 7 et 8 Hz, 1H, 3 α), 4.75 (dd large, J= 13 et 7 Hz, 1H: 5 ε₁), 4.83 (mt, 1H: 2α), 4.89 (d large, J= 10 Hz, 1H: 1α), 5.24 (dd, J=12 et 4 Hz, 1H: 4 α), 5.32 (d large, J= 6 Hz, 1H: 5 α), 5.89 (d, J= 9 Hz, 1H: 6 α), 5.90 (q large, J= 7.5 Hz, 1H: 1β), 6.53 (d, J= 9 Hz, 1H: NH 2), 6.53 (d, J= 8 Hz, 2H: 4ε), 7.03 (d, J= 8 Hz, 2H: 4δ), de 7.10 à 7.35 (mt, 5H: H Aromatiques 6), 7.46 (mt, 2H: 1' H₅ et 1' H₄), 7.85 (dd, J = 5.5 et 2 Hz, 1H: 1' H₆), 8.44 (d, J= 10 Hz, 1H: NH 1), 8.76 (d, J= 9 Hz, 1H: NH 6), 11.63 (s, 1H: OH).

### 4.2: Préparation de la 4ζ-amino-dés(4ζ-diméthylamino) pristinarriycine I_{A}

On réalise à l'échelle de 60 erlenmeyers comme décrit dans l'exemple 3 une culture de la souche SP92::pVRC508 en milieu de production avec ajout à 16h de 1 ml d'une solution à 5 g/l dans l'eau de 4-aminophénylalanine (S). Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. Le résidu seci est repris par 6 ml d'un mélange 65% eau et 35% acétonitrile et injecté sur une colonne semi-préparative Nucléosil 7µ C8 10x250 mm (Macherey Nagel) éluée dans un mélange de 65% de tampon phosphate 100 mM pH 2,9 et 35% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 5 mg de 4ζ-amino-dés(4ζ-diméthylamino) pristinamycine I_{A.}

Spectre de R.M.N. ¹ H (400 MHz, CDCl₃, δ en ppm, ref. TMS): 0.72 (dd, J= 16 et 5.5 Hz, 1H, 5 β₂), 0.90 (t, J= 7.5 Hz, 3H: CH₃ 2 γ), de 1.10 à 1.40 (mt, 2H: 3 β₂ et 3 γ₂), 1.33 (d, J= 7.5 Hz, 3H: CH₃ 1 γ), de 1.50 à 1.85 (mt, 3H: 3 γ₁ et CH₂ 2 β), 2.02 (mt, 1H, 3 β1), 2.19 (mt, 1H, 5 δ₂), 2.33 (d large, J= 16 Hz, 1H: 5 δ₁), 2.42 (d, J=16 Hz, 1H: 5 β₁), 2.81 (dt, J= 13 et 4 Hz, 1H: 5 ε₂), 2.90 (dd, J= 12 et 4 Hz, 1H: 4 β₂), 3.24 (s, 3H: NCH₃ 4), de 3.20 à 3.40 et 3.54 (2 mts, 1H chacun: CH₂ 3 δ), 3.30 (t, J= 12 Hz, 1H: 4 β₁), 3.72 (mf, 2H: ArNH₂), 4.54 (dd, J= 7.5 et 7 Hz, 1H, 3 α), 4.73 (dd large, J= 13 et 8 Hz, 1H: 5 ε₁), 4.82 (mt, 1H: 2α), 4.89 (d large, J= 10 Hz, 1H: 1α), 5.22 (dd, J= 12 et 4 Hz, 1H: 4 α), 5.32 (d large, J= 5.5 Hz, 1H: 5 α), 5.89 (mt, 2H: 6 α et 1β), 6.51 (d, J= 9.5 Hz, 1H: NH 2), 6.61 (d, J= 8 Hz, 2H: 4ε), 6.98 (d, J= 8 Hz, 2H: 4δ), de 7.15 à 7.35 (mt, 5H: H Aromatiques 6), 7.45 (dd, J= 8.5 et 1.5 Hz, 1H: 1' H₄), 7.48 (dd, J= 8.5 et 4 Hz, 1H: 1' H₅), 7.82 (dd, J = 4 et 1.5 Hz, 1H: 1' H₆), 8.43 (d, J= 10 Hz, 1H: NH 1), 8.76 (d, J= 9.5 Hz, 1H: NH 6), 11.63 (s, 1H: OH).

### EXEMPLE 5 : Préparation de la 4ζ-méthylthio-dés(4ζ-diméthylamino) pristinamycine I_{A}, de la 4ζ-méthylthio-dés(4ζ-diméthylamino) pristinamycine I_{H} et de la 5γhydroxy 4ζ-méthylthio-dés(4ζ-diméthylamino) pristinamycine I_{H}

On réalise à l'échelle de 60 erlenmeyers comme décrit dans l'exemple 3 une culture de la souche SP92::pVRC508 en milieu de production avec ajout à 16h de 1 ml d'une solution à 10 g/l dans la soude 0,1N de 4-méthylthiophénylalanine (R,S) synthétisée comme à l'exemple 33. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 65 mg de résidu sec. Celui-ci est repris par 6 ml d'un mélange 60% eau et 40% acétonitrile et injecté en 2 fois sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 45 mg de 4ζ-méthylthio-dés(4ζ-diméthylamino) pristinamycine I_{A.}

Spectre de R.M.N. ¹H (400 MHz, CDCl₃, δ en ppm, ref. TMS): 0.68 (dd, J= 16 et 5.5 Hz, 1H, 5 β₂), 0.93 (t, J= 7.5 Hz, 3H: CH₃ 2 γ), 1.13 (mt, 1H: 3 β₂), de 1.25 à 1.40 (mt, 1H: 3 γ₂), 1.33 (d, J= 7.5 Hz, 3H: CH₃ 1 γ), de 1.55 à 1.85 (mt, 3H: 3 γ₁ et CH₂ 2 β), 2.02 (mt, 1H, 3 β₁), 2.18 (mt, 1H, 5 δ₂), 2.38 (d large, J= 16.5 Hz, 1H: 5 δ₁), 2.46 (s, 3H: SCH₃), 2.48 (d, J=16 Hz, 1H, 5 β₁), 2.85 (dt, J= 13.5 et 4 Hz, 1H: 5 ε₂), 3.00 (dd, J= 12 et 5 Hz, 1H: 4 β₂), 3.23 (s, 3H: NCH₃ 4), 3.37 (t, J= 12 Hz, 1H: 4 β₁, 3.37 et 3.58 (2 mts, 1H chacun: CH₂ 3 δ), 4.55 (t, J= 7.5 Hz, 1H, 3 α), 4.77 (dd large, J= 13.5 et 8 Hz, 1H: 5 ε₁), 4.86 (mt, 1H: 2α), 4.89 (dd, J= 10 et 1.5 Hz, 1H: 1α), 5.30 (d large, J= 5.5 Hz, 1H: 5 α), 5.32 (dd, J= 12 et 5 Hz, 1H: 4 α), 5.90 (d, J= 9.5 Hz, 1H: 6 α), 5.92 (dq, J= 7.5 et 1.5 Hz, 1H:1β), 6.55 (d, J= 9.5 Hz, 1H: NH 2), 7.13 (d, J= 8 Hz, 2H: 4δ), de 7.15 à 7.35 (mt, 5H: H Aromatiques 6), 7.19 (d, J= 8 Hz, 2H: 4ε), 7.45 (mt, 2H: 1' H₄ et 1' H₅), 7.76 (t, J = 5 Hz, 1H: 1' H₆), 8.42 (d, J= 10 Hz, 1H: NH 1), 8.76 (d, J= 9.5 Hz, 1H: NH 6), 11.65 (s, 1H: OH).

A partir des fractions issues de la colonne de silice décrite ci-dessus, contenant le nouveau dérivé de pristinamycine I_{H}, 10 mg de 4ζ-méthylthio-dés(4ζ-diméthylamino) pristinamycine I_{H} sont isolés en opérant la chromatographie sur colonne semi-préparative comme décrit ci-dessus mais en portant la proportion d'acétonitrile de la phase éluante à 50%.

Spectre de R.M.N. ¹H (400 MHz, CDCl₃, δ en ppm, ref. TMS): 0.32 (mt, 1H, 5 β₂), 0.93 (t, J= 7.5 Hz, 3H: CH₃ 2 γ), de 1.20 à 1.35 (mt, 2H: 3 β₂ et 3 γ₂), 1.30(d, J= 7.5 Hz, 3H: CH₃ 1 γ), de 1.35 à 2.05 (mt, 9H: 3 γ₁ - 3 β₁ - CH₂ 2 β - CH₂ 5 ô - CH₂ 5γ et 5 β₁), 2.44 (dt, J= 13.5 et 1.5 Hz, 1H: 5 ε₂), 2.49 (s, 3H: SCH₃), 2.99 (dd, J= 12 et 5 Hz, 1H: 4 β₂), 3.09 (dd, J= 12.5 et 12 Hz, 1H: 4 β₁), 3.54 et 3.64 (2 mts, 1H chacun: CH₂ 3 δ), 4.17 (dd, J= 7 et 6 Hz, 1H: 3 α), 4.49 (d large, J= 13.5 Hz: 1H: 5 ε₁), de 4.70 à 4.80 (mt, 3H: 2α - 5 α et 4 α), 4.84 (dd, J=10 et 1.5 Hz, 1H: 1α), 5.51 (d, J= 7 Hz, 1H: 6 α), 5.73 (mt, 1H: 1β), 6.65 (d, J= 9.5 Hz, 1H: NH 2), 7.10 (d, J= 8 Hz, 2H: 4δ), 7.22 (d, J= 8 Hz, 2H: 4ε), de 7.20 à 7.40 (mt, 7H: H Aromatiques 6 - 1' H₄ et 1' H₅), 7.87 (d, J=4 Hz, 1H: 1' H₆), 8.55 (mf, 1H: NH 6), 8.55 (d, J= 10 Hz, 1H: NH 1), 11.70 (s, 1H: OH).

A partir des fractions issues de la colonne de silice décrite ci-dessus, contenant le nouveau dérivé de pristinamycine I, 3 mg de 5γ-hydroxy 4ζ-méthylthio-dés(4ζ-diméthylamino) pristinamycine ^{I} H sont isolés en opérant la chromatographie sur colonne semi-préparative comme décrite ci-dessus en gardant la proportion d'acétonitrile de la phase éluante à 45%.

Spectre de R.M.N. ¹ H (400 MHz, CDCl₃, δ en ppm, ref. TMS): on observe un isomère nettement majoritaire: le -OH en 5 γ en position axiale. 0.37 (d mt, J= 16 Hz, 1H, 5 β₂), 0.93 (t, J= 7.5 Hz, 3H: CH₃ 2 γ), de 1.20 à 1.45 (mt, 2H: 3 β₂ et 3 γ₂), 1.31 (d, J= 7.5 Hz, 3H: CH₃ 1 γ), de 1.40 à 1.85 (mt, 5H: 3 γ₁ - CH₂ 2 β et CH₂ 5 δ), 1.98 (mt, 1H, 3 β₁), 2.17 (d, J= 16 Hz, 1H: 5 β₁), 2.50 (s, 3H: SCH₃), 2.77 (dt, J= 13.5 et 2 Hz, 1H: 5 ε₂), 2.99 (dd, J= 12 et 4 Hz, 1H: 4 β₂), 3.11 (t, J= 12 Hz, 1H: 4 β₁), de 3.45 à 3.70 (mt, 2H: CH₂ 3 δ), 3.73 (mt, 1H: 5 γ en position équatoriale), 4.13 (t, J= 7 Hz, 1H, 3 α), 4.37 (d large, J= 13.5 Hz, 1H: 5 ε₁), de 4.75 à 4.95 (mt, 3H: 2α - 4 α et 5 α), 4.89 (dd, J= 10 et 1 Hz, 1H: 1α), 5.70 (d, J= 8 Hz, 1H: 6 α), 5.80 (dq, J= 7.5 et 1 Hz, 1H: 1β), 6.37 (d, J= 5 Hz, 1H: NH 4), 6.71 (d, J= 10 Hz, 1H: NH 2), 7.10 (d, J= 8 Hz, 2H: 4δ), 7.22(d, J= 8 Hz, 2H: 4 ε), de 7.20 à 7.40 (mt, 5H: H Aromatiques 6), 7.43 (dd, J= 8.5 et 1.5 Hz, 1H: 1' H₄), 7.47 (dd, J= 8.5 et 4 Hz, 1H: 1'H₅). 7.89 (dd, J = 4 et 1.5 Hz, 1H: 1'H₆), 8.55 (d, J= 10 Hz, 1H: NH 1), 9.15 (d, J= 8 Hz, 1H: NH 6), 11.70 (s, 1H: OH).

### EXEMPLE 6: Préparation de la 4ζ-méthyl-dés(4ζ-diméthylamino) pristinamycine I_{A} et de la 4ζ-méthyl-dés(4ζ-diméthylamino) pristinamycine I_{H}.

On réalise à l'échelle de 60 erlenmeyers comme décrit dans l'exemple 3 une culture de la souche SP92::pVRC508 en milieu de production avec ajout à 16h de 1 ml d'une solution à 5 g/l dans la soude 0.1N de 4-méthylphénylalanine (R,S). Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 49 mg de résidu sec. Celui-ci est repris par 6 ml d'un mélange 60% eau et 40% acétonitrile et injecté en 2 fois sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 44 mg de 4ζ-méthyl-dés(4ζ-diméthylamino) pristinamycine I_{A}.

Spectre de R.M.N. ¹H (400 MHz, CDCl₃, ô en ppm, ref. TMS): 0.52 (dd, J= 16 et 6 Hz, 1H, 5 β₂), 0.93 (t, J= 7.5 Hz, 3H: CH₃ 2 γ), 1.15 (mt, 1H: 3 β₂), del.20 à 1.40 (mt, 1H: 3 γ₂), 1.35 (d, J= 7.5 Hz, 3H: CH₃ 1 γ), de 1.50 à 1.85 (mt, 3H: 3 γ₁ et CH₂ 2 β), 2.04 (mt, 1H, 3 β₁), 2.18 (mt, 1H, 5 δ₂), de 2.25 à 2.45 (mt, 2H: 5 δ₁ et 5 β₁), 2.36 (s, 3H: ArCH₃), 2.83 (dt, J= 13 et 4 Hz, 1H: 5 ε₂), 2.99 (dd, J= 13 et 4 Hz, 1H: 4 β₂), 3.28 (s, 3H: NCH₃4), 3.31 et 3.59 (2 mts, 1H chacun: CH₂ 3 δ), 3.40 (t, J= 13 Hz, 1H: 4 β₁), 4.59 (t, J= 7.5 Hz, 1H, 3 α), 4.74 (dd large, J= 13 et 7 Hz, 1H: 5 ε₁), 4.85 (mt, 1H: 2α), 4.89 (d large, J= 10 Hz, 1H: 1α), de 5.25 à 5.35 (mt, 2H: 5 α et 4 α), de 5.85 à 5.95 (mt, 2H: 6 α et 1β), 6.52 (d, J= 9.5 Hz, 1H: NH 2), 7.14 (AB limite, J= 9 Hz, 4H: 4δ et 4ε), de 7.15 à 7.35 (mt, 5H: H Aromatiques 6), 7.50 (mt, 2H: 1'H₄ et 1'H₅), 7.81 (dd, J = 4 et 2Hz, 1H: 1'H₆), 8.41 (d, J= 10 Hz, 1H: NH 1), 8.74 (d, J= 9 Hz, 1H: NH 6), 11.63 (s, 1H: OH).

A partir des fractions issues de la colonne de silice décrite ci-dessus, contenant le nouveau dérivé de pristinamycine I_{H}, 21 mg de 4ζ-méthyl-dés(4-diméthylamino) pristinamycine I_{H} (spectrométrie de masse: M+H⁺= 810) sont isolés en opérant la chromatographie sur colonne semi-préparative comme décrit ci-dessus.

### EXEMPLE 7: Préparation de la 4ζ-méthoxy-dés(4ζ-diméthylamino) pristinamycine I_{A}.

On réalise à l'échelle de 12 erlenmeyers comme décrit dans l'exemple 3 une culture de la souche SP92::pVRC508 en milieu de production avec ajout à 16h de 1 ml d'une solution à 5 g/l dans la soude 0.1N de 4-méthoxyphénylalanine (R,S). Au terme de 40 h de culture, les 0,35 litres de moût issus de 12 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 14 mg de résidu sec. Celui-ci est repris par 3 ml d'un mélange 60% eau et 40% acétonitrile et injecté sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 12 mg de 4ζ-méthoxy-dés(4ζ-diméthylamino) pristinamycine I_{A}.

Spectre de R.M.N. ¹ H (400 MHz, CDCl₃, d en ppm, ref. TMS): 0.63 (dd, J= 16 et 5.5 Hz, 1H, 5 β₂), 0.96 (t, J= 7.5 Hz, 3H: CH₃ 2 γ),1.17 (mt, 1H: 3 β₂), de1.30 à 1.45 (mt, 1H: 3 γ₂). 1.38 (d, J= 7.5 Hz, 3H: CH₃ 1 γ), de 1.55 à 1.85 (mt, 3H: 3 γ₁ et CH₂ 2 β), 2.05 (mt, 1H, 3 β₁), 2.20 (mt, 1H, 5 δ₂). 2.40 (d large, J= 16 Hz, 1H: 5 δ₁), 2.47 (d, J= 16 Hz, 1H: 5 β₁), 2.88 (dt, J= 13 et 4 Hz, 1H: 5 ε₂), 2.99 (dd, J= 12.5 et 5 Hz, 1H: 4 β₂), 3.30 (s, 3H: NCH₃ 4), 3.32 et 3.60 (2 mts, 1H chacun: CH₂ 3 δ), 3.40 (t, J= 12.5 Hz, 1H: 4 β₁), 3.80 (s, 3H: OCH₃), 4.60 (t, J= 7.5 Hz, 1H, 3 α), 4.80 (dd large, J= 13 et 8.5 Hz, 1H: 5 ε₁). 4.88 (mt, 1H: 2α), 4.92 (d large, J= 10 Hz, 1H: 1α), 5.31 (dd, J= 12.5 et 5 Hz, 1H: 4 α), 5.34 (d large, J= 5.5 Hz, 1H: 5 α), 5.90(d, J= 9 Hz, 1H: 6 α), 5.93 (q large, J= 7.5 Hz,1H: 1β), 6.54 (d, J= 9 Hz, 1H: NH 2), 6.87 (d, J= 8 Hz, 2H: 4ε), 7.16 (d, J= 8 Hz, 2H: 4δ), de 7.15 à 7.40 (mt, 5H: H Aromatiques 6), 7.50 (mt, 2H: 1' H₅ et 1' H₄), 7.80 (dd, J = 4 et 2.5 Hz, 1H: l' H₆), 8.43 (d, J= 10 Hz, 1H: NH 1), 8.78 (d, J= 9 Hz, 1H: NH 6), 11.65 (s, 1H: OH).

### EXEMPLE 8: Préparation de la 4ζ-méthoxycarbonyl-dés(4ζ-diméthylamino) pristinamycine I_{A}.

On réalise à l'échelle de 60 erlenmeyers comme décrit dans l'exemple 3 une culture de la souche SP92::pVRC508 en milieu de production avec ajout à 16h de 1 ml d'une solution à 10 g/l de 4-méthoxycarbonylphénylalanine (R,S) synthétisée comme à l'exemple 33. Au terme de 24 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloroméhane et injecté sur une colonne de silice (30g) montée dans le dichloroméhane et éluée successivement par paliers de 0 à 10 % de méthanol dans le dichlorométhane. Les fractions contenant le nouveau dérivé de pristinamycine I_{A} sont regroupées et évaporées. On obtient 14 mg de résidu sec. Celui-ci est repris par 3 ml d'un mélange 60% eau et 40% acétonitrile et injecté sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 9 mg de 4ζ-méthoxycarbonyl-dés(4ζ-diméthylamino) pristinamycine I_{A}.

Spectre de R.M.N. ¹H (400 MHz, CDCl₃, δ en ppm, ref. TMS): 0.70 (dd, J=16 et 6 Hz, 1H, 5 β₂), 0.93 (t, J= 7.5 Hz, 3H: CH₃ 2 γ), 1.08 (mt, 1H: 3 β₂), de 1.30 à 1.40 (mt, 1H: 3 γ₂), 1.33 (d, J= 7.5 Hz, 3H: CH₃ 1 γ), de 1.55 à 1.85 (mt, 3H: 3 γ₁ et CH₂ 2 β), 2.02 (mt, 1H, 3 β₁), 2.13 (mt, 1H, 5 δ₂), 2.40 (d large, J= 16.5 Hz, 1H: 5 δ₁), 2.48 (d, J=16 Hz, 1H, 5 β₁), 2.89 (dt, J= 14.5 et 4.5 Hz, 1H: 5 ε₂), 3.10 (dd, J= 13.5 et 6 Hz, 1H: 4 β₂), 3.24 (s ,3H: NCH₃ 4), 3.38 et 3.61 (2 mts, 1H chacun: CH₂ 3 δ), 3.47 (t, J= 13.5 Hz, 1H: 4 β₁), 3.96 (s, 3H: COOCH₃). 4.55 (t, J= 7.5 Hz, 1H, 3 α), 4.78 (dd large, J= 14.5 et 8 Hz, 1H: 5 ε₁), 4.86 (mt, 1H: 2α), 4.89 (d large, J= 10 Hz, 1H: 1α), 5.33 (d large, J= 6 Hz, 1H: 5 α), 5.42 (dd, J= 13.5 et 6 Hz, 1H: 4 α), 5.92 (d (J= 9.5 Hz) et mt, 1H chacun: respectivement 6 α et 1β), 6.52 (d, J= 10 Hz, 1H: NH 2), de 7.15 à 7.35 (mt, 5H: H Aromatiques 6), 7.28 (d, J= 8 Hz, 2H: 4δ), 7.43 (dd, J= 9 et 1.5 Hz, 1H: 1' H₄), 7.47 (dd, J= 9 et 5 Hz, 1H: l' H₅), 7.66 (d, J = 5 et 1.5 Hz, 1H: 1' H₆), 7.98 (d, J= 8 Hz, 2H: 4ε), 8.38 (d, J= 10 Hz, 1H: NH 1), 8.76 (d, J= 9.5 Hz, 1H: NH 6), 11.70 (s, 1H: OH).

### EXEMPLE 9: Préparation de la 4ζ-chloro-dés(4ζ-diméthylamino) pristinamycine I_{A}.

On réalise à l'échelle de 60 erlenmeyers comme décrit dans l'exemple 3 une culture de la souche SP92::pVRC508 en milieu de production avec ajout à 16h de 1 ml d'une solution à 10 g/l dans la soude 0,1N de 4-chlorophénylalanine (R,S). Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. Le résidu sec est repris par 3 ml d'un mélange 60% eau et 40% acétonitrile et injecté sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 1 mg de 4ζ-chloro-dés(4ζ-diméthylamino) pristinamycine I_{A}.

Spectre de R.M.N. ¹H (400 MHz, CDCl₃, δ en ppm, ref. TMS): 0.93 (t, J= 7.5 Hz, 3H: CH₃ 2 γ), 0.95 (dd, J= 16 et 5 Hz, 1H, 5 β₂), 1.09 (mt, 1H: 3 β₂), de 1.20 à 1.40 (mt, 1H: 3 γ₂),1.35 (d, J= 7.5 Hz, 3H: CH₃ 1 γ), de 1.50 à 1.85 (mt, 3H: 3 γ₁ et CH₂ 2 β), 2.02 (mt, 1H, 3 β₁), 2.17 (mt, 1H, 5 δ₂), 2.43 (d large, J= 16 Hz, 1H: 5 δ₁), 2.59 (d, J=16 Hz, 1H, 5 β₁), 2.90 (dt, J= 13.5 et 4 Hz, 1H: 5 ε₂), 3.04 (dd, J= 13 et 6 Hz, 1H: 4 β₂), 3.21 (s, 3H: 4 NCH₃), 3.36 (t, J=13 Hz, 1H: 4 β₁), 3.39 et 3.59 (2 mts, 1H chacun: CH₂ 3 δ), 4.53 (t, J= 7.5 Hz, 1H, 3 α), 4.76 (dd large, J= 13.5 et 8 Hz, 1H: 5 ε₁), 4.86 (mt, 1H: 2α), 4.87(d large, J= 10 Hz, 1H: 1α), 5.38 (mt, 2H: 5 α et 4 α), 5.93 (mt, 2H: 6 α et 1β), 6.52 (d, J= 10 Hz, 1H: NH 2), 7.12 (d, J = 8 Hz, 2H: 4δ), de 7.15 à 7.35 (mt, 7H: H Aromatiques 6 et 4ε), 7.38 (dd, J= 9 et 4.5 Hz, 1H:1' H₅), 7.43 (d large, J= 9 Hz, 1H:1' H₄), 7.68 (dd, J = 4.5 et 1 Hz, 1H: 1' H₆), 8.36 (d, J=10 Hz, 1H: NH 1), 8.75 (d, J= 9 Hz,1H: NH 6), 11.65 (s, 1H: OH).

### EXEMPLE 10: Préparation de la 4ζ-bromo-dés(4ζ-diméthylamino) pristinamycine I_{A} et de la 4ζ-bromo-dés(4ζ-diméthylamino) pristinamycine I_{H}.

On réalise à l'échelle de 60 erlenmeyers comme décrit dans l'exemple 3 une culture de la souche SP92::pVRC508 en milieu de production avec ajout à 16h de 1 ml d'une solution à 10 g/l dans la soude 0.1N de 4-bromophénylalanine (R,S). Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichloromethane et éluée successivement par paliers de 0 à 10 % de méthanol dans le dichlorométhane. Les fractions contenant le nouveau dérivé de pristinamycine I_{A} sont regroupées et évaporées. Le résidu sec est repris par 6 ml d'un mélange 60% eau et 40% acétonitrile et injecté en 2 fois sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 6 mg de 4ζ-bromo-dés(4ζ-diméthylamino) pristinamycine I_{A.}

Spectre de R.M.N. ¹H (400 MHz, CDCl₃, ô en ppm, ref. TMS): 0.93 (t, J= 7.5 Hz, 3H: CH₃ 2 γ), 0.95 (dd, J= 16 et 5 Hz, 1H, 5 β₂), 1.10 (mt, 1H: 3 β₂), 1.35 (d, J= 7.5 Hz, 3H: CH₃ 1 γ), 1.36 (mt, 1H: 3 γ₂), de 1.50 à 1.85 (mt, 3H: 3 γ₁ et CH₂ 2 β), 2.02 (mt, 1H, 3 β₁), 2.18 (mt, 1H, 5 δ₂), 2.43 (d large, J= 16 Hz, 1H: 5 δ₁), 2.59 (d, J=16 Hz, 1H, 5 β₁), 2.90 (dt, J= 13 et 4 Hz, 1H: 5 ε₂), 3.02 (dd, J= 13 et 5.5 Hz, 1H: 4 β₂), 3.21 (s, 3H: 4 NCH₃), 3.33 (dd, J= 13 - 11 Hz, 1H: 4 β₁), 3.39 et 3.59 (2 mts, 1H chacun: CH₂ 3 δ), 4.53 (t, J= 7.5 Hz, 1H, 3 α), 4.76 (dd large, J= 13 et 7 Hz, 1H: 5 ε₁), 4.86 (mt, 1H: 2α), 4.89 (d large, J= 10 Hz, 1H: 1α), 5.37 (d large, J= 5 Hz, 1H: 5 α), 5.39 (dd, J= 11 et 5.5 Hz, 1H: 4 α), 5.92 (mt, 2H: 6 α et 1β), 6.56 (d, J= 9.5 Hz, 1H: NH 2), 7.08 (d, J = 8 Hz, 2H: 4δ), de 7.15 à 7.35 (mt, 5H: H Aromatiques 6), 7.40 (mt, 4H: 1' H₄ - 1' H₅ et 4ε), 7.70 (d large, J = 5 Hz, 1H: 1' H₆), 8.40 (d, J= 10 Hz, 1H: NH 1), 8.77 (d, J= 9 Hz, 1H: NH 6), 11.68 (s, 1H: OH).

A partir des fractions issues de la colonne de silice décrite ci-dessus, contenant le nouveau dérivé de pristinamycine I_{H}, 3 mg de 4ζ-bnomo-dés(4ζ-diméthylamino) pristinamycine I_{H} (spectrométrie de masse: M+H⁺= 874) sont isolés en opérant la chromatographie sur colonne semi-préparative comme décrit ci-dessus.

### EXEMPLE 11: Préparation de la 4ζ-iodo-dés(4ζ-diméthylamino) pristinamycine I_{A} et de la 4ζ-iodo-dés(4ζ-diméthylamino) pristinamycine I_{H}.

On réalise à l'échelle de 60 erlenmeyers comme décrit dans l'exemple 3 une culture de la souche SP92::pVRC508 en milieu de production avec ajout à 16h de 1 ml d'une solution à 10 g/l dans la soude de 4-iodophénylalanine (R,S). Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichloromethane et éluée successivement par paliers de 0 à 10 % de méthanol dans le dichlorométhane. Les fractions contenant le nouveau dérivé de pristinamycine I_{A} sont regroupées et évaporées. Le résidu sec est repris par 6 ml d'un mélange 60% eau et 40% acétonitrile et injecté en 2 fois sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 12 mg de 4ζ-iodo-dés(4ζ-diméthylamino) pristinamycine I_{A}.

Spectre de R.M.N. ¹H (400 MHz, CDCl₃, δ en ppm, ref. TMS): 0.93 (t, J= 7.5 Hz, 3H: CH₃ 2 γ), 0.95 (dd, J= 16 et 5.5 Hz, 1H, 5 β₂), 1.10 (mt, 1H: 3 β₂), 1.35 (d, J= 7.5 Hz, 3H: CH₃ 1 γ), 1.38 (mt, 1H: 3 γ₂), de 1.55 à 1.85 (mt, 3H: 3 γ₁ et CH₂ 2 β), 2.02 (mt, 1H, 3 β₁), 2.17 (mt, 1H, 5 δ₂), 2.43 (d large, J= 16.5 Hz, 1H: 5 δ₁), 2.60 (d, J=16 Hz, 1H, 5 β₁), 2.89 (dt, J= 14 et 4.5 Hz, 1H: 5 ε₂), 3.02 (dd, J= 13 et 5.5 Hz, 1H: 4 β₂), 3.21 (s, 3H: NCH₃ 4), 3.31 (dd, J= 13 et 11 Hz, 1H: 4 β₁), 3.39 et 3.59 (2 mts, 1H chacun: CH₂ 3 δ), 4.53 (t, J= 7.5 Hz, 1H, 3 α), 4.75 (dd large, J= 14 et 8 Hz, 1H: 5 ε₁), 4.83 (mt, 1H: 2α), 4.88 (d large, J= 10 Hz, 1H: 1α), 5.37 (d large, J= 5.5 Hz, 1H: 5 α), 5.39 (dd, J= 11 et 5.5 Hz, 1H: 4 α), 5.92 (mt, 2H: 6 α et 1β), 6.54 (d, J= 9.5 Hz, 1H: NH 2), 6.94 (d, J = 7.5 Hz, 2H: 4δ), de 7.15 à 7.50 (mt, 5H: H Aromatiques 6), 7.36 (dd, J = 9 et 4 Hz, 1H: 1' H₅), 7.43 (d large, J= 9 Hz, 1H: 1' H₄), 7.62 (d, J= 7.5 Hz, 2H: 4ε), 7.68 (d, J = 4 Hz, 1H: 1' H₆), 8.38 (d, J= 10 Hz, 1H: NH 1), 8.76 (d, J= 9 Hz, 1H: NH 6), 11.60 (s, 1H: OH).

A partir des fractions issues de la colonne de silice décrite ci-dessus, contenant le nouveau dérivé de pristinamycine I_{H}, 6 mg de 4ζ-iodo-dés(4ζ-diméthylamino) pristinamycine I_{H} (spectrométrie de masse: M+H⁺= 922) sont isolés en opérant la chromatographie sur colonne semi-préparative comme décrit ci-dessus.

### EXEMPLE 12: Préparation de la 4ζ-trifluorométhyl-dés(4ζ-diméthylamino) pristinamycine I_{A} et de la 4ζ-trifluorométhyl-dés(4ζ-diméthylamino) pristinamycine I_{H}.

On réalise à l'échelle de 60 erlenmeyers comme décrit dans l'exemple 3 une culture de la souche SP92::pVRC508 en milieu de production avec ajout à 16h de 1 ml d'une solution à 5 g/l dans la soude 0,1N de 4-trifluorométhylphénylalanine (S). Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. Le résidu sec est repris par 3 ml d'un mélange 60% eau et 40% acétonitrile et injecté sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 5 mg de 4ζ-trifluorométhyl-dés(4ζ-diméthylamino) pristinarnycine I_{A.}

Spectre de R.M.N. ¹ H (400 MHz, CDCl₃, δ en ppm, ref. TMS): 0.86 (dd, J=16 et 5.5 Hz, 1H, 5 β₂), 0.91 (t, J= 7.5 Hz, 3H: CH₃ 2 γ), 1.13 (mt, 1H: 3 β₂), 1.31 (d, J= 7.5 Hz, 3H: CH₃ 1 γ), 1.42 (mt, 1H: 3 γ₂), de 1.55 à 1.80 (mt, 3H: 3 γ₁ et CH₂ 2 β), 2.02 (mt, 1H, 3 β₁), 2.15 (mt, 1H, 5 δ₂), 2.40 (d large, J= 16.5 Hz, 1H: 5 δ₁). 2.55 (d, J=16 Hz, 1H, 5 β₁), 2.88 (dt, J= 14 et 4 Hz, 1H: 5 ε₂), 3.18 (s, 3H: NCH₃ 4), 3.20 et 3.31 (2 dd, respectivement J=13 et 6 Hz et J= 13 et 10 Hz,, 1H chacun: 4 β₂ et 4 β₁), 3.42 et 3.60 (2 mts, 1H chacun: CH₂ 3 δ), 4.50 (t, J= 7.5 Hz, 1H, 3 α), 4.73 (dd large, J=14 et 7.5 Hz, 1H: 5 ε₁), 4.83 (mt, 1H: 2α), 4.91 (d large, J= 10 Hz, 1H: 1α), 5.40 (d large, J= 5.5 Hz, 1H: 5 α), 5.55 (dd, J= 10 et 6 Hz, 1H: 4 α), 5.87 (d, J= 9.5 Hz, 1H: 6 α), 5.90 (q large, J= 7.5 Hz, 1H:1β), 6.68 (d, J= 9.5 Hz, 1H: NH 2), de 7.15 à 7.40 (mt, 9 H: 4δ - H Aromatiques 6 - 1' H₅ et 1' H₄), 7.52 (d, J= 8 Hz, 2H: 4ε), 7.68 (d, J = 4 et 1.5 Hz, 1H: 1' H₆), 8.43 (d, J=10 Hz, 1H: NH 1), 8.76 (d, J= 9.5 Hz, 1H: NH 6), 11.70 (s, 1H: OH).

A partir des fractions issues de la colonne de silice décrite ci-dessus, contenant le nouveau dérivé de pristinamycine I_{H}, 4 mg de ζ-trifluorométhyl-dés(4ζ-diméthylamino) pristinamycine I_{H} (spectrométrie de masse: M+H⁺= 864) sont isolés en opérant la chromatographie sur colonne semi-préparative comme décrit ci-dessus.

### EXEMPLE 13: Préparation de la 4ζ-tert-butyl-dés(4ζ-diméthylamino) pristinamycine I_{A}.

On réalise à l'échelle de 60 erlenmeyers comme décrit dans l'exemple 3 une culture de la souche SP92::pVRC508 en milieu de production avec ajout à 16h de 1 ml d'une solution à 5 g/l dans la soude 0,1N de 4-tert-butylphénylalanine(R,S) synthétisée comme à l'exemple 35-1. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichloromethane et éluée successivement par paliers de 0 à 10 % de méthanol dans le dichlorométhane. Les fractions contenant le nouveau dérivé de pristinamycine I_{A} sont regroupées et évaporées. Le résidu sec est repris par 7 ml d'un mélange 60% eau et 40% acétonitrile et injecté en 2 fois sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 30 mg de 4ζ-tert-butyl-dés(4ζ-diméthylamino) pristinamycine I_{A}.

Spectre de R.M.N. ¹ H (400 MHz, CDCl₃, δ en ppm, ref. TMS, ref. TMS): 0.21 (dd, J=16 et 5.5 Hz, 1H, 5 β₂), 0.91 (t, J= 7.5 Hz, 3H: CH₃ 2 γ), 1.17 (mt, 1H: 3 β₂), de1.20 à 1.40 (mt, 1H: 3 γ₂), 1.33 (s, 9H: CH₃ du tert-butyle), 1.35 (d, J= 7.5 Hz, 3H: CH₃ 1 γ), de 1.50 à 1.85 (mt, 3H: 3 γ₁ et CH₂ 2 β), 2.04 (mt, 1H, 3 β₁), 2.13 (mt, 1H, 5 δ₂), 2.30 (mt, 2H: 5 δ₁ et 5 β₁), 2.80 (dt, J= 13 et 4 Hz, 1H: 5 ε₂), 3.00 (dd, J= 12 et 4 Hz, 1H: 4 β₂), 3.29 (s, 3H: NCH₃ 4), 3.31 et 3.59 (2 mts, 1H chacun: CH₂ 3 δ), 3.40 (t, J=12 Hz, 1H: 4 β₁), 4.57 (t, J= 7.5 Hz, 1H, 3 α), 4.74 (dd large, J= 13 et 7 Hz, 1H: 5 ε₁), 4.85 (mt, 1H: 2α), 4.90 (d large, J=10 Hz, 1H: 1α), 5.21 (d large, J= 5.5 Hz, 1H: 5 α), 5.25 (dd, J= 12 et 4 Hz, 1H: 4 α), 5.87(d, J= 9 Hz, 1H: 6 α), 5.92 (q large, J= 7.5 Hz, 1H: 1 1H: 1'H₆), 8.45 (d, J= 10 Hz, 1H: NH 1), 8.74 (d, J= 9 Hz, 1H: NH 6), 11.65 (s, 1H: OH).

### EXEMPLE 14: Préparation de la 4ζ-isopropyl-dés(4ζ-diméthylamino) pristinamycine I_{A}. et de la 4ζ-isopropyl-dés(4ζ-diméthylamino) pristinamycine I_{E}.

On réalise à l'échelle de 60 erlenmeyers comme décrit dans l'exemple 3 une culture de la souche SP92::pVRC508 en milieu de production avec ajout à 16h de 1 ml d'une solution à 10 g/l dans la soude 0,1N de 4-isopropylphénylalanine (R,S) synthétisée comme à l'exemple 36-1. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichloromethane et éluée successivement par paliers de 0 à 10 % de méthanol dans le dichlorométhane. Les fractions contenant le nouveau dérivé de pristinamycine I_{A} sont regroupées et évaporées. On obtient 61 mg de résidu sec. Celui-ci est repris par 9 ml d'un mélange 60% eau et 40% acétonitrile et injecté en 3 fois sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 51 mg de 4ζ-isopropyl-dés(4ζ-diméthylamino) pristinamycine I_{A.}

Spectre de R.M.N. ¹H (250 MHz, CDCl₃, δ en ppm, ref. TMS, ref. TMS): 0.31 (dd, J=16 et 5.5 Hz, 1H, 5 β₂), 0.91 (t, J= 7.5 Hz, 3H: CH₃ 2 γ), de 1.00 à 1.45 (mt, 2H: 3 β₂ et 3 γ₂), 1.25 (d, J= 7.5 Hz, 6H: CH₃ de l' isopropyle), 1.35 (d, J= 7.5 Hz, 3H: CH₃ 1 γ), de 1.50 à 1.85 (mt, 3H: 3 γ₁ et CH₂ 2 β), de 1.95 à 2.20 (mt, 2H, 3 β₁ et 5 δ₂), 2.30 (mt, 2H: 5 δ₁ et 5 β₁), 2.80 (dt, J= 13 et 4 Hz, 1H: 5 ε₂), 2.88 (mt, 1H: CH de l' isopropyle), 2.98 (dd, J=12 et 4 Hz, 1H: 4 β₂), 3.30 (s, 3H: NCH₃ 4), 3.32 et 3.55 (2 mts, 1H chacun: CH₂ 3 δ), 3.38 (t, J= 12 Hz, 1H: 4 β₁), 4.55 (t, J= 7.5 Hz, 1H, 3 α), 4.72 (dd large, J= 13 et 7 Hz, 1H: 5 ε₁), 4.85 (mt, 1H: 2α), 4.88 (d large, J= 10 Hz, 1H: 1α), 5.21 (d large, J= 5.5 Hz, 1H: 5 α), 5.25 (dd, J= 12 et 4 Hz, 1H: 4 α), 5.87(d, J= 9 Hz, 1H: 6 α), 5.90 (q large, J= 7.5 Hz, 1H: 1β), 6.50 (d, J= 9.5 Hz, 1H: NH 2), de 7.05 à 7.35 (mt, 9H: H Aromatiques 6 - 4ε et 4δ), 7.50 (mt, 2H: 1' H₅ et 1' H₄), 7.86 (dd, J = 4 et 1.5 Hz, 1H: 1' H₆). 8.40 (d, J= 10 Hz, 1H: NH 1), 8.72 (d, J= 9 Hz, 1H: NH 6), 11.60 (s, 1H: OH).

A partir des mêmes fractions issues de la colonne de silice décrite ci-dessus, contenant également le nouveau dérivé de pristinamycine I_{E} , 5 mg de ζ-isopropyl-dés(4ζ-diméthylamino) pristinamycine I_{E} sont isolés en opérant la chromatographie sur colonne semi-préparative comme décrit ci-dessus.

Spectre de R.M.N.¹ H (400 MHz, CDCl₃, δ en ppm, ref. TMS): 0.20 (mt, 1H, 5 β₂), 0.92 (t, J= 7.5 Hz, 3H: CH₃ 2 γ), de 1.15 à 1.40 (mt, 2H: 3 β₂ et 3 γ₂), 1.24(d, J= 7.5 Hz, 6H: CH₃ de l'isopropyle), 1.34 (d, J= 7.5 Hz, 3H: CH₃ 1 γ), de 1.35 à 2.05 (mt, 9H: 3 γ₁ - 3 β₁ - CH₂ 2 β - CH₂ 5 δ - CH₂ 5γ et 5 β₁), 2.45 (dt, J= 13 et 1.5 Hz, 1H: 5 ε₂), 2.89 (mt, 1H: ArCH), 3.09 (dd, J= 14 et 7 Hz, 1H: 4 β₂), 3.17 (s, 3H: NCH₃ 4),3.25 (dd, J=14 et 9 Hz, 1H: 4 β₁), 3.32 et 3.52 (2 mts, 1H chacun: CH₂ 3 δ), 4.55 (mt, 2H: 3 α et 5 ε₁), 4.80 (mt, 1H: 2α), 4.89 (dd, J=10 et 1.5 Hz, 1H: 1α), 4.90 (mt, 1H: 5 α), 5.35 (dd, J= 9 et 7 Hz, 1H: 4 α), 5.60 (d, J= 8 Hz, 1H: 6 α), 5.89 (dq, J= 7.5 et 1.5 Hz, 1H: 1β), 6.65 (d, J= 9.5 Hz, 1H: NH 2), 7.08 (d, J= 8 Hz, 2H: 45), 7.14 (d, J= 8 Hz, 2H: 4ε), de 7.20 à 7.40 (mt, 7H: H Aromatiques 6 - 1' H₄ et 1' H₅), 7.77 (d large, J=4 Hz, 1H: 1' H₆), 8.46 (d, J= 10 Hz, 1H: NH 1), 8.48 (d, J= 8 Hz, 1H: NH 6),11.70 (s, 1H: OH).

### EXEMPLE 15 : Préparation de la 4ε-méthylamino-dés(4ζ-diméthylamino) pristinamycine I_{A}.

On réalise à l'échelle de 60 erlenmeyers comme décrit dans l'exemple 3 une culture de la souche SP92::pVRC508 en milieu de production avec ajout à 16h de 1 ml d'une solution à 10 g/l dans l'eau de 3-méthylaminophénylalanine (R,S) synthétisée comme à l'exemple 35-3. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 19 mg de résidu sec. Celui-ci est repris par 3 ml d'un mélange 60% eau et 40% acétonitrile et injecté sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchées sur sulfate de sodium puis évaporée. On obtient 8 mg de 4ε-méthylamino-dés(4ε-diméthylamino) pristinamycine I_{A.}

Spectre de RM.N. ¹ H (400 MHz, CDCl₃, δ en ppm, ref. TMS): 0.93 (t, J= 7.5 Hz, 3H: CH₃ 2 γ), 1.00 (dd, J= 16 et 6 Hz, 1H, 5 β₂), 1.17 (mt, 1H: 3 β₂), de 1.25 à 1.40 (mt, 2H: 3 γ₂), 1.35 (d, J= 7.5 Hz, 3H: CH₃ 1 γ), de 1.55 à 1.80 (mt, 3H: 3 γ₁ et CH₂ 2 β), 2.03 (mt, 1H, 3 β₁), 2.23 (mt, 1H, 5 δ₂), 2.39 (d large, J= 16 Hz, 1H: 5 δ₁), 2.52 (d, J= 16 Hz, 1H: 5 β₁), 2.78 (s, 3H: ArNCH₃ 4), 2.85 (dt, J= 13 et 4 Hz, 1H: 5 ε₂), 2.99 (dd, J= 13 et 4.5 Hz, 1H: 4 β₂), 3.23 (s, 3H: NCH₃ 4), 3.25 (t, J= 13 Hz, 1H: 4 β₁), 3.38 et 3.58 (2 mts, 1H chacun: CH₂ 3 δ), 4.05 (mf, 1H: ArNH), 4.58 (dd, J= 6.5 et 7.5 Hz, 1H, 3 α), 4.76 (dd large, J=13 et 8 Hz, 1H: 5 ε₁), 4.85 (mt, 1H: 2α), 4.87 (d large, J= 10 Hz, 1H: 1α), 5.35 (dd, J= 13 et 4.5 Hz, 1H: 4 α), 5.38 (d large, J= 6 Hz, 1H: 5 α), 5.90 (d, J=9.5 Hz, 1H: 6 α), 5.91 (mt, 1H: 1β), 6.36 (s large, 1H: H 2 de 1' aromatique en 4), de 6.45 à 6.55 (mt, 2H: H 4 et H 6 de 1' aromatique en 4), 6.53 (d, J= 10 Hz, 1H: NH 2), 7.12 (t, J= 8 Hz, 1H: H 5 de 1' aromatique en 4), de 7.15 à 7.45 (mt, 5H: H Aromatiques 6), 7.35 (mt, 2H: 1' H₄ et 1' H₅), 7.75 (t, J = 3 Hz, 1H: 1'H₆), 8.40 (d, J= 10 Hz, 1H: NH 1), 8.78 (d, J= 9.5 Hz, 1H: NH 6), 11.60 (s, 1H: OH).

### EXEMPLE 16: Préparation de la 4ε-méthoxy-dcs(4ζ-diméthylamino) pristinamycine I A et de la 4ε-méthoxy-dés(4ζ-diméthylamino) pristinamycine I H.

On réalise à l'échelle de 60 erlenmeyers comme décrit dans l'exemple 3 une culture de la souche SP92::pVRC508 en milieu de production avec ajout à 16h de 1 ml d'une solution à 5 g/l dans la soude 0.1N de 3-méthoxyphénylalanine (S). Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichloromethane et éluée successivement par paliers de 0 à 10 % de méthanol dans le dichlorométhane. Les fractions contenant le nouveau dérivé de pristinamycine IA sont regroupées et évaporées. On obtient 41 mg de résidu sec. Celui-ci est repris par 6 ml d'un mélange 60% eau et 40% acétonitrile et injecté en 2 fois sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 28 mg de 4ε-méthoxy-dés(4ζ-diméthylamino) pristinamycine I A.

Spectre de R.M.N. ¹ H (400 MHz, CDCl₃, δ en ppm, ref. TMS): 0.52 (dd, J=16 et 5.5 Hz, 1H, 5 β₂), 0.90 (t, J= 7.5 Hz, 3H: CH₃ 2 γ), de 1.10 à 1.34 (mt, 2H: 3 β₂ et 3 γ₂), 1.34 (d, J= 7.5 Hz, 3H: CH₃ 1γ), de 1.50 à 1.80 (mt, 3H: 3 γ₁ et CH₂ β), 2.04 (mt, 1H, 3 β₁), 2.20 (mt, 1H, 5 δ₂), 2.35 (d large, J= 16 Hz, 1H: 5 δ₁), 2.38 (d, J=16 Hz, 1H: 5 β₁), 2.83 (dt, J= 13 et 4 Hz, 1H: 5 ε₂), 2.97 (dd, J= 12 et 4 Hz, 1H: 4 β₂), 3.28 (s, 3H: NCH₃ 4), 3.28 et 3.56 (2 mts, 1H chacun: CH₂ 3 δ), 3.40 (t, J= 12 Hz, 1H: 4 β₁), 3.80 (s, 3H: OCH₃), 4.58 (t, J= 7.5 Hz, 1H, 3 α), 4.76 (dd large, J= 13 et 8 Hz, 1H: 5 ε₁), 4.85 (mt, 1H: 2α), 4.90 (d large, J= 10 Hz, 1H: 1α), 5.27 (dd, J= 12 et 4 Hz, 1H: 4 α), 5.30 (d large, J= 5.5 Hz, 1H: 5 α), 5.89 (d, J= 9.5Hz, 1H: 6 α), 5.91 (q large, J= 7.5 Hz, 1H: 1β), 6.51 (d, J= 10 Hz, 1H: NH 2), de 6.80 à 6.90 (mt, 3H: H 2 - H 4 et H 6 de 1' aromatique en 4), de 7.15 à 7.40 (mt, 6H: H 5 de 1' aromatique en 4 et H Aromatiques 6), 7.45 (d large, J= 9 Hz, 1H: 1' H₄), 7.50 (dd, J= 9 et 4 Hz, 1H:1' H₅), 7.80 (d large, J = 4 Hz, 1H: 1' H₆), 8.40 (d, J= 10 Hz, 1H: NH 1), 8.73 (d, J= 9.5 Hz, 1H: NH 6), 11.62 (s, 1H: OH).

A partir des fractions issues de la colonne de silice décrite ci-dessus, contenant le nouveau dérivé de pristinamycine I H, 7 mg de 4ε-méthoxy-dés(4ζ-diméthylamino) pristinamycine I H (spectrométrie de masse: M+H⁺= 826)sont isolés en opérant la chromatographie sur colonne semi-préparative comme décrit ci-dessus.

### EXEMPLE 17: Préparation de 4ε-fluoro 4ζ-méthyl-dés(4ζ-diméthylamino) pristinamycine I A.

On réalise à l'échelle de 60 erlenmeyers comme décrit dans l'exemple 3 une culture de la souche SP92::pVRC508 en milieu de production avec ajout à 16h de 1 ml d'une solution à 10 g/l dans la soude 0,1N de 3-fluoro 4-méthylphénylalanine (R,S) synthétisée comme à l'exemple 34-5. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichloromethane et éluée successivement par paliers de 0 à 10 % de méthanol dans le dichlorométhane. Les fractions contenant le nouveau dérivé de pristinamycine IA sont regroupées et évaporées. On obtient 15 mg de résidu sec. Celui-ci est repris par 3 ml d'un mélange 60% eau et 40% acétonitrile et injecté sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 9 mg de 4ε-fluoro 4ζ-méthyl-dés(4ζ-diméthylamino) pristinamycine I A.

Spectre de R.M.N. ¹H (400 MHz, CDCl₃, δ en ppm, ref. TMS): 0.60 (dd, J= 16 et 5.5 Hz, 1H, 5 β₂), 0.91 (t, J= 7.5 Hz, 3H: CH₃ 2 γ), 1.12 (mt, 1H: 3 β₂), de 1.25 à 1.35 (mt, 1H: 3 γ₂), 1.33 (d, J= 7.5 Hz, 3H: CH₃ 1 γ), de 1.50 à 1.85 (mt, 3H: 3 γ₁ et CH₂ 2 β), 2.02 (mt, 1H, 3 β₁), 2.13 (mt, 1H, 5 δ₂), 2.27 (s, 3H: ArCH₃), 2.36 (d large, J= 16 Hz, 1H: 5 δ₁), 2.45 (d, J= 16 Hz, 1H: 5 β₁), 2.85 (dt, J= 13 et 4.5 Hz, 1H: 5 ε₂), 2.97 (dd, J= 12.5 et 4.5 Hz, 1H: 4 β₂), 3.23 (s, 3H: NCH₃ 4), 3.30 et 3.56 (2 mts, 1H chacun: CH₂ 3 δ), 3.37 (t, J= 12.5 Hz, 1H: 4 β₁), 4.55 (t, J= 7.5 Hz, 1H, 3 α), 4.75 (dd large, J= 13 et 8 Hz, 1H: 5 ε₁), 4.83 (mt, 1H: 2α), 4.89 (d large, J= 10 Hz, 1H: 1α), 5.29 (dd, J= 12.5 et 4.5 Hz, 1H: 4 α), 5.32 (d large, J= 5.5 Hz, 1H: 5 α), 5.89 (d, J= 9.5 Hz, 1H: 6 α), 5.92 (mt, 1H: 1β), 6.49 (d, J= 10 Hz, 1H: NH 2), 6.90 (mt, 2H: H 2 et H 6 de l' aromatique en4), 7.11 (t, J= 8 Hz, 1H: H 5 de l' aromatique en 4), de 7.10 à 7.30 (mt, 5H: H Aromatiques 6), 7.43 (dd, J= 8.5 et 1 Hz, 1H: 1' H₄), 7.49 (dd, J= 8.5 et 4.5 Hz, 1H: 1' H₅), 7.75 (dd, J = 4.5 et 1Hz, 1H: 1' H₆), 8.48 (d, J= 10 Hz, 1H: NH 1), 8.70 (d, J= 9.5 Hz, 1H: NH 6), 11.60 (s, 1H: OH).

### EXEMPLE 18 : Préparation de la 4ζ-éthylamino-dés(4ζ-diméthylamino) pristinamycine I_{A}

On réalise à l'échelle de 50 erlenmeyers comme décrit dans l'exemple 3 une culture de la souche SP92::pVRC508 en milieu de production avec ajout à 16h de 1 ml d'une solution à 20 g/l dans la soude 0,1N de dichlorhydrate de 4-éthylaminophénylalanine (R,S) synthétisé comme à l'exemple 33. Au terme de 40 h de culture, les 1,5 litres de moût issus de 50 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-éthylamino-dés (4ζ-diméthylamino) pristinamycine I_{A} sont regroupées et évaporées. Le résidu sec est repris par 7 ml d'un mélange 65% eau et 35% acétonitrile et injecté sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la 4ζ-éthylamino-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 10 mg de 4ζ-éthylamino-dés(4ζ-diméthylamino) pristinamycine I_{A}.

Spectre de R.M.N. ¹ H (400 MHz, CDCl₃, δ en ppm, ref. TMS): 0,72 (dd, J = 16 et 6 Hz, 1H : 1H du CH₂ en 5 β) ; 0,90 (t, J = 7,5 Hz, 3H : CH₃ en 2 γ) ; 1,15 (mt, 1H : 1H du CH₂ en 3 β) ; de 1,20 à 1,40 (mt, 1H : 1H du CH₂ en 3 γ) ; 1,27 (t, J = 7,5 Hz, 3H : CH₃ de l'éthyle) ; 1,33 (d, J = 7 Hz, 3H : CH₃ en 1 γ) ; de 1,50 à 1,65 (mt, 1H : l'autre H du CH₂ en 3 γ) ; 1,60 et 1,74 (2 mts, 1H chacun : CH₂ en 2 β) ; 2,02 (mt, 1H : l'autre H du CH₂ en 3 β) ; 2,21 et 2,33 (respectivement mt et d large, J = 16,5 Hz, 1H chacun : CH₂ en 5 δ) ; 2,40 (d, J = 16 Hz, 1H : l'autre H du CH₂ en 5 (3) ; 2,82 (dt, J = 13 et 4,5 Hz, 1H : 1H du CH₂ en 5 ε) ; 2,89 (dd, J = 12 et 4 Hz, 1H : 1H du CH₂ en 4 β) ; 3,10 (mt, 2H : NCH₂ de l'éthyle) ; de 3,20 à 3,35 (mt, 1H : 1H du CH₂ en 3 δ) ; 3,26 (s, 3H : NCH₃) ; 3,31 (t, J = 12 Hz, 1H : l'autre H du CH₂ en 4 β) ; 3,54 (mt, 1H : l'autre H du CH₂ en 3 δ) ; 3,67 (mf, 1H : NH) ; 4,56 (dd, J = 6,5 et 7 Hz, 1H : 3 α) ; 4,75 (dd large, J = 13 et 8 Hz, 1H : l'autre H du CH₂ en 5 ε) ; 4,84 (mt, 1H : 2 α) ; 4,90 (d large, J = 10 Hz, 1H : 1 α) ; 5,24 (dd, J = 12 et 4 Hz, 1H : 4 α) ; 5,32 (d large, J = 6 Hz, 1H : 5 α) ; 5,88 (d, J = 9,5 Hz, 1H : 6 α) ; 5,90 (mt, 1H : 1 β) ; 6,52 (d, J = 8 Hz, 3H : NH en 2 et H Aromatiques en 4 ε) ; 7,00 (d, J = 8 Hz, 2H : H Aromatiques en 4 δ) ; de 7,10 à 7,35 (mt, 5H : H Aromatiques en 6) ; 7,46 (AB limite, 2H : 1' H₄ et 1' H₅) ; 7,84 (dd, J = 4 et 1 Hz, 1H : 1' H₆) ; 8,45 (d, J = 10 Hz, 1H : NH en 1) ; 8,77 (d, J = 9,5 Hz, 1H : NH en 6) ; 11,65 (s, 1H : OH).

### EXEMPLE 19 : Préparation de la 4ζ-diéthylamino-dés (4ζ-diméthylamino) pristinamycine I_{A}

On réalise à l'échelle de 50 erlenmeyers comme décrit dans l'exemple 3 une culture de la souche SP92::pVRC508 en milieu de production avec ajout à 16h de 1 ml d'une solution à 20 g/l dans la soude 0,1N de dichlorhydrate de 4-diéthylaminophénylalanine (R,S) synthétisé comme à l'exemple 33. Au terme de 40 h de culture, les 1,5 litres de moût issus de 50 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-diéthylamino-dés (4ζ-diméthylamino) pristinamycine I_{A} sont regroupées et évaporées. Le résidu sec est repris par 7 ml d'un mélange 60% eau et 40% acétonitrile et injecté en deux fois sur une colonne semi-préparative Nucléosil 7µ C8 10x250 mm (Macherey Nagel) éluée dans un mélange de 68% de tampon phosphate 100 mM pH 2,9 et 32% d'acétonitrile. Les fractions contenant la 4ζ-diéthylamino-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 50 mg de 4ζ-diéthylamino-dés (4ζ-diméthylamino) pristinamycine I_{A}.

Spectre de R.M.N. ¹ H (400 MHz, CDCl₃, δ en ppm, ref. TMS): 0,65 (dd, J = 16 et 6 Hz, 1H : 1H du CH₂ en 5 β) ; 0,90 (t, J = 7,5 Hz, 3H : CH₃ en 2 γ) ; 1,14 (t, J = 7 Hz, 6H : CH₃ de l'éthyle) ; 1,15 (mt, 1H : 1H du CH₂ en 3 β) ; 1,26 (mt, 1H : 1H du CH₂ en 3 γ) ; 1,32 (d, J = 6,5 Hz, 3H : CH₃ en 1 γ) ; 1,55 (mt, 1H : l'autre H du CH₂ en 3 γ) ; 1,63 et 1,75 (2 mts, 1H chacun : CH₂ en 2 β) ; 2,02 (mt, 1H : l'autre H du CH₂ en 3 β) ; 2,22 et 2,31 (respectivement mt et d large, J = 16,5 Hz, 1H chacun : CH₂ en 5 δ) ; 2,37 (d, J = 16 Hz, 1H : l'autre H du CH₂ en 5 β) ; 2,80 (dt, J = 13 et 4,5 Hz, 1H : 1H du CH₂ en 5 e) ; 2,89 (dd, J = 12,5 et 4 Hz, 1H : 1H du CH₂ en 4 β) ; de 3,20 à 3,40 (mt, 6H : NCH₂ de l'éthyle - 1H du CH₂ en 3 δ et l'autre H du CH₂ en 4 β) ; 3,27 (s, 3H : NCH₃) ; 3,55 (mt, 1H : l'autre H du CH₂ en 3 δ) ; 4,58 (dd, J = 8 et 6 Hz, 1H : 3 α) ; 4,76 (dd large, J = 13 et 7,5 Hz, 1H : l'autre H du CH₂ en 5 ε) ; 4,84 (mt, 1H : 2 α) ; 4,89 (dd, J = 10 et 1 Hz, 1H : 1 α) ; 5,21 (dd, J = 12,5 et 4 Hz, 1H : 4 α) ; 5,28 (d large, J = 6 Hz, 1H : 5 α) ; 5,87 (d, J = 9,5 Hz, 1H : 6 α) ; 5,90 (mt, 1H : 1 β) ; 6,52 (d, J = 9,5 Hz, 1H : NH en 2) ; 6,60 (d, J = 8 Hz, 2H : H Aromatiques en 4 ε) ; 7,02 (d, J = 8 Hz, 2H : H Aromatiques en 4 δ) ; de 7,10 à 7,35 (mt, 5H : H Aromatiques en 6) ; 7,46 (AB limite, 2H : 1' H₄ et 1' H₅) ; 7,88 (dd, J = 4,5 et 2,5 Hz, 1H : 1' H₆) ; 8,43 (d, J = 10 Hz, 1H : NH en 1) ; 8,76 (d, J = 9,5 Hz, 1H : NH en 6) ; 11,62 (s, 1H : OH).

### EXEMPLE 20 : Préparation de la 4ζ-diallylamino-dés(4ζ-diméthylamino) pristinamycine I_{A}

On réalise à l'échelle de 94 erlenmeyers comme décrit dans l'exemple 3 une culture de la souche SP92::pVRC508 en milieu de production avec ajout à 16h de 1 ml d'une solution à 20 g/l dans l'eau de dichlorhydrate de 4-diallylaminophénylalanine (R,S) synthétisé comme à l'exemple 38-1 . Au terme de 40 h de culture, les 2,8 litres de moût issus de 94 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-diallylamino-dés (4ζ-diméthylamino) pristinamycine I_{A} sont regroupées et évaporées. Le résidu sec est repris par 7 ml d'un mélange 60% eau et 40% acétonitrile et injecté sur une colonne semi-préparative Nucléosil 7µ C8 10x250 mm (Macherey Nagel) éluée dans un mélange de 52% de tampon phosphate 100 mM pH 2,9 et 48% d'acétonitrile. Les fractions contenant la 4ζ-diallylamino-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 15 mg de 4ζ-diallylamino-dés (4ζ-diméthylamino) prisrinamycine I_{A}.

Spectre de R.M.N. ¹ H (400 MHz, CDCl₃, δ en ppm, ref. TMS): 0,55 (dd, J = 16 et 6 Hz, 1H : 1H du CH₂ en 5 β) ; 0,93 (t, J = 7,5 Hz, 3H : CH₃ en 2 γ) ; 1,18 (mt, 1H : 1H du CH₂ en 3 β) ; 1,25 (mt, 1H : 1H du CH₂ en 3 γ) ; 1,34 (d, J = 6,5 Hz, 3H : CH₃ en 1 γ) ; 1,59 (mt, 1H : l'autre H du CH₂ en 3 γ) ; 1,68 et 1,78 (2 mts, 1H chacun : CH₂ en 2 β) ; 2,04 (mt, 1H : l'autre H du CH₂ en 3 β) ; 2,25 et 2,34 (respectivement mt et d large, J = 16,5 Hz, 1H chacun : CH₂ en 5 δ) ; 2,40 (d, J = 16 Hz, 1H : l'autre H du CH₂ en 5 β) ; 2,83 (dt, J = 13 et 4,5 Hz, 1H : 1H du CH₂ en 5 ε) ; 2,92 (dd, J = 12 et 4 Hz, 1H : 1H du CH₂ en 4 β) ; de 3,20 à 3,30 (mt, 1H : 1H du CH₂ en 3 δ) ; 3,29 (s, 3H : NCH₃) ; 3,33 (t, J = 12 Hz, 1H : l'autre H du CH₂ en 4 β) ; 3,57 (mt, 1H : l'autre H du CH₂ en 3 δ) ; 3,93 (AB limite, 4H : NCH₂ de l'allyle) ; 4,60 (dd, J = 8 et 6,5Hz, 1H : 3 α) ; 4,78 (dd large, J = 13 et 7,5 Hz, 1H : l'autre H du CH₂ en 5 e) ; 4,87 (mt, 1H : 2 α) ; 4,92 (dd, J = 10 et 1 Hz, 1H : 1 α) ; de 5,10 à 5,25 (mt, 5H : 4 α et =CH₂ de l'allyle) ; 5,28 (d large, J = 6 Hz, 1H : 5 α) ; 5,85 (mt, 2H : CH= de l'allyle) ; 5,92 (d, J = 9,5 Hz, 1H : 6 α) ; 5,94 (mt, 1H : 1 β) ; 6,54 (d, J = 10 Hz, 1H : NH en 2) ; 6,65 (d, J = 8 Hz, 2H : H Aromatiques en 4 ε) ; 7,05 (d, J = 8 Hz, 2H : H Aromatiques en 4 δ) ; de 7,10 à 7,35 (mt, 5H : H Aromatiques en 6) ; 7,51 (AB limite, 2H : 1' H₄ et 1' H₅) ; 7,88 (dd, J = 4 et 2 Hz, 1H : 1' H₆) ; 8,43 (d, J = 10 Hz, 1H : NH en 1) ; 8,77 (d, J = 9,5 Hz, 1H : NH en 6) ; 11,65 (s, 1H : OH).

### EXEMPLE 21 : Préparation de la 4ζ-allyléthylamino-dés (4ζ-diméthylamino) pristinamycine I_{A}

On réalise à l'échelle de 26 erlenmeyers comme décrit dans l'exemple 3 une culture de la souche SP92::pVRC508 en milieu de production avec ajout à 16h de 1 ml d'une solution à 20 g/l dans la soude 0.1N de dichlorhydrate de 4-allyléthylaminophénylalanine (R,S) synthétisée comme à l'exemple 39-4. Au terme de 40 h de culture, les 0,78 litre de moût issus de 26 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloroméhane et injecté sur une colonne de silice (30g) montée dans le dichlorométhane et éluée successivement par paliers de 0 à 10 % de méthanol dans le dichlorométhane. Les fractions contenant la 4ζ-allyléthylamino-dés (4ζ-diméthylamino) pristinamycine I_{A} sont regroupées et évaporées. Le résidu sec est repris par 7 ml d'un mélange 60% eau et 40% acétonitrile et injecté sur une colonne semi-préparative Nucléosil 7µ C8 10x250 mm (Macherey Nagel) éluée dans un mélange de 52% de tampon phosphate 100 mM pH 2,9 et 48% d'acétonitrile. Les fractions contenant la 4ζ-allyléthylamino-dés(4ζ-diméthylamino) pristinamycine I_{A} sont regroupées et extraites par un volume de dichlorométhane. La phase organique est lavée à l'eau, séchée sur sulfate de sodium puis évaporée. On obtient 20 mg de 4ζ-allyléthylamino-dés (4ζ-diméthylamino) pristinamycine I_{A}.

Spectre de R.M.N. ¹ H (400 MHz, CDCl₃, δ en ppm, ref. TMS): 0,58 (dd, J = 16 et 6 Hz, 1H : 1H du CH₂ en 5 β) ; 0,91 (t, J = 7,5 Hz, 3H : CH₃ en 2 γ) ; 1,16 (t, J = 7 Hz, 3H : CH₃ de l'éthyle) ; 1,16 (mt, 1H : 1H du CH₂ en 3 β) ; 1,25 (mt, 1H : 1H du CH₂ en 3 γ) ; 1,32 (d, J = 6,5 Hz, 3H : CH₃ en 1 γ) ; 1,54 (mt, 1H : l'autre H du CH₂ en 3 γ) ; 1,63 et 1,75 (2 mts, 1H chacun : CH₂ en 2 β) ; 2,02 (mt, 1H : l'autre H du CH₂ en 3 β) ; 2,23 et 2,31 (respectivement mt et d large, J = 16,5 Hz, 1H chacun : CH₂ en 5 δ) ; 2,37 (d, J = 16 Hz, 1H : l'autre H du CH₂ en 5 β) ; 2,80 (dt, J = 13 et 4,5 Hz, 1H : 1H du CH₂ en 5 ε) ; 2,87 (dd, J = 12 et 4 Hz, 1H : 1H du CH₂ en 4 β) ; de 3,15 à 3,30 (mt, 1H : 1H du CH₂ en 3 δ) ; 3,26 (s, 3H : NCH₃) ; 3,30 (t, J = 12 Hz, 1H : l'autre H du CH₂ en 4 β) ; 3,36 (mt, 2H : NCH₂ de l'éthyle) ; 3,54 (mt, 1H : l'autre H du CH₂ en 3 δ) ; 3,90 (AB limite, 2H : NCH₂ de l'allyle) ; 4,57 (dd, J = 8 et 6 Hz, 1H : 3 α) ; 4,76 (dd large, J = 13 et 7,5 Hz, 1H : l'autre H du CH₂ en 5 ε) ; 4,84 (mt, 1H : 2 α) ; 4,89 (dd, J = 10 et 1 Hz, 1H : 1 α) ; de 5,05 à 5,20 (mt, 3H : 4 α et =CH₂ de l'allyle) ; 5,27 (d large, J = 6 Hz, 1H : 5 α) ; 5,83 (mt, 1H : CH= de l'allyle) ; 5,88 (d, J = 9,5 Hz, 1H : 6 α) ; 5,91 (mt, 1H : 1 β) ; 6,50 (d, J = 10 Hz, 1H : NH en 2) ; 6,60 (d, J = 8 Hz, 2H : H Aromatiques en 4 ε) ; 7,02 (d, J = 8 Hz, 2H : H Aromatiques en 4 δ) ; de 7,10 à 7,35 (mt, 5H : H Aromatiques en 6) ; 7,47 (AB limite, 2H : 1' H₄ et 1' H₅) ; 7,88 (dd, J = 4 et 2 Hz, 1H : 1' H₆) ; 8,41 (d, J = 10 Hz, 1H : NH en 1) ; 8,75 (d, J = 9,5 Hz, 1H : NH en 6) ; 11,62 (s, 1H : OH).

### EXEMPLE 22 : Préparation de la 4ζ-éthyl propylamino-dés(4ζ-diméthylamino) pristinamycine I_{A}

On réalise à l'échelle de 60 erlenmeyers comme décrit dans l'exemple 3 une culture de la souche SP92::pVRC508 en milieu de production avec ajout à 16h de 1 ml d'une solution à 20 g/l dans la soude 0,1N de dichlorhydrate de 4-éthylpropylaminophénylalanine (R,S) synthétisée comme à l'exemple 39-6. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-éthyl propylamino-dés (4ζ-diméthylamino) pristinamycine I_{A} sont regroupées et évaporées. Le résidu sec est repris par 7 ml d'un mélange 60% eau et 40% acétonitrile et injecté sur une colonne semi-préparative Nucléosil 7µ C8 10x250 mm (Macherey Nagel) éluée dans un mélange de 63% de tampon phosphate 100 mM pH 2,9 et 37% d'acétonitrile. Les fractions contenant la 4ζ-éthyl propylamino-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 16 mg de 4ζ-éthyl propylamino-dés (4ζ-diméthylamino) pristinamycine I_{A}.

Spectre de R.M.N. ¹ H (400 MHz, CDCl₃, δ en ppm, ref. TMS): 0,67 (dd, J = 16 et 6 Hz, 1H : 1H du CH₂ en 5 β) ; 0,91 (t, J = 7,5 Hz, 3H : CH₃ en 2 γ) ; 0,95 (t, J = 7,5 Hz, 3H : CH₃ du propyle) ; 1,14 (t, J = 7 Hz, 3H : CH₃ de l'éthyle) ; 1,15 (mt, 1H : 1H du CH₂ en 3 β) ; 1,25 (mt, 1H : 1H du CH₂ en 3 γ) ; 1,33 (d, J = 7 Hz, 3H : CH₃ en 1 γ) ; de 1,45 à 1,65 (mt, 3H : l'autre H du CH₂ en 3 γ et CH₂ propyle) ; 1,63 et 1,75 (2 mts, 1H chacun : CH₂ en 2 β) ; 2,02 (mt, 1H : l'autre H du CH₂ en 3 β) ; 2,23 et 2,33 (respectivement mt et d large, J = 16,5 Hz, 1H chacun : CH₂ en 5 δ) ; 2,37 (d, J = 16 Hz, 1H : l'autre H du CH₂ en 5 β) ; 2,80 (dt, J = 13 et 5 Hz, 1H : 1H du CH₂ en 5 ε) ; 2,89 (dd, J = 12 et 4 Hz, 1H : 1H du CH₂ en 4 β) ; de 3,10 à 3,25 (mt, 3H : 1H du CH₂ en 3 δ et NCH₂ du propyle) ; 3,26 (s, 3H : NCH₃) ; de 3,25 à 3,40 (mt, 2H : NCH₂ de l'éthyle) ; 3,34 (t, J = 12 Hz, 1H : l'autre H du CH₂ en 4 β) ; 3,54 (mt, 1H : l'autre H du CH₂ en 3 δ) ; 4,57 (dd, J = 7,5 et 6 Hz, 1H : 3 α) ; 4,76 (dd large, J = 13 et 7,5 Hz, 1H : l'autre H du CH₂ en 5 ε) ; 4,84 (mt, 1H : 2 α) ; 4,89 (dd, J = 10 et 1 Hz, 1H : 1 α) ; 5,21 (dd, J = 12 et 4 Hz, 1H : 4 α) ; 5,28 (d large, J = 6 Hz, 1H : 5 α) ; 5,88 (d, J = 9,5 Hz, 1H : 6 α) ; 5,91 (mt, 1H : 1 β) ; 6,48 (d, J = 10 Hz, 1H : NH en 2) ; 6,60 (d, J = 8 Hz, 2H : H Aromatiques en 4 ε) ; 7,03 (d, J = 8 Hz, 2H : H Aromatiques en 4 δ) ; de 7,10 à 7,35 (mt, 5H : H Aromatiques en 6) ; 7,47 (AB limite, 2H : 1' H₄ et 1' H₅) ; 7,89 (mt, 1H : 1' H₆) ; 8,42 (d, J = 10 Hz, 1H : NH en 1) ; 8,76 (d, J = 9,5 Hz, 1H : NH en 6) ; 11,62 (s, 1H : OH).

### EXEMPLE 23 : Préparation de la 4ζ-trifluorométhoxy-dés(4ζ-diméthylamino) pristinamycine I_{A}

On réalise à l'échelle de 60 erlenmeyers comme décrit dans l'exemple 3 une culture de la souche SP92::pVRC508 en milieu de production avec ajout à 16h de 1 ml d'une solution à 20 g/l dans l'eau de chlorhydrate de 4-O-trifluorométhyltyrosine (R,S) synthétisé comme à l'exemple 34-8. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois volumes de dichlorométhane. Les phases chlorométhylèniques sont lavées à l'eau puis combinées, séchées sur sulfate de sodium et évaporées. L'extrait sec est repris par ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-trifluorométhoxy-dés (4ζ-diméthylamino) pristinamycine I_{A} sont regroupées et évaporées. Le résidu sec est repris par 7 ml d'un mélange 60% eau et 40% acétonitrile et injecté en deux fois sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la 4ζ-trifluorométhoxy-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 46,5 mg de 4ζ-trifluorométhoxy-dés (4ζ-diméthylamino) pristinamycine I_{A}.

Spectre de R.M.N. ¹ H (400 MHz, CDCl₃, δ en ppm, ref. TMS): 0,77 (dd, J = 16 et 5,5 Hz, 1H : 1H du CH₂ en 5 β) ; 0,92 (t, J = 7,5 Hz, 3H : CH₃ en 2 γ) ; 1,08 (mt, 1H : 1H du CH₂ en 3 β) ; de 1,30 à 1,40 (mt, 1H : 1H du CH₂ en 3 γ) ; 1,33 (d, J = 7 Hz, 3H : CH₃ en 1 γ) ; de 1,55 à 1,70 (mt, 1H : l'autre H du CH₂ en 3 γ) ; 1,65 et 1,76 (2 mts, 1H chacun : CH₂ en 2 β) ; 2,02 (mt, 1H : l'autre H du CH₂ en 3 (3) ; 2,11 et 2,40 (respectivement mt et d large, J = 16,5 Hz, 1H chacun : CH₂ en 5 δ) ; 2,54 (d, J = 16 Hz, 1H : l'autre H du CH₂ en 5 β) ; 2,88 (dt, J = 13 et 4 Hz, 1H : 1H du CH₂ en 5 e) ; 3,08 (dd, J = 12 et 5 Hz, 1H : 1H du CH₂ en 4 β) ; 3,22 (s, 3H : NCH₃) ; de 3,30 à 3,45 (mt, 1H : 1H du CH₂ en 3 δ) ; 3,39 (t, J = 12 Hz, 1H : l'autre H du CH₂ en 4 β) ; 3,59 (mt, 1H : l'autre H du CH₂ en 3 δ) ; 4,53 (t, J = 7,5 Hz, 1H : 3 α) ; 4,75 (dd large, J = 13 et 8 Hz, 1H : l'autre H du CH₂ en 5 e) ; 4,85 (mt, 1H : 2 α) ; 4,89 (dd, J = 10 et 1,5 Hz, 1H : 1 α) ; 5,35 (d large, J = 5,5 Hz, 1H : 5 α) ; 5,41 (dd, J = 12 et 5 Hz, 1H : 4 α) ; 5,92 (d, J = 10 Hz, 1H : 6 α) ; 5,93 (mt, 1H : 1 β) ; 6,53 (d, J = 9,5 Hz, 1H : NH en 2) ; de 7,15 à 7,35 (mt, 5H : H Aromatiques en 6) ; 7,16 (d, J = 8 Hz, 2H : H Aromatiques en 4 ε) ; 7,26 (d, J = 8 Hz, 2H : H Aromatiques en 4 δ) ; 7,37 (dd, J = 8,5 et 4 Hz, 1H : 1' H₅) ; 7,42 (dd, J = 8,5 et 1,5 Hz, 1H : 1' H₄) ; 7,70 (dd, J = 4 et 1,5 Hz, 1H : 1' H₆) ; 8,37 (d, J = 10 Hz, 1H : NH en 1) ; 8,75 (d, J = 10 Hz, 1H : NH en 6) ; 11,66 (s, 1H : OH).

### EXEMPLE 24 : Préparation de la 4ζ-allyloxy-dés (4ζ-diméthylamino) pristinamycine I_{A}

On réalise à l'échelle de 90 erlenmeyers comme décrit dans l'exemple 3 une culture de la souche SP92::pVRC508 en milieu de production avec ajout à 16h de 1 ml d'une solution à 20 g/l dans l'acide chlorhydrique 0.1N de chlorhydrate de 4-O-allyltyrosine (S) synthétisée comme à l'exemple 33. Au terme de 40 h de culture, les 2,7 litres de moût issus de 90 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-allyloxy-dés (4ζ-diméthylamino) pristinamycine I_{A} sont regroupées et évaporées. Le résidu sec est repris par 7 ml d'un mélange 60% eau et 40% acétonitrile et injecté sur une colonne semi-préparative Nucléosil 7µ C8 10x250 mm (Macherey Nagel) éluée dans un mélange de 52% de tampon phosphate 100 mM pH 2,9 et 48% d'acétonitrile. Les fractions contenant la 4ζ-allyloxy-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 29 mg de 4ζ-allyloxy-dés (4ζ-diméthylamino) pristinamycine I_{A}.

Spectre de R.M.N. ¹ H (400 MHz, CDCl₃, δ en ppm, ref. TMS): 0,63 (dd, J = 16 et 6 Hz, 1H : 1H du CH₂ en 5 β) ; 0,91 (t, J = 7,5 Hz, 3H : CH₃ en 2 γ) ; 1,13 (mt, 1H : 1H du CH₂ en 3 β) ; 1,29 (mt, 1H : 1H du CH₂ en 3 γ) ; 1,33 (d, J = 6,5 Hz, 3H : CH₃ en 1 γ) ; 1,57 (mt, 1H : l'autre H du CH₂ en 3 γ) ; 1,65 et 1,74 (2 mts, 1H chacun : CH_{2,} en 2 β) ; 2,02 (mt, 1H : l'autre H du CH₂ en 3 β) ; 2,14 et 2,34 (respectivement mt et d large, J = 16,5 Hz, 1H chacun : CH₂ en 5 δ) ; 2,43 (d, J = 16 Hz, 1H : l'autre H du CH₂ en 5 β) ; 2,85 (dt, J = 13 et 4 Hz, 1H : 1H du CH₂ en 5 ε) ; 2,95 (dd, J = 12 et 4 Hz, 1H : 1H du CH₂ en 4 β) ; 3,25 (s, 3H : NCH₃) ; 3,33 (mt, 1H : 1H du CH₂ en 3 δ) ; 3,36 (t, J = 12 Hz, 1H : l'autre H du CH₂ en 4 β) ; 3,56 (mt, 1H : l'autre H du CH₂ en 3 δ) ; 4,51 (AB limite, 2H : OCH₂ de l'allyle) ; 4,56 (t, J = 7,5 Hz, 1H : 3 α) ; 4,75 (dd large, J = 13 et 8 Hz, 1H : l'autre H du CH₂ en 5 e) ; 4,84 (mt, 1H : 2 α) ; 4,88 (dd, J = 10 et 1 Hz, 1H : 1 α) ; 5,27 (dd, J = 12 et 4 Hz, 1H : 4 α) ; 5,32 (d large, J = 6 Hz, 1H : 5 α) ; 5,30 et 5,40 (respectivement mt et dd, J = 17 et 1,5 Hz, 1H chacun : =CH₂ de l'allyle) ; 5,89 (d, J = 9,5 Hz, 1H : 6 α) ; 5,91 (mt, 1H : 1 β) ; 6,02 (mt, 1H : CH= de l'allyle) ; 6,50 (d, J = 10 Hz, 1H : NH en 2) ; 6,85 (d, J = 8 Hz, 2H : H Aromatiques en 4 ε) ; 7,12 (d, J = 8 Hz, 2H : H Aromatiques en 4 δ) ; de 7,10 à 7,35 (mt, 5H : H Aromatiques en 6) ; 7,45 (dd, J = 8,5 et 1,5 Hz, 1H : 1' H₄) ; 7,57 (dd, J = 8,5 et 4 Hz, 1H : 1' H₅) ; 7,77 (dd, J = 4 et 1,5 Hz, 1H : 1' H₆) ; 8,41 (d, J = 10 Hz, 1H : NH en 1) ; 8,74 (d, J = 9,5 Hz, 1H : NH en 6) ; 11,63 (s, 1H : OH).

### EXEMPLE 25 _{:} Préparation de la 4ζ-éthoxy-dés(4ζ-diméthylamino) pristinamycine I_{A}

On réalise à l'échelle de 90 erlenmeyers comme décrit dans l'exemple 3 une culture de la souche SP92::pVRC508 en milieu de production avec ajout à 16h de 1 ml d'une solution à 20 g/l dans l'acide chlorhydrique 0,1N de chlorhydrate de 4-O-éthyltyrosine (S) synthétisé comme à l'exemple 33. Au terme de 40 h de culture, les 2,7 litres de moût issus de 90 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-éthoxy-dés (4ζ-diméthylamino) pristinamycine I_{A} sont regroupées et évaporées. Le résidu sec est repris par 7 ml d'un mélange 60% eau et 40% acétonitrile et injecté sur une colonne semi-préparative Nucléosil 7µ C8 10x250 mm (Macherey Nagel) éluée dans un mélange de 52% de tampon phosphate 100 mM pH 2,9 et 48% d'acétonitrile. Les fractions contenant la 4ζ-éthoxy-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 29 mg de 4ζ-éthoxy-dés (4ζ-diméthylamino) pristinamycine I_{A}.

Spectre de R.M.N. ¹ H (400 MHz, CDCl₃, δ en ppm, ref. TMS): 0,64 (dd, J = 16 et 5,5 Hz, 1H : 1H du CH₂ en 5 β) ; 0,90 (t, J = 7,5 Hz, 3H : CH₃ en 2 γ) ; 1,12 (mt, 1H : 1H du CH₂ en 3 β) ; 1,25 (mt, 1H : 1H du CH₂ en 3 γ) ; 1,33 (d, J = 7 Hz, 3H : CH₃ en 1 γ) ; 1,42 (t, J = 7 Hz, 3H : CH₃ de l'éthyle) ; 1,57 (mt, 1H : l'autre H du CH₂ en 3 γ) ; 1,63 et 1,74 (2 mts, 1H chacun : CH₂ en 2 β) ; 2,02 (mt, 1H : l'autre H du CH₂ en 3 β) ; 2,16 et 2,35 (respectivement mt et d large, J = 16,5 Hz, 1H chacun : CH₂ en 5 δ) ; 2,43 (d, J = 16 Hz, 1H : l'autre H du CH₂ en 5 β) ; 2,83 (dt, J = 13 et 4 Hz, 1H : 1H du CH₂ en 5 ε) ; 2,93 (dd, J = 12 et 4 Hz, 1H : 1H du CH₂ en 4 β) ; de 3,15 à 3,30 (mt, 1H : 1H du CH₂ en 3 δ) ; 3,24 (s, 3H : NCH₃) ; 3,35 (t, J = 12 Hz, 1H : l'autre H du CH₂ en 4 β) ; 3,55 (mt, 1H : l'autre H du CH₂ en 3 δ) ; 3,95 (AB limite, 2H : OCH₂ de l'éthyle) ; 4,56 (dd, J = 7,5 et 6 Hz, 1H : 3 α) ; 4,75 (dd large, J = 13 et 8 Hz, 1H : l'autre H du CH₂ en 5 ε) ; 4,84 (mt, 1H : 2 α) ; 4,87 (dd, J = 10 et 1 Hz, 1H : 1 α) ; 5,26 (dd, J = 12 et 4 Hz, 1H : 4 α) ; 5,32 (d large, J = 5,5 Hz, 1H : 5 α) ; 5,88 (d, J = 10 Hz, 1H : 6 α) ; 5,92 (mt, 1H : 1 β) ; 6,48 (d, J = 10 Hz, 1H : NH en 2) ; 6,83 (d, J = 8 Hz, 2H : H Aromatiques en 4 ε) ; 7,10 (d, J = 8 Hz, 2H : H Aromatiques en 4 δ) ; de 7,10 à 7,35 (mt, 5H : H Aromatiques en 6) ; 7,44 (dd, J = 8,5 et 1,5 Hz, 1H : 1' H₄) ; 7,57 (dd, J = 8,5 et 4,5 Hz, 1H : 1' H₅) ; 7,77 (dd, J = 4,5 et 1,5 Hz, 1H : 1' H₆) ; 8,38 (d, J = 10 Hz, 1H : NH en 1) ; 8,75 (d, J = 10 Hz, 1H : NH en 6) ; 11,60 (s, 1H : OH).

### EXEMPLE 26 : Préparation de la 4ζ-(2-chloroéthoxy) -dés (4ζ-diméthylamino) pristinamycine I_{A}

On réalise à l'échelle de 60 erlenmeyers comme décrit dans l'exemple 3 une culture de la souche SP92::pVRC508 en milieu de production avec ajout à 16h de 1 ml d'une solution à 20 g/l dans l'eau de chlorydrate de 4-O(2-chloroéthyl) tyrosine (S) synthétisé comme à l'exemple 42-1. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-(2-chloroéthoxy)-dés (4ζ-diméthylamino) pristinamycine I_{A} sont regroupées et évaporées. Le résidu sec est repris par 7 ml d'un mélange 60% eau et 40% acétonitrile et injecté sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la 4ζ-(2-chloroéthoxy)-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 3,2 mg de 4ζ-(2-chloroéthoxy)-dés (4ζ-diméthylamino) pristinamycine I_{A.}

Spectre de RM.N. ¹H (400 MHz, CDCl₃, δ en ppm, ref. TMS): 0,66 (dd, J = 16 et 5,5 Hz, 1H : 1H du CH₂ en 5 β) ; 0,91 (t, J = 7,5 Hz, 3H : CH₃ en 2 γ) ; 1,13 (mt, 1H : 1H du CH₂ en 3 β) ; 1,28 (mt, 1H : 1H du CH₂ en 3 γ) ; 1,33 (d, J = 7 Hz, 3H : CH₃ en 1 γ) ; 1,57 (mt, 1H : l'autre H du CH₂ en 3 γ) ; 1,66 et 1,76 (2 mts, 1H chacun : CH₂ en 2 β) ; 2,02 (mt, 1H : l'autre H du CH₂ en 3 β) ; 2,16 et 2,37 (respectivement mt et d large, J = 16,5 Hz, 1H chacun : CH₂ en 5 δ) ; 2,47 (d, J = 16 Hz, 1H : l'autre H du CH₂ en 5 β) ; 2,86 (dt, J = 13 et 4 Hz, 1H : 1H du CH₂ en 5 ε) ; 2,95 (dd, J = 12 et 4 Hz, 1H : 1H du CH₂ en 4 β) ; 3,23 (s, 3H : NCH₃); 3,32 (mt, 1H : 1H du CH₂ en 3 δ) ; 3,37 (t, J = 12 Hz, 1H : l'autre H du CH₂ en 4 β) ; 3,57 (mt, 1H : l'autre H du CH₂ en 3 δ) ; 3,82 (t, J = 6 Hz, 2H : CH₂Cl) ; 4,19 (AB limite, 2H : OCH₂ de l'éthyle) ; 4,55 (dd, J = 7,5 et 7 Hz, 1H : 3 α) ; 4,75 (dd large, J = 13 et 8 Hz, 1H : l'autre H du CH₂ en 5 ε) ; 4,84 (mt, 1H : 2 α) ; 4,87 (d large, J = 10 Hz, 1H : 1 α) ; 5,28 (dd, J = 12 et 4 Hz, 1H : 4 α) ; 5,32 (d large, J = 5,5 Hz, 1H : 5 α) ; 5,88 (d, J = 10 Hz, 1H : 6 α) ; 5,90 (mt, 1H : 1 β) ; 6,50 (d, J = 10 Hz, 1H : NH en 2) ; 6,86 (d, J = 8 Hz, 2H : H Aromatiques en 4 ε) ; 7,13 (d, J = 8 Hz, 2H : H Aromatiques en 4 δ) ; de 7,10 à 7,35 (mt, 5H : H Aromatiques en 6) ; 7,45 (AB limite, 2H : 1' H₄ et 1' H₅) 7,75 (dd, J = 4 et 2 Hz, 1H : 1' H₆) ; -8,38 (d, J = 10 Hz, 1H : NH en 1) ; 8,74 (d, J = 10 Hz, 1H : NH en 6) ; 11,62 (s, 1H : OH).

### EXEMPLE 27 : Préparation de la 4ζ-acétyl -dés 4ζ-diméthylamino) pristinamycine I_{A}

On réalise à l'échelle de 60 erlenmeyers comme décrit dans l'exemple 3 une culture de la souche SP92::pVRC508 en milieu de production avec ajout à 16h de 1 ml d'une solution à 20 g/l dans la soude 0,1N de 4-acétyl phénylalanine (S) synthétisée comme à l'exemple 33. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-acétyl)-dés (4ζ-diméthylamino) pristinamycine I_{A} sont regroupées et évaporées. Le résidu sec est repris par 7 ml d'un mélange 60% eau et 40% acétonitrile et injecté sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la 4ζ-acétyl-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 4,2 mg de 4ζ-acétyl-dés (4ζ-diméthylamino) pristinamycine I_{A.}

Spectre de R.M.N. ¹H (400 MHz, CDCl₃, δ en ppm, ref. TMS): 0,73 (dd, J = 16 et 6 Hz, 1H : 1H du CH₂ en 5 β) ; 0,93 (t, J = 7,5 Hz, 3H : CH₃ en 2 γ) ; 1,12 (mt, 1H : 1H du CH₂ en 3 β) ; de 1,25 à 1,45 (mt, 1H : 1H du CH₂ en 3 γ) ; 1,33 (d, J = 7 Hz, 3H : CH₃ en 1 γ) ; 1,62 (mt, 1H : l'autre H du CH₂ en 3 γ) ; de 1,60 à 1,85 (mt, 2H CH₂ en 2 β) ; 2,02 (mt, 1H : l'autre H du CH₂ en 3 (3) ; 2,20 et 2,42 (respectivement mt et d large, J = 16,5 Hz, 1H chacun : CH₂ en 5 δ) ; 2,52 (d, J = 16 Hz, 1H : l'autre H du CH₂ en 5 β) ; 2,60 (s, 3H : ArCOCH₃) ; 2,88 (dt, J = 13 et 4,5 Hz, 1H : 1H du CH₂ en 5 ε) ; 3,13 (dd, J = 13,5 et 5,5 Hz, 1H : 1H du CH₂ en 4 β) ; 3,21 (s, 3H : NCH₃) ; de 3,30 à 3,50 (mt, 1H : l'autre H du CH₂ en 4 β) ; de 3,30 à 3,50 et 3,63 (2 mts, 1H chacun : CH₂ en 3 δ) ; 4,53 (t, J = 7,5 Hz, 1H : 3 α) ; 4,75 (dd large, J = 13 et 8 Hz, 1H : l'autre H du CH₂ en 5 ε) ; 4,84 (mt, 1H : 2 α) ; 4,88 (dd, J = 10 et 1 Hz, 1H : 1 α) ; 5,35 (d large, J = 6 Hz, 1H : 5 α) ; 5,43 (dd, J = 10,5 et 4 Hz, 1H : 4 α) ; 5,90 (d, J = 9,5 Hz, 1H : 6 α) ; 5,92 (mt, 1H : 1 β) ; 6,56 (d, J = 9,5 Hz, 1H : NH en 2) ; de 7,10 à 7,35 (mt, 5H : H Aromatiques en 6) ; 7,28 (d, J = 8 Hz, 2H : H Aromatiques en 4 δ) ; 7,38 (dd, J = 8,5 et 2 Hz, 1H : 1' H₄) ; 7,42 (dd, J = 8,5 et 4,5 Hz, 1H : 1' H₅) ; 7,66 (dd, J = 4,5 et 2 Hz, 1H : 1' H₆) ; 7,88 (d, J = 8 Hz, 2H : H Aromatiques en 4 ε) ; 8,38 (d, J = 10 Hz, 1H : NH en 1) ; 8,74 (d, J = 9,5 Hz, 1H : NH en 6) ; 11,65 (s, 1H : OH).

### EXEMPLE 28 : Préparation de la 4ε-diméthylamino-dés (4ζ-diméthylamino) pristinamycine I_{A}.

On réalise à l'échelle de 60 erlenmeyers comme décrit dans l'exemple 3 une culture de la souche SP92::pVRC508 en milieu de production avec ajout à 16h de 1 ml d'une solution à 20 g/l dans la soude 0,1N de dichlorhydrate de 3-diméthylaminophénylalanine (R,S) synthétisée comme à l'exemple 35-10. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichloromethane et éluée successivement par paliers de 0 à 10 % de méthanol dans le dichlorométhane. Les fractions contenant la 4ε-diméthylamino-dés (4ζ-diméthylamino) pristinamycine I_{A} sont regroupées et évaporées. Le résidu sec est repris par 3 ml d'un mélange 60% eau et 40% acétonitrile et injecté sur une colonne semi-préparative Nucléosil 7µ C8 10x250 mm (Macherey Nagel) éluée dans un mélange de 57% de tampon phosphate 100 mM pH 2,9 et 43% d'acétonitrile. Les fractions contenant la 4ε-diméthylamino-dés (4ζ-diméthylamino) pristinamycine I_{A} sont regroupées et extraites par un volume de dichlorométhane. La phase organique est lavée à l'eau, séchée sur sulfate de sodium puis évaporée. On obtient 1,1 mg de 4ε-diméthylamino-dés (4ζ-diméthylamino) pristinamycine I_{A}.

Spectre de R.M.N. ¹H (400 MHz, CDCl₃, δ en ppm, ref. TMS): 0,63 (dd, J = 16 et 5 Hz, 1H : 1H du CH₂ en 5 β) ; 0,91 (t, J = 7,5 Hz, 3H : CH₃ en 2 γ) ; 1,13 (mt, 1H : 1H du CH₂ en 3 β) ; de 1,20 à 1,35 (mt, 1H : 1H du CH₂ en 3 γ) ; 1,32 (d, J = 6,5 Hz, 3H : CH₃ en 1 γ) ; 1,57 (mt, 1H : l'autre H du CH₂ en 3 γ) ; 1,63 et 1,76 (2 mts, 1H chacun : CH₂ en 2 β) ; 2,02 (mt, 1H : l'autre H du CH₂ en 3 β) ; 2,08 et 2,31 (respectivement mt et d large, J = 16,5 Hz, 1H chacun : CH₂ en 5 δ) ; 2,35 (d, J = 16 Hz, 1H : l'autre H du CH₂ en 5 β) ; 2,81 (dt, J = 13 et 4 Hz, 1H : 1H du CH₂ en 5 ε) ; 2,90 (s, 6H : N(CH₃)₂) ; 2,97 (dd, J = 12 et 4 Hz, 1H : 1H du CH₂ en 4 β) ; de 3,20 à 3,30 (mt, 1H : 1H du CH₂ en 3 δ) ; 3,28 (s, 3H : NCH₃) ; 3,37 (t, J = 12 Hz, 1H : l'autre H du CH₂ en 4 β) ; 3,57 (mt, 1H : l'autre H du CH₂ en 3 δ) ; 4,58 (t, J = 7,5 Hz, 1H : 3 α) ; 4,74 (dd large, J = 13 et 8 Hz, 1H : l'autre H du CH₂ en 5 ε) ; 4,86 (mt, 1H : 2 α) ; 4,89 (d large, J = 10 Hz, 1H : 1 α) ; 5,27 (dd, J = 12 et 4 Hz, 1H : 4 α) ; 5,29 (d large, J = 5 Hz, 1H : 5 α) ; 5,89 (d, J = 9,5 Hz, 1H : 6 α) ; 5,90 (mt, 1H : 1 β) ; 6,50 (d, J = 10 Hz, 1H : NH en 2) ; de 6,50 à 6,70 (mt, 3H : H Aromatiques en ortho et en para du diméthylamino) ; de 7,15 à 7,35 (mt, 5H : H Aromatiques en 6) ; 7,20 (t, J = ml d'une solution à 20 g/l dans la soude 0.1N de chlorhydrate de 3-méthylthiophénylalanine (R,S) synthétisé comme à l'exemple 34-11. Au terme de 40 h de culture, les 1,68 litres de moût issus de 56 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichloromethane et éluée successivement par paliers de 0 à 10 % de méthanol dans le dichlorométhane. Les fractions contenant le nouveau dérivé de pristinamycine I_{A} sont regroupées et évaporées. Le résidu sec est repris par 7 ml d'un mélange 54% eau et 46% acétonitrile et injecté sur une colonne semi-préparative Nucléosil 7µ C8 10x250 mm (Macherey Nagel) éluée dans un mélange de 55% de tampon phosphate 100 mM pH 2,9 et 45% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 20 mg de 4ε-méthylthio-dés (4ζ-diméthylamino) pristinamycine I_{A}.

Spectre de R.M.N. ¹H (400 MHz, CDCl₃, δ en ppm, ref. TMS): 0,56 (dd, J = 16 et 5,5 Hz, 1H : 1H du CH₂ en 5 β) ; 0,90 (t, J = 7,5 Hz, 3H : CH₃ en 2 γ) ; 1,13 (mt, 1H : 1H du CH₂ en 3 β) ; 1,28 (mt, 1H : 1H du CH₂ en 3 γ) ; 1,32 (d, J = 6,5 Hz, 3H : CH₃ en 1 γ) ; 1,58 (mt, 1H : l'autre H du CH₂ en 3 γ) ; 1,62 et 1,74 (2 mts, 1H chacun : CH₂ en 2 β) ; 2,02 (mt, 1H : l'autre H du CH₂ en 3 β) ; 2,25 et 2,35 (respectivement mt et d large, J = 16,5 Hz, 1H chacun : CH₂ en 5 δ) ; 2,39 (d, J = 16 Hz, 1H : l'autre H du CH₂ en 5 β) ; 2,43 (s, 3H : SCH₃) ; 2,82 (dt, J = 13 et 4 Hz, 1H : 1H du CH₂ en 5 ε) ; 2,98 (dd, J = 12 et 4,5 Hz, 1H : 1H du CH₂ en 4 β) ; 3,26 (s, 3H : NCH₃) ; 3,30 (t, J = 12 Hz 1H : 1H du CH₂ en 3 δ) ; 3,38 (mt, 1H : l'autre H du CH₂ en 4 β) ; 3,57 (mt, 1H : l'autre H du CH₂ en 3 δ) ; 4,56 (t, J = 7,5 Hz, 1H : 3 α) ; 4,74 (dd large, J = 13 et 8 Hz, 1H : l'autre H du CH₂ en 5 ε) ; 4,84 (mt, 1H : 2 α) ; 4,89 (dd, J = 10 et 1 Hz, 1H : 1 α) ; 5,29 (dd, J = 12 et 4,5 Hz, 1H : 4 α) ; 5,32 (d large, J = 5,5 Hz, 1H : 5 α) ; 5,88 (d, J = 9,5 Hz, 1H : 6 α) ; 5,90 (mt, 1H : 1 β) ; 6,51 (d, J = 10 Hz, 1H : NH en 2) ; 6,99 (d large, J = 8 Hz, 1H : H Aromatique en para du méthylthio) ; 7,10 et 7,15 (respectivement s large et d large, J = 8 Hz, 1H chacun : H Aromatiques en ortho du méthylthio) ; de 7,15 à 7,35 (mt, 6H : H Aromatiques en 6 et H Aromatiques en méta du méthylthio) ; 7,43 (d large, J = 8 Hz, 1H : 1' H₄) ; 7,52 (dd, J = 8 et 4 Hz, 1H : 1' H₅) ; 7,79 (d large, J = 4 Hz, 1H : 1' H₆) ; 8,38 (d, J = 10 Hz, 1H : NH en 1) ; 8,73 (d, J = 9,5 Hz, 1H : NH en 6) ; 11,62 (s, 1H : OH).

### EXEMPLE 30 : Préparation de la 4ε-éthoxy-dés(4ζ-diméthylamino) pristinamycine I_{A}.

On réalise à l'échelle de 60 erlenmeyers comme décrit dans l'exemple 3 une culture de la souche SP92::pVRC508 en milieu de production avec ajout à 16h de 1 ml d'une solution à 20 g/l dans la soude 0,2N de chlorhydrate de 3-O-éthyltyrosine (S) synthétisé comme à l'exemple 37-1. Au terme de 40 h de culture, les 1,8 litres de moût issus de 60 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichloromethane et injecté sur une colonne de silice (30g) montée dans le dichlorométhane et éluée successivement par paliers de 0 à 10 % de méthanol dans le dichlorométhane. Les fractions contenant le nouveau dérivé de pristinamycine I_{A} sont regroupées et évaporées. On obtient 19 mg de résidu sec. Celui-ci est repris par 3 ml d'un mélange 60% eau et 40% acétonitrile et injecté sur une colonne semi-préparative Nucléosil 7µ C8 10x250 mm (Macherey Nagel) éluée dans un mélange de 60% de tampon phosphate 100 mM pH 2,9 et 40% d'acétonitrile. Les fractions contenant la nouvelle pristinamycine sont regroupées et extraites par un volume de dichlorométhane. La phase organique est lavée à l'eau, séchée sur sulfate de sodium puis évaporée. On obtient 15,8 mg de 4ε-O-éthoxy-dés (4ζ-diméthylamino) pristinamycine I_{A}.

Spectre de R.M.N. ¹H (400 MHz, CDCl₃, δ en ppm, ref. TMS): 0,55 (dd, J = 16 et 5,5 Hz, 1H : 1H du CH₂ en 5 β) ; 0,90 (t, J = 7,5 Hz, 3H : CH₃ en 2 γ) ; 1,12 (mt, 1H : 1H du CH₂ en 3 β) ; 1,20 (mt, 1H : 1H du CH₂ en 3 γ) ; 1,31 (d, J = 6,5 Hz, 3H : CH₃ en 1 γ) ; 1,49 (t, J = 7 Hz, 3H : CH₃ de l'éthyle) ; 1,54 (mt, 1H : l'autre H du CH₂ en 3 γ) ; 1,63 et 1,73 (2 mts, 1H chacun : CH₂ en 2 β) ; 2,02 (mt, 1H : l'autre H du CH₂ en 3 β) ; 2,22 et 2,33 (respectivement mt et d large, J = 16,5 Hz, 1H chacun : CH₂ en 5 δ) ; 2,46 (d, J = 16 Hz, 1H : l'autre H du CH₂ en 5 β) ; 2,83 (dt, J = 13 et 4 Hz, 1H : 1H du CH₂ en 5 ε) ; 2,95 (dd, J = 12 et 4 Hz, 1H : 1H du CH₂ en 4 β) ; 3,22 (mt, 1H : 1H du CH₂ en 3 δ) ; 3,27 (s, 3H : NCH₃) ; 3,39 (t, J = 12 Hz, 1H : l'autre H du CH₂ en 4 β) ; 3,53 (mt, 1H : l'autre H du CH₂ en 3 δ) ; 3,93 et 4,03 (2 mts, 1H chacun : OCH₂ de l'éthyle) ; 4,56 (dd, J = 7 et 5,5 Hz, 1H : 3 α) ; 4,75 (dd large, J = 13 et 8 Hz, 1H : l'autre H du CH₂ en 5 ε) ; 4,82 (mt, 1H : 2 α) ; 4,88 (dd, J = 10 et 1 Hz, 1H : 1 α) ; 5,23 (dd, J = 12 et 4 Hz, 1H : 4 α) ; 5,23 (d large, J = 5,5 Hz, 1H : 5 α) ; 5,87 (d, J = 9,5 Hz, 1H : 6 α) ; 5,92 (mt, 1H : 1 β) ; 6,47 (d, J = 10 Hz, 1H : NH en 2) ; 6,80 (mt, 3H : H Aromatiques en ortho et en para de l'éthoxy) ; de 7,10 à 7,35 (mt, 6H : H Aromatiques en 6 et H Aromatiques en méta de l'éthoxy) ; 7,43 (dd, J = 8 et 1 Hz, 1H : 1' H₄) ; 7,50 (dd, J = 8 et 4 Hz, 1H : 1' H₅) ; 7,77 (dd, J = 4 et 1 Hz, 1H : 1' H₆) ; 8,38 (d, J = 10 Hz, 1H : NH en 1) ; 8,70 (d, J = 9,5 Hz, 1H : NH en 6) ; 11,60 (s, 1H : OH).

### EXEMPLE 31 : Préparation de la 4ζ-éthylthio-dés(4ζ-diméthylamino) pristinamycine I_{A}

On réalise à l'échelle de 2 erlenmeyers comme décrit dans l'exemple 3 une culture de la souche SP92::pVRC508 en milieu de production avec ajout à 16h de 1 ml d'une solution à 20 g/l dans la soude 0.1N de chlorhydrate de 4-éthylthiophénylalanine (S) synthétisée comme à l'exemple 33. Au terme de 40 h de culture, les 60 ml de moût issus de 2 erlenmeyers sont extraits par 2 volumes d'un mélange de 66% de tampon phosphate 100 mM pH 2,9 et 34% d'acétonitrile, puis centrifugés. Le surnageant est extrait par 2 fois 0,5 volumes de dichlorométhane. Les phases chlorométhylèniques sont lavées à l'eau puis combinées, séchées sur sulfate de sodium et évaporées. L'extrait sec est repris par 20 ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-éthylthio-dés(4ζ-diméthylamino) pristinamycine I_{A} sont regroupées et évaporées. Le résidu sec est repris par 7 ml d'un mélange 60% eau et 40% acétonitrile et injecté sur une colonne semi-préparative Nucléosil 7µ C8 10x250 mm (Macherey Nagel) éluée dans un mélange de 52% de tampon phosphate 100 mM pH 2,9 et 48% d'acétonitrile. Les fractions contenant la 4ζ-éthylthio-dés (4ζ-diméthylamino) pristinamycine I_{A} sont regroupées et extraites par un volume de dichlorométhane. La phase organique est lavée à l'eau, séchée sur sulfate de sodium puis évaporée. On obtient ? mg de 4ζ-éthylthio-dés (4ζ-diméthylamino) pristinamycine I_{A.}

Spectre de R.M.N. ¹H (400 MHz, CDCl₃, δ en ppm) : 0,68 (dd, J = 16 et 6 Hz, 1H : 1H du CH₂ en 5 β) ; 0,92 (t, J = 7,5 Hz, 3H : CH₃ en 2 γ) ; de 1,10 à 1,40 (mt, 5H : 1H du CH₂ en 3 β et 1H du CH₂ en 3 γ et CH₃ de l'éthyle) ; 1,32 (d, J = 7 Hz, 3H : CH₃ en 1 γ) ; de 1,45 à 1,85 (mt, 3H : l'autre H du CH₂ en 3 γ et CH₂ en 2 β) ; 2,02 (mt, 1H : l'autre H du CH₂ en 3 (3) ; 2,18 et 2,37 (respectivement mt et d large, J = 16,5 Hz, 1H chacun : CH₂ en 5 δ) ; 2,45 (d large, J = 16 Hz, 1H : l'autre H du CH₂ en 5 β) ; 2,85 (dt, J = 13 et 4 Hz, 1H : 1H du CH₂ en 5 ε) ; 2,90 (mt, 2H : ArSCH₂ éthyle) ; 2,98 (dd, J = 12 et 4 Hz, 1H : 1H du CH₂ en 4 β) ; 3,25 (s, 3H : NCH₃) ; 3,35 (mt, 1H : 1H du CH₂ en 3 δ) ; 3,39 (t, J = 12 Hz, 1H : l'autre H du CH₂ en 4 β) ; 3,57 (mt, 1H : l'autre H du CH₂ en 3 δ) ; 4,55 (t, J = 7,5 Hz, 1H : 3 α) ; 4,75 (dd large, J = 13 et 7,5 Hz, 1H : l'autre H du CH₂ en 5 ε) ; 4,85 (mt, 1H : 2 α) ; 4,89 (dd, J = 10 et 1 Hz, 1H : 1 α) ; de 5,25 à 5,40 (mt, 2H : 5 α et 4 α) ; 5,88 (d, J = 9,5 Hz, 1H : 6 α) ; 5,91 (mt, 1H : 1 β) ; 6,55 (d, J = 9,5 Hz, 1H : NH en 2) ; 7,10 (d, J = 8 Hz, 2H : H Aromatiques en 4 δ) ; de 7,10 à 7,35 (mt, 7H : H Aromatiques en 6 et 4 ε) ; 7,44 (AB limite, 2H : 1' H₄ et 1' H₅) ; 7,74 (mt, 1H : 1' H₆) ; 8,38 (d, J = 10 Hz, 1H : NH en 1) ; 8,75 (d, J = 9,5 Hz, 1H : NH en 6) ; 11,62 (s, 1H : OH).

### EXEMPLE 32 : Préparation de la 4ζ-éthyl-dés(4ζ-diméthylamino) pristinamycine I_{A}

On réalise à l'échelle de 2 erlenmeyers comme décrit dans l'exemple 3 une culture de la souche SP92::pVRC508 en milieu de production avec ajout à 16h de 1 ml d'une solution à 20 g/l dans la soude 0,1N de 4-éthylphénylalanine (R,S) synthétisée comme à l'exemple 33. Au terme de 40 h de culture, les 60ml de moût issus des 2 erlenmeyers sont extraits par 2 volumes d'un mélange de 66% de tampon phosphate 100 mM pH 2,9 et 34% d'acétonitrile, puis centrifugés. Le surnageant est extrait par 2 fois volumes de dichlorométhane. Les phases chlorométhylèniques sont lavées à l'eau puis combinées, séchées sur sulfate de sodium et évaporées. L'extrait sec est repris par 20ml de dichlorométhane et injecté sur une colonne de silice (30g) montée dans le dichlorométhane et éluée successivement par paliers de 0 à 10 % de méthanol dans le dichlorométhane. Les fractions contenant la 4ζ-éthyl-dés(4ζ-diméthylamino) pristinamycine I_{A} sont regroupées et évaporées. Le résidu sec est repris par 7ml d'un mélange 52% eau et 48% acétonitrile et injecté sur une colonne semi-préparative Nucléosil 7µ C8 10x250 mm (Macherey Nagel) éluée dans un mélange de 52% de tampon phosphate 100 mM pH 2,9 et 48% d'acétonitrile. Les fractions contenant la 4ζ-éthyl-dés(4ζ-diméthylamino) pristinamycine I_{A} sont regroupées et extraites par un volume de dichlorométhane. La phase organique est lavée à l'eau, séchée sur sulfate de sodium puis évaporée. On obtient 0,50 mg de 4ζ-éthyl-dés(4ζ-diméthylamino) pristinamycine I_{A}.

Spectre de R.M.N. ¹H (400 MHz, CDCl₃, δ en ppm, ref. TMS): 0,42 (dd, J = 16 et 5,5 Hz, 1H : 1H du CH₂ en 5 β) ; 0,92 (t, J = 7,5 Hz, 3H : CH₃ en 2 γ) ; de 1,10 à 1,40 (mt, 2H : 1H du CH₂ en 3 β et 1H du CH₂ en 3 γ) ; 1,23 (t, J = 7,5 Hz, 3H : CH₃ de l'éthyle) ; 1,35 (d, J = 7 Hz, 3H : CH₃ en 1 γ) ; de 1,45 à 1,85 (mt, 3H : l'autre H du CH₂ en 3 γ et CH₂ en 2 β) ; 2,02 (mt, 1H : l'autre H du CH₂ en 3 β) ; 2,15 et de 2,25 à 2,40 (2 mts, 1H chacun : CH₂ en 5 δ) ; de 2,25 à 2,40 (mt, 1H : l'autre H du CH₂ en 5 β) ; 2,60 (q, J = 7,5 Hz, 2H : ArCH₂ de l'éthyle) ; 2,83 (dt, J = 13 et 4 Hz, 1H : 1H du CH₂ en 5 e) ; 2,98 (dd, J = 12 et 4 Hz, 1H : 1H du CH₂ en 4 β) ; de 3,25 à 3,35 (mt, 1H : 1H du CH₂ en 3 δ) ; 3,27 (s, 3H : NCH₃) ; 3,39 (t, J = 12 Hz, 1H : l'autre H du CH₂ en 4 β) ; 3,59 (mt, 1H : l'autre H du CH₂ en 3 δ) ; 4,58 (dd, J = 7 et 6,5 Hz, 1H : 3 α) ; 4,75 (dd large, J = 13 et 8 Hz, 1H : l'autre H du CH₂ en 5 e) ; 4,87 (mt, 1H : 2 α) ; 4,89 (dd, J = 10 et 1 Hz, 1H : 1 α) ; 5,24 (d large, J = 5,5 Hz, 1H : 5 α) ; 5,29 (dd, J = 12 et 4 Hz, 1H : 4 α) ; 5,88 (d, J = 10 Hz, 1H : 6 α) ; 5,92 (mt, 1H : 1 β) ; 6,73 (d, J = 10 Hz, 1H : NH en 2) ; de 7,10 à 7,35 (mt, 9H : H Aromatiques en 6 - 4 ε et 4 δ) ; 7,44 (dd, J = 8,5 et 1,5 Hz, 1H : 1' H₄) ; 7,50 (dd, J = 8,5 et 4,5 Hz, 1H : 1' H₅) ; 7,80 (dd, J = 4,5 et 1,5 Hz, 1H : 1' H₆) ; 8,38 (d, J = 10 Hz, 1H : NH en 1) ; 8,75 (d, J = 10 Hz, 1H : NH en 6) ; 11,66 (s, 1H : OH).

A partir des mêmes fractions issues de la colonne de silice décrite ci-dessus, contenant également le nouveau dérivé de pristinamycine I_{H} , 0,3mg de ζ-éthyl-dés(4ζ-diméthylamino) pristinamycine I_{H} sont isolés en opérant la chromatographie sur colonne semi-préparative comme décrit ci-dessus.

Spectre de R.M.N. ¹H (400 MHz, CDCl₃, δ en ppm) : 0,04 (mt, 1H : 1H du CH₂ en 5 β) ; 0,92 (t, J = 7,5 Hz, 3H : CH₃ en 2 γ) ; de 1,10 à 1,40 (mt, 2H : 1H du CH₂ en 5 δ et 1H du CH₂ en 5 γ) ; 1,18 (t, J = 7,5 Hz, 3H : CH₃ de l'éthyle) ; 1,30 (d, J = 6,5 Hz, 3H : CH₃ en 1 γ) ; de 1,45 à 1,85 (mt, 7H : l'autre H du CH₂ en 5 γ - l'autre H du CH₂ en 5 δ - 1H du CH₂ en 3 β - CH₂ en 3 γ et CH₂ en 2 β) ; 1,81 (d large, J = 13 Hz, 1H : l'autre H du CH₂ en 5 β) ; 2,02 (mt, 1H : l'autre H du CH₂ en 3 β) ; 2,40 (dt, J = 13 et 4 Hz, 1H : 1H du CH₂ en 5 e) ; 2,65 (q, J = 7,5 Hz, 2H : Ar CH₂ de l'éthyle) ; 2,97 et 3,09 (respectivement dd et t, J = 12 et 5 Hz et J = 12 Hz, 1H chacun : CH₂ en 4 β) ; 3,50 et 3,60 (2 mts, 1H chacun : CH₂ en 3 δ) ; 4,13 (dd, J = 8 et 5 Hz, 1H : 3 α) ; 4,49 (d large, J = 13 Hz, 1H : l'autre H du CH₂ en 5 ε) ; 4,70 (mt, 2H : 5 α et 4 α) ; 4,77 (mt, 1H : 2 α) ; 4,83 (dd, J = 10 et 1 Hz, 1H : 1 α) ; 5,50 (d, J = 7 Hz, 1H : 6 α) ; 5,74 (mt, 1H : 1 β) ; 6,09 (d, J = 4 Hz, 1H : NH en 4) ; 6,72 (mf, 1H : NH en 2) ; 7,07 (d, J = 8 Hz, 2H : H Aromatiques en 4 e) ; 7,15 (d, J = 8 Hz, 2H : H Aromatiques en 4 δ) ; de 7,15 à 7,35 (mt, 5H : H Aromatiques en 6) ; 7,40 (dd, J = 8 et 1 Hz, 1H : 1' H₄) ; 7,45 (dd, J = 8 et 4 Hz, 1H : 1' H₅) ; 7,92 (dd, J = 4 et 1 Hz, 1H : 1' H₆) ; 8,40 (mf, 1H : NH en 6) ; 8,50 (d, J = 10 Hz, 1H : NH en 1) ; 11,72 (s, 1H : OH).

### EXEMPLE 33 : Préparation de dérivés de phénylalanine et d'acide phénylpyruvique déja décrits.

La phénylalanine, et les dérivés 4-méthoxyphénylalanine, 4-bromophénylalanine, 4-chlorophénylalanine, 4-iodophénylalanine, 4-trifluorométhylphénylalanine, 4-aminophénylalanine, 3-méthoxyphénylalanine utilisés sont commerciaux.

Les dérivés suivants de phénylalanine peuvent être préparées selon les méthodes décrites dans la littérature.
**4-Diméthylaminophénylalanine (RS)**
D.F. Elliott, A.T. Fuller, C.R. Harrington, J. Chem. Soc., 1948, 85-89.
**4-Diéthylaminophénylalanine (RS)**
Moldaver B.L., Pushkareva Z.V., Zhur. Obshchei Khim,. 31, 1560-1569 (1961);
C.A. 1961, 22226f.; J.A Stock, J. Chem. Soc, 1959, 90-97
**4-Ethylaminophénylalanine (RS)**
F. Bergel, J.A. Stock, J. Chem. Soc, 1959, 90-97.
**4-Phénylphénylalanine (RS)**
J.V. Braun, J. Nelles, Berichte, 66B, 193, 1464-1470.
**4-Méthylphénylalanine (RS)**
R.R.,Herr, T. Enjoki, J.P. Dailey, J. Am. Chem. Soc, 1957, 79, 4229-4231.
**4-Méthylthiophénylalanine (RS) et 4- Ethylthiophénylalanine (R,S)**
R.L.Colescott, R.R.Herr, J.P. Dailey J. Am. Chem. Soc, 1957, 79, 4232-4235.
**4-Méthoxycarbonylphénylalanine (RS)**
H.Cleland, J. Org.Chem., 1969, 34, 747.
**2,4 Diméthylphénylalanine (RS)**
R.R.,Herr, T. Enjoki, J.P. Dailey, J. Am. Chem. Soc, 1957, 79, 4229-4231.
**3,4 Diméthylphénylalanine (RS)**
RR.,Herr, T. Enjoki, J.P. Dailey, J. Am. Chem. Soc, 1957, 79, 4229-4231.
**3-Trifluorométhylphénylalanine (RS), chlorhydrate**
R. Filler and H.Novar, J. Org.Chem, 1960, 25, 733-736.
**4-Aminométhyl phénylalanine (S)**
G.E. Stokker, W.F. Hoffman and C.F. Homnick, J.Org.Chem., 1993, 58, 5015-5017.
**3- Méthylphénylalanine (R,S)**
J.H Burckhalter, V.C Stephens, J.A.C.S. 1951, 73, 56-58.
**4-Acétyl phénylalanine (R,S)**
J.I. Degaw et coll., J. Med.Che., 1969, 11, 225-227
**4-O-Allyl tyrosine (S)**
A. Loffet, H. Zang, Int. J. Pept. Protein Res. ,1993, 42, 346
**4-O-Ethyl tyrosine (S)**
Y. Sasaki et colll, Chem. Pharm, Bull., 1982, 30,4435
**4-Ethyl phénylalanine (RS)**
A. Zhuze et coll., Coll., Czech. Chem. Commmm., 1965, 62, 2648
L' **acide 4-tert-butylphénylpyruvique peut être préparé suivant**
R. Breslow, J.W. Canary, M. Varney, S.T. Waddell and D. Yang, J. Am.Chem.Soc., 1990, 112, 5212-5219.

Les autres dérivés de phénylalanine ont été préparés suivant les exemples 34 à 42 indiqués ci-dessous. Dans ces exemples, les chromatographies flash sont effectuées sous une pression d'azote moyenne de 50 kPa, en utilisant une silice de granulométrie 40-53 µm, selon Still et al., J. Org. Chem., 43, 2923, (1978).

### EXEMPLE 34: Préparation de dérivés de phénylalanine et d'un dérivé d'acide phénylpyruvique par la méthode A.

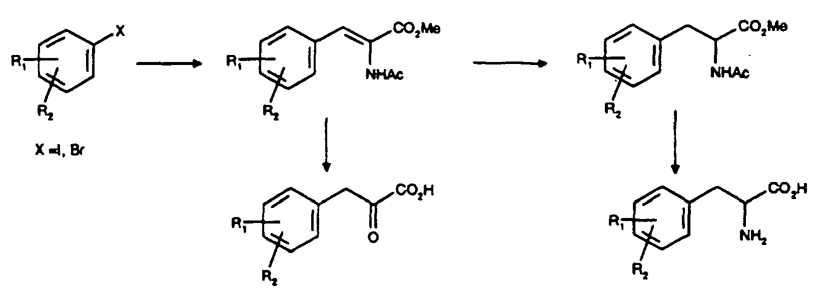

### 34-1: 4-Méthylaminophénylalanine (RS), dichlorhydrate

A 3,70 g de N-acétyl 4-méthylaminophénylalaninate de méthyle on ajoute 37 ml d'acide chlorhydrique 12 N et le mélange est chauffé au reflux sous agitation pendant 8 h. Après une nuit à température ambiante , le milieu réactionnel est concentré à sec sous pression réduite (50 kPa), repris par un mélange de 50 ml de toluène et 50 ml d'éthanol puis à nouveau concentré. Après séchage en dessicateur sous pression réduite (2,6 kPa), on obtient 4,18 g (100%) de dichlorhydrate de 4-méthylaminophénylalanine (RS) sous forme d'un solide beige clair hygroscopique fondant à 158°C.

### 34-2: N-Acétyl 4-méthylaminophénylalaninate de méthyle (RS)

A 4 g de 4-méthylamino 2-acétamido cinnamate de méthyle placé sous atmosphère d'azote dans un autoclave, on ajoute 0,4 g de palladium sur charbon à 10% puis 50 ml d'éthanol absolu. Le mélange est placé sous une pression de 5,5 bars d'hydrogène et chauffé 15 h à 50°C sous agitation. Après stabilisation de la température à 26°C et remise à pression atmosphérique, le milieu réactionnel est filtré sur Clarcel®, rincé à l'éthanol puis concentré à sec sous sous pression réduite (2,6 kPa). On obtient ainsi 3,73 g de N-acétyl 4-méthylaminophénylalaninate de méthyle sous forme de cristaux blancs fondant à 118°C.

### 34-3: 4-Méthylamino 2-acétamido cinnamate de méthyle

Dans un tricol placé sous azote on ajoute 5,75 g de 2-acétamido acrylate de méthyle, 0,185 g d'acétate de palladium, 8,1 g de chlorure de tétrabutyl ammonium et 6,03 g d'hydrogénocarbonate de sodium puis on additionne à ce mélange 6,5 g de 4-iodo N-méthylaniline en solution dans 200 ml de DMF. Le mélange est chauffé 16 h 30 mn à 82°C puis après refroidissement versé sur 1000 ml d'eau distillée. Le milieu est repris par 250 ml de CH₂Cl₂, la phase organique est décantée et la phase aqueuse lavée par 2 fois 250 ml de CH₂Cl₂. Les phases organiques sont rassemblées, séchées sur sulfate de sodium, filtrées et concentrées sous pression réduite (50 kPa) à 70°C pour donner une huile brune qui est purifiée par flash-chromatographie ( éluant AcOEt / cyclohexane puis AcOEt pur).

On obtient ainsi 4 g de 4-méthylamino 2-acétamido cinnamate de méthyle, sous forme d'un solide jaune (Silice Merck 5719, Rf= 0,48) qui est utilisé tel quel.

La N-méthyl-p-iodoaniline peut être préparée suivant : S. Krishnamurthy, Tetrahedron Letters, 33, 3315-3318, 1982.

### 34-4: Acide 4-méthylamino phénylpyruvique

On place dans un ballon 2,4 g de 4-méthylamino 2-acétamido cinnamate de méthyle et 32 ml d'acide chlorhydrique 12 N. Le mélange est chauffé au reflux 3 h puis refroidi et lavé par 2 fois 20 ml d'éther diéthylique. La phase aqueuse est refroidie à -10°C et le précipité obtenu est filtré puis rinçé par un minimum d'acide chlorhydrique froid. Le solide obtenu est séché au dessicateur sous pression réduite pour donner 1,1 g d'acide 4-méthylamino phénylpyruvique sous forme d'un solide beige clair fondant à 210°C.

### 34-5: 3-Fluoro 4-méthylphénylalanine (R,S), chlorhydrate

En opérant comme à l'exemple 34-1 mais à partir de 1,6 g de N-Acétyl (3-fluoro-4 méthyl) phénylalaninate de méthyle, on obtient 0,6 g de chlorhydrate de 3-fluoro 4-méthyl phénylalanine (R,S) sous forme de cristaux blancs fondant à une température supérieure à 260°C.

### 34-6: N-Acétyl (3-fluoro-4 méthyl)phénylalaninate de méthyle( R.S)

En opérant comme à l'exemple 34-2 mais à partir de 1,9 g de (4-méthyl 3-fluoro) 2-acétamido cinnamate de méthyle, de 0,2 g de palladium sur charbon à 10% dans 230 ml d'éthanol, on obtient 1,6 g de N-acétyl (3-fluoro-4 méthyl) phénylalaninate de méthyle sous forme d'une huile incolore (Silice Merck 5719, Rf= 0,46; éluant CH₂Cl₂ / AcOEt 50/50).

### 34-7: (3-Fluoro 4-méthyl) 2-acétamido cinnamate de méthyle

En opérant comme à l'exemple 34-3 mais à partir de 3,6 g de 2-acétamido acrylate de méthyle, 0,12 g d'acétate de palladium, 5,2 g de chlorure de tétrabutyl ammonium, de 3,8 g d'hydrogénocarbonate de sodium et de 4 g de 2-fluoro 4-bromo toluène en solution dans 120 ml de DMF anhydre, on obtient 2,6 g de (3-fluoro 4-méthyl) 2-acétamido cinnamate de méthyle sous forme d'un solide blanc fondant à 163°C.

### 34-8: 4-Trifluorométhoxyphénylalanine (R,S), chlorhydrate ou O-trifluorométhyl tyrosine, chlorhydrate (R,S)

En opérant comme à l'exemple 34-1 mais à partir de 3 g de N-acétyl (4-trifluorométhoxy) phénylalaninate de méthyle et de 30 ml d'acide chlorhydrique 12 N, on obtient 1,5 g de chlorhydrate de 4-trifluorométhoxy phénylalanine (RS) sous forme de cristaux blancs fondant à 260°C.

### 34-9: N-Acétyl (4-trifluorométhoxy)phéylalaninate de méthyle (R,S)

En opérant comme à l'exemple 34-2 mais à partir de 3,1 g de (4-trifluorométhoxy) 2-acétamido cinnamate de méthyle, de 0,3 g de palladium sur charbon à 10% dans 50 ml d'éthanol, on obtient 3 g de N-acétyl (4-trifluorométhoxy) phénylalaninate de méthyle sous forme d'un solide blanc fondant à 80°C.

### 34-10: 4-Trifluorométhoxy 2-acétamido cinnamate de méthyle

En opérant comme à l'exemple 34-3 mais à partir de 4,3 g de 2-acétamido acrylate de méthyle, 0,14 g d'acétate de palladium, 6,1 g de chlorure de tétrabutyl ammonium, de 4,6 g d'hydrogénocarbonate de sodium et de 5 g de 4-trifluorométhoxy bromo benzène en solution dans 150 ml de DMF anhydre.

On obtient 3,1 g de (4-trifluorométhoxy) 2-acétamido cinnamate de méthyle sous forme d'un solide blanc fondant à 135°C.

### 34-11: 3-Méthylthiophénylalanine (RS), chlornydrate

En opérant comme à l'exemple 34-1 mais à partir de 3,3 g de N-acétyl 3-méthylthio phénylalaninate de méthyle et de 40 ml d'acide chlorhydrique 12N, on obtient 1,38 g de chlorhydrate de 3-méthylthio phénylalanine (RS) sous forme de cristaux blancs fondant à 190°C.

### 34-12: N-Acétyl 3-méthylthio phénylalaninate de méthyle (RS)

On place dans un ballon 3,72 g de 3-méthylthio 2-acétamido cinnamate de méthyle en solution dans 100 ml de méthanol et 30 ml de tétrahydrofuranne puis on ajoute 1,4 g de magnésium. Après 20 mn de réaction on refroidi le milieu par un bain de glace puis on ajoute à nouveau 1,4 g de magnésium. Le mélange est agité à température ambiante pendant 18 h, versé sur 1,4 1 d'eau distillée et 300 ml de CH₂Cl₂ puis filtré sur Carcel®. La phase aqueuse est ajustée à pH 6 par addition d'acide chlorhydrique 12 N puis décantée et lavée par 100 ml de CH₂Cl₂. Les phases organiques sont rassemblées, séchées sur sulfate de magnésium, filtrées puis concentrées à sec sous pression réduite pour donner 3,42 g de N-acétyl 3-méthylthio phénylalaninate de méthyle sous forme d'une huile incolore (Silice Merck 5719, Rf= 0,5 ; AcOEt).

### 34-13: 3-Méthylthio 2-acétamido cinnamate de méthyle

En opérant comme à l'exemple 34-3 mais à partir de 5,6 g de 2-acétamido acrylate de méthyle, 0,18 g d'acétate de palladium, 8,2 g de chlorure de tétrabutyl ammonium, de 5,86 g d'hydrogénocarbonate de sodium et de 6,5 g de 3-iodo 1-méthylthiobenzène en solution dans 160 ml de DMF anhydre, on obtient 4,8 g de (3-méthylthio) 2-acétamido cinnamate de méthyle sous forme d'un solide blanc fondant à 139°C.

### 34-14: 3-Iodométhylthiobenzène

On place sous agitation dans un tricol 20 ml d'eau distillée et 20 ml d'acide chlorhydrique 12 N puis on ajoute par une ampoule de coulée 10 ml de 3-méthylthio aniline. Le mélange est tiédi pour assurer la dissolution puis refroidi à 5°C. On ajoute ensuite lentement par une ampoule de coulée 5,86 g de nitrite de sodium en solution dans 15 ml d'eau en maintenant la température entre 5 et 8°C. 20mn après la fin de l'addition, 13,57 g de iodure de potassium en solution dans 15 ml d'eau sont ajoutés en 10mn puis le mélange est agité 15 h à température ambiante. L'huile formée est séparée de la phase aqueuse par décantation, puis additionnée d'une solution aqueuse de thiosulfate de sodium. La phase aqueuse est décantée, et le produit extrait par 100 ml de dichlorométhane. La phase organique est lavée avec 100 ml d'eau, la phase aqueuse ajustée à pH 9 avec de la soude concentrée puis décantée. La phase organique est lavée par 2 fois 100 ml d'eau, décantée, séchée sur sulfate de magnésium, filtrée puis concentrée à sec sous pression réduite (50 kPa) à 40°C. Le produit résultant est purifié par chromatographie flash (éluant cyclohexane) pour donner 13 g de 3-iodo 1-méthylthiobenzène sous forme d'un liquide jaune (Silice Merck 5719, Rf= 0,8 / cyclohexane).

### EXEMPLE 35 : Préparation de dérivés de phénylalanine par la méthode B.

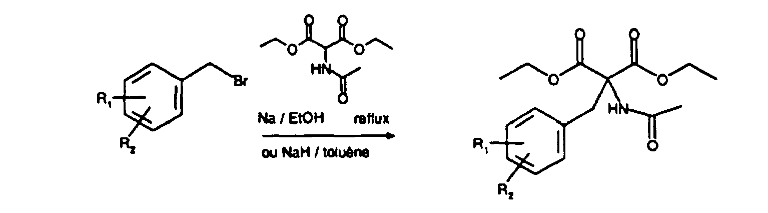

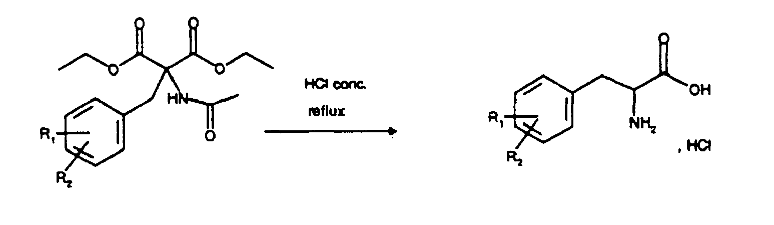

### 35-1: 4-tert-Butylphénylalanine (RS)

Dans un tricol surmonté d'un réfrigérant sont additionnés 25 g de 4-(tert butyl) benzyl acétamidomalonate de diéthyle et 250 ml d'acide chlorhydrique à 37%. Le mélange est agité et chauffé au reflux jusqu'à ce qu'il n'y ait plus de dégagement gazeux. Après refroidissement du milieu réactionnel le précipité obtenu est filtré puis recristallisé dans l'acétonitrile pour donner 25, 6 g de chlorhydrate de 4-tert-butylphénylalanine (R,S) sous forme d'un solide blanc fondant à 234°C. (Voir également Journal of the Takeda Research Laboratories Vol. 43; N°3/4, Déc1984 p53-76).

### 35-2: 4-(tert butyl) benzyl acétamidomalonate de diéthyle

Dans un tricol surmonté d'un réfrigérant sont additionnés sous atmosphère d'azote, 25 g de bromure de 4-tert-butyl benzyle, 50 ml de toluène anhydre et 3,1 g d'hydrure de sodium en suspension dans l'huile à 80% puis 21,8 g d' acétamido malonate de diéthyle. Le mélange est chauffé à 110°C pendant 17 h. Après refroidissement on ajoute lentement à l'aide d'une ampoule de coulée, 15 ml d'éthanol absolu puis 15 ml d'éthanol à 50% puis 50 ml d'eau. La phase organique est décantée et la phase aqueuse lavée par 3 fois 50 ml d'éther diéthylique. Les phases organiques sont réunies, lavées à l'eau puis séchées sur sulfate de sodium. Après filtration et concentration sous pression réduite, le produit est cristallisé dans l'éther de pétrole pour donner 25 g de 4-(tert-butyl) benzyl acétamidomalonate de diéthyle sous forme d'un solide blanc fondant à 80°C.

### 35-3: 3-Méthylaminophénylalanine (R,S), dichlorhydrate

En opérant comme à l'exemple 35-1 mais à partir de 1,17 g 3-méthylamino benzyl acétamidomalonate de diéthyle et 20 ml d'acide chlorhydrique 12 N, on obtient 1, 03 g d'un solide jaune beige. Celui-ci est dissous dans 20 ml d'éthanol absolu et additionné de 0,4 g de noir animal. La solution est filtrée sur Carcel®, puis filtrée et concentrée sous pression réduite (50 kPa) La même opération est recommencée avec 1 g de noir animal et le solide obtenu trituré dans 20 ml d'éther. Aprés filtration et séchage sous pression réduite (2,7 kPa) à 50°C, on obtient 0,65 g de dichlorhydrate de 3-méthylaminophénylalanine (R,S) sous forme d'une poudre blanche fondant vers 135°C (décomposition).

### 35-4: 3-Méthylamino benzyl acétamidomalonate de diéthyle

On place dans un tricol maintenu sous atmosphère d'azote 3,11 ml d'anhydride acétique. On ajoute ensuite en 3 mn à 0°C, 1,51 ml d'acide formique puis on chauffe à 50°C pendant 2 heures. On laisse le mélange revenir à température ambiante en agitant pendant 3h 20 et on ajoute 4 ml de THF anhydre sous azote et on refroidi à - 20°C. On ajoute en 10 mn, une solution de 4 g de 3-aminobenzyl acétamidomalonate de diéthyle dans un mélange de 15 ml de THF anhydre et de 15 ml de dichlorométhane anhydre. L'agitation est poursuivie 1h 10mn à -20°C puis à 20°C pendant 16 h. Le mélange réactionnel est concentré à sec sous pression réduite (50 kPa) à 30°C, puis coévaporé avec 30 ml de toluène anhydre pour donner un solide blanc qui est dissous dans un mélange de 10 ml de THF anhydre et de 20 ml de dichloro 1,2 éthane anhydre puis placé dans un tricol sous azote.

Le milieu est refroidi à -5°C puis 1,55 ml de complexe borane-diméthylsulfure (solution 2M dans le THF) est ajouté en 10 mn. On laisse le milieu revenir à température ambiante et la solution et chauffée 3 h au reflux puis agitée 15 h à température ambiante. Le milieu réactionnel est refroidi à 0°C puis on ajoute en 25 mn, 10 ml de MeOH. On agite 45 mn à 0°C puis 30 mn à température ambiante. On refroidit à 0°C puis on fait barboter HCl gaz jusqu'à pH 2. On chauffe 1 h à reflux puis le mélange est concentré à sec sous pression réduite à 30°C pour donner 5 g d'un produit qui est repris par 30 ml d'une solution aqueuse de NaHCO₃ et de 30 ml de CH₂Cl₂. La phase organique est décantée et la phase aqueuse lavée par 20 ml d'eau. Les phases organiques sont réunies, séchées sur sulfate de magnésium, filtrées puis concentrées à sec sous pression réduite (2,6 kPa) pour donner 3,43 g d'une huile jaune qui est purifiée par chromatographie flash (éluant AcOEt-cyclohexane 50/50). On obtient ainsi après séchage sous pression réduite (2,7 kPa) à 20°C, 1,18 g de 3-méthylamino benzyl acétamidomalonate de diéthyle sous forme d'un solide beige clair fondant à 122°C.

### 35-5: 3-Aminobenzyl acétamidomalonate de diéthyle

Le 3-amino benzyl acétamidomalonate de diéthyle peut être préparé comme décrit dans :
T.S. Osdene, D.N.Ward, W.H. Chapman and H. Rakoff, J. Am. Chem. Soc., 81, 1959, 3100-3102.

### 35-6: 3-Ethylaminophénylalanine (R,S), dichlorhydrate

En opérant comme à l'exemple 34-1 mais à partir de 2 g de N-acétyl 3-éthylamino phénylalaninate d'éthyle (R,S) et de 30 ml d'acide chlorhydrique 12N, on obtient 1,7 g de dichlorhydrate de 3-éthylamino phénylalanine (R,S) sous forme d'un solide beige clair hygroscopique contenant 10% molaire de dichlorhydrate de 3-diéthylamino phénylalanine (R,S).

### 35-7: N-Acétyl 3-éthylamino phénylalaninate d'éthyle(RS)

On place dans un ballon sous atmosphère d'azote, 3 g de N-acétyl 3-amino phénylalaninate d'éthyle (R,S), 40 ml d'éthanol et 14 g de nickel de Raney préalablement lavé à l'eau distillée et à l'éthanol. Le mélange est chauffé au reflux 19 h, refroidi, filtré sur Carcel® puis concentré à sec sous pression réduite (50 kPa) pour donner 3,07 g d'une huile incolore qui est purifiée par chromatographie flash (éluant AcOEt) pour donner 2,1 g de N-acétyl 3-éthylamino phénylalaninate d'éthyle (R,S) sous forme d'une huile incolore (Silice Merck 5719, Rf= 0,6 :AcOEt) contenant 10% de N-acétyl 3-diéthylamino phénylalaninate d'éthyle (R,S).

### 35-8: N-acétyl 3-amino phénylalaninate d'éthyle(R.S)

25 g d'un mélange de N-acétyl 3-nitro phénylalaninate d'éthyle (R,S) (75% mol./mol.) et de 3-nitro benzyl acétamidomalonate de diéthyle (25% mol./mol.) sont placés sous azote dans un autoclave. On ajoute 2,5 g de palladium sur charbon à 10% puis 200ml de dichlorométhane. Le mélange est placé sous une pression de 9 bars d'hydrogène puis agité à 18°C pendant 4 h. Après remise à la pression atmosphérique, le milieu réactionnel est filtré sur Clarcel®, rincé par du dichlorométhane puis concentré à sec sous pression réduite (50 kPa) pour donner un solide qui est recristallisé dans 450 ml d'eau distillée au reflux en présence de 4 g de noir animal 3S. Après filtration à chaud sur Claroel® la cristallisation est abandonnée à 4°C, les cristaux sont filtrés puis séchés pour donner 9,9 g de N-acétyl 3-amino phénylalaninate d'éthyle (R,S) sous forme d'un solide beige clair fondant à 106°C et contenant 5% de 3-amino benzyl acétamido malonate de diéthyle.

### 35-9: N-acétyl 3-nitro phénylalaninate d'éthyle(R,S) et 3-nitro benzol acétamidomalonate de diéthyle.

Dans un tricol surmonté d'un réfrigérant sont placés sous atmosphère d'azote, 600 ml d'éthanol absolu puis 7,9 g de sodium. Après dissolution totale, on ajoute 74,5 g d' acétamido malonate de diéthyle puis 60 g de chlorure de 4-nitro benzyle dans 200 ml d'éthanol anhydre. Le mélange est chauffé au reflux pendant 16h 30mn. Après refroidissement le milieu réactionnel est concentré sous pression réduite (50 kPa) puis repris par un mélange de 500 ml de CH₂Cl₂ et de 500 ml d'eau. Le pH est ajusté à 7 par addition d'acide sulfurique 0,5N puis la phase organique est décantée et la phase aqueuse lavée par 2 fois 200 ml de CH₂Cl₂. Les phases organiques sont réunies, lavées avec 200 ml d'eau saturée en bicarbonate de sodium, décantée puis séchées sur sulfate de magnésium. Après filtration et concentration sous pression réduite (50 kPa), le produit est recristallisé dans 600 ml d'éthanol au reflux pour donner après cristallisation à température ambiante, filtration et séchage, 70,4 g de 3-nitro benzyl acétamidomalonate de diéthyle.sous forme de cristaux blancs fondant à 156°C. Les eaux-mères sont concentrées puis purifiées par chromatographie flash (éluant AcOEt) pour donner 25,6 g d' un mélange de N-acétyl 3-nitro phénylalaninate d'éthyle (75% mol/mol) et de 3-nitro benzyl acétamidomalonate de diéthyle (25% mol/mol) sous forme d'un solide beige clair utilisé tel que dans l'étape suivante.

### 35-10 : 3-Diméthylamino phénylalanine (RS), dichlorhydrate

En opérant comme à l'exemple 35-1 mais à partir de 0,72 g de N-acétyl 3-diméthylamino phénylalaninate d'éthyle (RS) et de 8,6 ml d'acide chlorhydrique à 10N, on obtient après évaporation un solide qui est trituré dans 50ml d'acétone, filtré puis séché sous pression réduite (2,7 kPa) à 40°C. On obtient 0,68 g (93%) de dichlorhydrate de 3-diméthylamino phénylalanine (RS) sous forme d'un solide blanc fondant vers 120°C (décomposition).

### 35-11: N-acétyl 3-diméthylamino phénylalaninate d'éthyle (RS)

On place dans un tricol sous atmosphère d'azote, 4g de N-acétyl 3-amino phénylalaninate d'éthyle (RS), préparé comme décrit à l'exemple 35-8 dans 15ml de DMF et on ajoute 5,5 ml de triéthylamine puis 2,5 ml de iodure de méthyle et 4 ml de dichlorométhane en maintenant la température vers 30°C à l'aide d'un bain de glace. Le mélange est ensuite chauffé 18h à 35°C. On ajoute alors lentement 1ml de iodure de méthyle en solution dans 1ml de DMF en maintenant la température vers 30°C, puis 2,2ml de triéthylamine puis le mélange est chauffé 5h supplémentaires à 35°C. Le mélange est ramené à température ambiante puis extrait par 100ml d'acétate d'éthyle et 150l d'eau distillée. La phase aqueuse est décantée puis relavée par 2 fois 70ml d'acétate d'éthyle. Les phases organiques sont réunies, lavées par 2 fois 80 ml d'eau distillée puis par 50ml d'eau distillée saturée en NaCl. La phase organique est décantée, séchée sur sulfate de magnésium, filtrée, puis concentrée à sec sous pression réduite pour donner 2,4g d'un produit qui est purifié par chromatographie flash (dichlorométhane, MeOH 90/10). On obtient ainsi 0,72g (16%) de 3-N-acétyl 3-diméthylamino phénylalaninate d'éthyle (RS) sous forme de cristaux jaunes.

### EXEMPLE 36: Préparation de dérivés de phénylalanine par la méthode C.

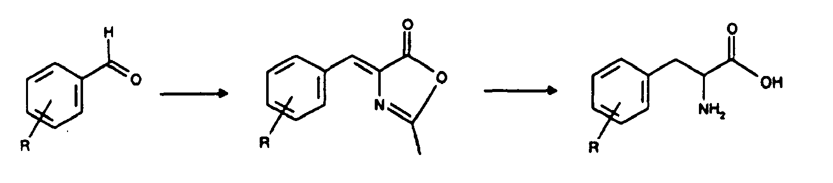

### 36-1: 4-Isopropylphénylalanine (R,S)

On place dans un tricol 7 g de phosphore rouge et 8 g de 4-(isopropyl benzylidène) 2-méthyl 5-oxazolone dans 45 ml d'anhydride acétique puis on ajoute lentement sous agitation à l'aide d'une ampoule de coulée 35 ml d'acide iodhydrique à 57%. En fin d'addition le mélange est chauffé 3h 30mn au reflux puis laissé 3 jours à température ambiante. Le milieu réactionnel est filtré, le solide obtenu rincé par 2 fois 10 ml d'acide acétique puis le filtrat concentré à sec sous pression réduite. Le résidu obtenu est repris par 100 ml d'eau distillée, concentré à sec sous pression réduite pour donné un solide qui est repris dans 50 ml d'eau distillée puis extrait par 3 fois 50 ml d'éther diéthylique après addition de 0,5g de sulfite de sodium. L'éther est décanté et la phase aqueuse placée sous pression réduite pour éliminer les traces d'éther diéthylique. On ajoute à la phase aqueuse 2 g de noir animal, on chauffe à 40-50°C, on filtre sur Clarcel® puis on rince avec un minimum d'eau. Le pH est ajusté à 5 par addition, à 4°C, d'ammoniaque à 32%. Le précipité obtenu est filtré à froid, rincé par 2 fois 10 ml d'eau, 10 ml 'éthanol puis par 2 fois 10 ml d'éther pour donner après séchage sous pression réduite à 20°C, 3,97 g de 4-isopropylphénylalanine (R,S) sous forme d'un solide blanc fondant à une température supérieure à 260°C. (Voir également Journal of the Takeda Research Laboratories Vol. 43; N°3/4, Déc1984 p53-76).

### 36-2: 4-(isopropyl benzylidène) 2-méthyl 5-oxazolone

On place dans un ballon muni d'un réfrigérant, 18,52 g de N-acétylglycine, 10,6 g d'acétate de sodium, 20 ml de 4-isopropylbenzaldéhyde et 57 ml d'anhydride acétique. On agite 30 mn puis le mélange est agité 1 h à 110°C puis 15 h à température ambiante. Le milieu réactionnel est versé sur 600 ml d'eau et 400 ml d'éther de pétrole préalablement chauffé à 50°C. La phase organique est décantée et la phase aqueuse lavée par 2 fois 150 ml d'éther de pétrole.

Les phases organiques sont rassemblées, séchées sur sulfate de magnésium, filtrées et concentrées sous pression réduite jusqu'à 100 ml et obtention d'un précipité. Celui-ci est filtré, lavé par 2 fois 50 ml de pentane pour donner 8,2 g de 4-(isopropyl benzylidène) 2-méthyl 5-oxazolone sous forme d'un solide jaune fondant à 77°C.

### 36-3: 4-butylphénylalanine (R,S)

En opérant comme à l'exemple 36-1 mais à partir de 1,49 g de phosphore rouge, de 1,8 g de 4-(butyl benzylidène) 2-méthyl 5-oxazolone dans 9,23 ml d'anhydride acétique et de 7,39 ml d'acide iodhydrique à 57%, on obtient 0,35 g de 4-butylphénylalanine (R,S) sous forme d'un solide beige clair fondant à une température supérieure à 260°C.

### 36-4: 4-(bulyl benzylidène) 2-méthyl 5-oxazolone

En opérant comme à l'exemple 36-2 mais à partir de 8,43 g de N-acétylglycine, 4,92 g d'acétate de sodium, 9,8 g de 4-butylbenzaldéhyde et 26 ml d'anhydride acétique, on obtient1,89 g de.4-(butyl benzylidène) 2-méthyl 5-oxazolone, sous forme d'un solide jaune fondant à 74°C.

### EXEMPLE 37 : Préparation d'un dérivé de phénylalanine par la méthode D.

### 37-1: 3-Ethoxy phénylalanine(R,S), chlorhydrate (ou 3-O-Ethyltyrosine (R,S), chlorhydrate)

On place dans un ballon 1 g de N-tert butoxy carbonyl 3-éthoxy phénylalanine (R,S) en solution dans 3,6 ml de dioxanne chlorhydrique puis le mélange est agité 5 h à température ambiante. Le précipité formé est filtré, rincé par du dioxanne puis à l'éther puis séché sous pression réduite (2,7 kPa) à 40°C pour donner 0,65 g de 3-éthoxy phénylalanine (R,S), chlorhydrate sous forme d'un solide blanc fondant à 200°C.

### 37-2: N-tert Butoxy carbonyl 3-éthoxy phénylalanine (R,S)

On place dans un ballon 1,33 g de N-tert butoxy carbonyl 3-éthoxy phénylalaninate d'éthyle (R,S) en solution dans 8 ml de méthanol puis on ajoute 8 ml de soude 1N. Après 18 h d'agitation à température ambiante, le mélange est évaporé sous pression réduite, puis acidifié par 8,56 ml d'acide chlorhydrique 1N. Le produit est extrait par 2 fois 10 ml d'acétate d'éthyle, les phases organiques sont réunies, lavées par 2 fois 10 ml d'eau, séchées filtrées puis concentrées à sec sous pression réduite pour donner 1 g de N-tert butoxy carbonyl 3-éthoxy phénylalanine (R,S) sous forme d'une huile jaune (Silice Merck 5719, Rf= 0,7, éluant : toluène 80 / MeOH 10 /diéthylamine 10).

### 37-3: N-tert Butoxy carbonyl 3-éthoxy phénylalaninate d'éthyle (R,S)

On place dans un tricol sous atmosphère d'azote, 1,5 g de N-tert butoxy carbonyl 3-tyrosine (R,S) en solution dans 7,5 ml de DMF sec puis on ajoute, 0,508 g d'hydrure de sodium à 50% dans l'huile. Après 2 h d'agitation à température ambiante on ajoute 0,86 ml de iodoéthane puis le mélange est agité 4 h à température ambiante. Le milieu est filtré, le solide résultant lavé par 3 fois 10 ml d'eau puis 2 fois 10 ml d'éther de pétrole pour donner après séchage sous pression réduite (2,7 kPa) à 30°C, 1,33 g de N-tert butoxy carbonyl 3-éthoxy phénylalaninate d'éthyle (R,S) sous forme d'un solide blanc.

### 37-4: N-tert Butoxy carbonyl 3-tyrosine (R.S)

On place sous agitation dans un tricol, 18 g de 3-tyrosine (R,S) en solution dans 180 ml de dioxanne puis on ajoute 99 ml de soude 1N puis 26 g de di-tert butyl dicarbonate en solution dans 160 ml de dioxanne. Après 36 h d'agitation le milieu est concentré sous pression réduite à 30°C, repris avec 100 ml d'eau distillée, acidifié avec de l'acide chlorhydrique 1N jusqu'à pH 5 puis extrait par 2 fois 200 ml d'acétate d'éthyle. La phase organique est séchée sur sulfate de magnésium, filtrée puis concentrée à sec sous pression réduite à 30°C, pour donner 30 g de N-tert butoxy carbonyl 3-tyrosine (R,S) sous forme d'un solide blanc (Silice Merck 5719, Rf= 0,25, éluant : toluène 80, MeOH 10, diéthylamine 10).

### EXEMPLE 38: Préparation de dérivés de phénylalanine par la méthode E.

### 38- 1: 4-Diallylaminophénylalanine (RS), dichlorhydrate

En opérant comme à l'exemple 35-1 mais à partir de 5,8 g de 4-diallylaminobenzyl acétamido malonate de diéthyle et de 48 ml d'acide chlorhydrique 10N, on obtient après évaporation un solide qui est trituré dans 50ml d'acétone, filtré puis trituré dans 10ml de dichlorométhane, filtré, puis rincé par 3 fois 10 ml d'éther éthylique. Aprés séchage sous pression réduite (2,7 kPa) à 40°C, on obtient 4,41 g de dichlorhydrate de 4-diallylaminophénylalanine (RS) sous forme d'un solide blanc cassé fondant vers 135°C (décomposition).

### 38- 2: 4-Allylaminophénylalanine (RS), dichlorhydrate

En opérant comme à l'exemple 35-1 mais à partir de 3,27 g de 4-allylaminobenzyl acétamido malonate de diéthyle et de 30 ml d'acide chlorhydrique 10N, on obtient après évaporation un solide qui est trituré dans 50ml d'acétone, filtré puis séché sous pression réduite (2,7 kPa) à 40°C. On obtient 2,3 g de dichlorhydrate de 4-allylaminophénylalanine (RS) sous forme d'un solide blanc fondant vers 134°C (décomposition).

### 38-3: 4-Diallylaminobenzyl acétamido malonate de diéthyle et 4-Allylaminobenzyl acétamido malonate de diéthyle

On place dans un tricol surmonté d'une ampoule de coulée et maintenu sous atmosphère d'azote 10g de 4-aminobenzylacétamidomalonate de diéthyle en solution dans 150ml de DMF. On ajoute lentement à température ambiante, 6,57 ml de bromure d'allyle puis 10,76ml de triéthylamine sous agitation. Après 19 h d'agitation on ajoute à nouveau, 1,31ml de bromure d'allyle et 2,15ml de triéthylamine et le mélange est agité 26h. Le milieu réactionnel est versé sur 1,51 d'eau distillée et extrait par 11 d'acétate d'éthyle. La phase aqueuse est décantée, lavée par 2 fois 500ml d'acétate d'éthyle. Les phases organiques sont réunies, lavées par 500ml d'eau distillée puis par 500ml d'eau saturée en chlorure de sodium, décantées, séchées sur sulfate de magnésium, filtrées puis concentrées à sec pour donner une huile marron qui est purifiée par chromatographie flash (éluant CH₂Cl₂ 90/ AcOEt 10) pour donner 6,66 g de 4-diallylaminobenzyl acétamido malonate de diéthyle sous forme d'un solide beige fondant à 94-96°C (Rf =0,6 AcOEt 50/ cyclohexane 50) et 3,49g de 4-allylaminobenzyl acétamido malonate de diéthyle sous forme d'un solide beige fondant à 104-106°C (Rf =0,45 AcOEt 50/ cyclohexane 50)

Le 4-aminobenzyl acétamido malonate de diéthyle peut être préparé comme décrit dans J.B. Burckhalter, VC Stephens, J. Am. Chem. Soc., 56, 1951, 73.

### EXEMPLE 39: Préparation de dérivés de phénylalanine par la méthode F

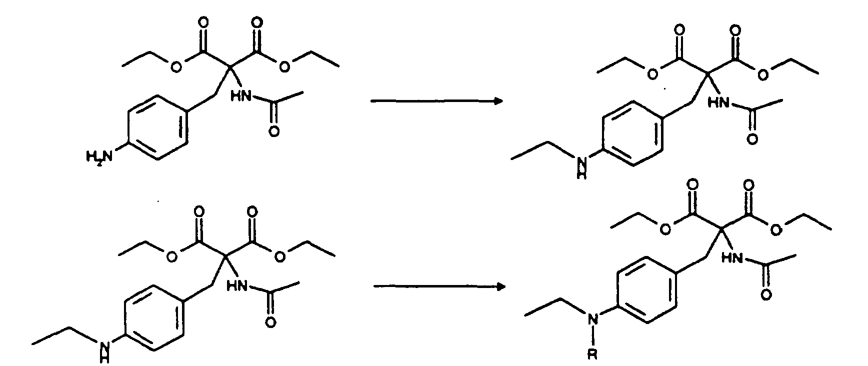

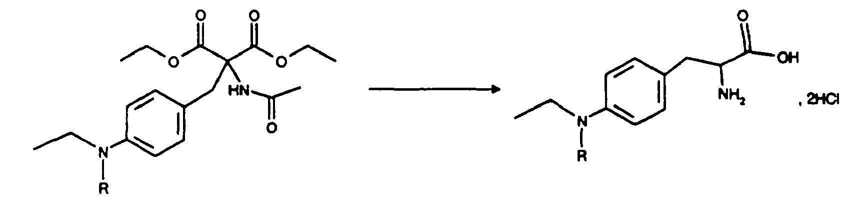

### 39-1: 4-Ethyl isopropyl phénylalanine (RS), dichlorhydrate

En opérant comme à l'exemple 35-1 mais à partir de 2,9 g de 4-éthyl isopropylbenzyl acétamido malonate de diéthyle et de 24,6 ml d'acide chlorhydrique 10N, on obtient après évaporation un solide qui est trituré dans 20 ml d'acétone, filtré puis séché sous pression réduite (2,7 kPa) à 40°C. On obtient 2 g de dichlorhydrate de 4-éthyl isopropylaminophénylalanine (RS) sous forme d'un solide blanc fondant vers 147°C (décomposition).

### 39-2: 4-Ethyl isopropyl aminobenzyl acétamido malonate de diéthyle

On place dans un tricol maintenu sous atmosphère d'azote, 15g de 4-éthyl aminobenzylacétamido malonate de diéthyle dans 70ml de THF, on ajoute 6,4ml de 2-iodopropane puis 8,4ml de 1,5-diazabicyclo[4-3-0]non-5-ène puis le mélange est chauffé 24h à 60°C. On ajoute alors 2,13ml de 2-iodopropane, puis 8,4ml de 1,5-diazabicyclo[4-3-0]non-5-ène puis le mélange est chauffé 24h supplémentaires à 60°C.

Le mélange est ramené à température ambiante puis extrait par 50ml de dichlorométhane et 50ml d'eau distillée. La phase aqueuse est décantée puis relavée par 2 fois 30ml de dichlorométhane. Les phases organiques sont réunies, lavées par 60 ml d'eau distillée puis par 50ml d'eau distillée saturée en NaCl. la phase organique est décantée, séchée sur sulfate de magnésium, filtrée, puis concentrée à sec sous pression réduite pour donner 16,2g d'un produit qui est purifié par chromatographie flash (dichlorométhane, MeOH 90/10). On obtient ainsi 4,59g d'un produit qui est recristallisé dans 45ml de cyclohexane pour donner 3,44g de 4-éthyl isopropyl aminobenzyl acétamido malonate de diéthyle sous forme de cristaux blancs fondants à 80°C.

### 39-3: 4-Ethylaminobenzylacétamido malonate de diéthyle

Le 4-éthyl aminobenzylacétamido malonate de diéthyle peut être préparé en opérant comme à l'exemple 35-7 mais à partir de 22 g de 4-aminobenzyl acétamidomalonate de diéthyle, 500 ml d'éthanol et 70 g de nickel de raney. On obtient ainsi, 23,8 g de 4-éthylamino benzylacétamido malonate de diéthyle sous forme d'un solide blanc cassé fondant à 136°C.

### 39-4: 4-Allyl éthylaminophénylalanine (R,S), dichlorhydrate

En opérant comme à l'exemple 35-1 mais à partir de 4,54 g de 4-allyl éthylbenzyl acétamido malonate de diéthyle et de 37,9 ml d'acide chlorhydrique à 10N, on obtient après évaporation un solide qui est séché sous pression réduite (2,7 kPa) à 40°C. On obtient 3,67g de dichlorhydrate de 4-allyl éthylaminophénylalanine (RS) sous forme d'un solide brun fondant vers 130°C (décomposition).

### 39-5: 4-Allyl éthylaminobenzyl acétamido malonate de diéthyle

En opérant comme à l'exemple 39-2 mais à partir de 8 g de 4-éthylaminobenzylacétamido malonate de diéthyle, 4 ml de bromure d'allyle, 5,82 ml de 1,5-diazabicyclo[4-3-0]non-5-ène, dans 50ml de THF, on obtient après purification par chromatographie flash (éluant CH2Cl2 / AcOEt 90-10 en volume) 5, 6g d'un solide qui est recristallisé dans 35ml de cyclohexane. On obtient ainsi 5,43g de 4-allyl éthylaminobenzyl acétamido malonate de diéthyle sous forme d'un solide blanc fondant à 86°C.

### 39-6: 4-Ethyl propylaminophénylalanine (RS), dichlorhydrate

En opérant comme à l'exemple 35-1 mais à partir de 2,5 g de 4-éthyl pcopylaminobenzyl acétamido malonate de diéthyle et de 21 ml d'acide chlorhydrique à 10N, on obtient après évaporation un solide qui est séché sous pression réduite (2,7 kPa) à 40°C. On obtient 2 g (97%) de dichlorhydrate de 4-éthyl propylaminophénylalanine (RS) sous forme d'un solide blanc fondant vers 147°C (décomposition).

### 39-7: 4-Ethyl propylaminobenzyl acétamido malonate de diéthyle

En opérant comme à l'exemple 39-2 mais à partir de 10 g de 4-éthylaminobenzylacétamido malonate de diéthyle, 5,6 ml de 1-iodo propane, 7,2 ml de 1,5-diazabicyclo[4-3-0]non-5-ène, dans 70ml de THF, on obtient après 36heures de réaction puis purification par chromatographie flash (éluant CH2Cl2 / MeOH 97-3 en volume), 2,8g d'un solide qui est recristallisé dans 26 ml de cyclohexane.On obtient ainsi 2,9g de 4-éthyl propylaminobenzyl acétamido malonate de diéthyle sous forme d'un solide blanc fondant à 84-86°C.

### 39-8: 4-Ethyl méthylcyclopropylaminophénylalanine (RS) dichlorhydrate

En opérant comme à l'exemple 35-1 mais à partir de 3 g de 4-éthyl méthylcyclopropylaminobenzyl acétamido malonate de diéthyle et de 25 ml d'acide chlorhydrique 10N, on obtient après 3 jours de réaction, puis évaporation un solide qui est trituré dans 40 ml d'acétone, filtré puis séché sous pression réduite (2,7 kPa) à 40°C. On obtient 2,24 g de dichlorhydrate de 4-éthyl méthylcyclopropylaminophénylalanine (RS) sous forme d'un solide blanc fondant vers 140°C (décomposition).

### 39-9: 4-Ethyl méthylcyclopropylaminobenzyl acétamido malonate de diéthyle

En opérant comme à l'exemple 39-2 mais à partir de 8 g de 4-éthylaminobenzylacétamido malonate de diéthyle, 2,6 ml de bromométhylcyclopropane, 2,97 ml de 1,5-diazabicyclo[4-3-0]non-5-ène, dans 50ml de THF, on obtient après 3 jours de réaction puis purification par chromatographie flash (éluant CH2Cl2 / AcOEt 90-10 en volume), 3,3 g de 4-éthyl méthylcyclopropylaminobenzyl acétamido malonate de diéthyle sous forme d'un solide blanc fondant à 112-114°C.

### EXEMPLE 40: Préparation de dérivés de phénylalanine par la méthode G

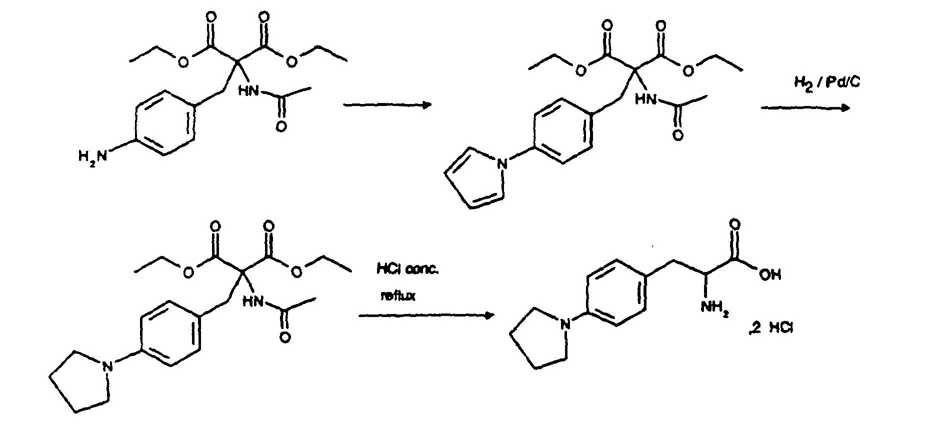

### 40-1: 4-(1-pyrrolidinyl) phénylalanine (RS) dichlorhydrate

En opérant comme à l'exemple 35-1 mais à partir de 1,5 g de 4-(1-pyrrolidinyl) benzyl acétamidomalonate de diéthyle et de 40 ml d'acide chlorhydrique 5N, on obtient après évaporation un solide qui est trituré dans 15 ml d'acétone, filtré puis séché sous pression réduite (2,7 kPa) à 40°C. On obtient 0,6 g de dichlorhydrate de 4-(1-pyrrolidinyl) phénylalanine (RS) sous forme d'un solide blanc cassé.

### 40-2: 4-(1-pyrrolidinyl) benzyl acétamidomalonate de diéthyle

On place dans un autoclave 4g de 4-(1-pyrrolyl) benzyl acétamidomalonate de diéthyle en solution dans 100ml de MeOH et 1g de palladium sur charbon à 10%. Après avoir purgé 3 fois à l'azote, le produit est hydrogéné sous une pression de 14 bars d'hydrogène à 19°C. Après 25 heures d'agitation, l'hydrogénation est arrêtée, le produit filtré sur Clarcel®, rincé au dichlorométhane puis la solution concentrée sous pression réduite pour donner 3,85 g d'un solide qui est trituré dans un mélange de 50ml d'heptane et de 10 ml d'éther éthylique. Le solide obtenu est filtré, séché puis purifié par chromatographie flash (éluant CH₂Cl₂ / acétone 90/10 en volume) pour donner 1,6g de 4-(1-pyrrolidinyl) benzyl acétamidomalonate de diéthyle sous forme d'un solide blanc fondant à 132°C

### 40-3: 4-(1-pyrrolyl) benzyl acétamidomalonate de diéthyle

On place dans un tricol maintenu sous azote, 4,6 g de 4-aminobenzyl acétamidomalonate de diéthyle dans 104 ml d'acide acétique.On ajoute 7,02 g d'acétate de sodium puis 1,87 ml de 2,5-diméthoxytétrahydrofuranne. Le mélange est chauffé à 65°C pendant 1h 15mn, refroidi, puis extrait par 100ml de dichlorométhane et 100ml d'eau distillée. La phase aqueuse est décantée puis lavée par 3 fois 100 ml de dichlorométhane. Les phases organiques sont réunies, lavées par 100 ml d'eau puis par 100 ml d'une solution saturée en NaCl, décantées puis séchées sur sulfate de magnésium, filtrées puis évaporées à sec sous pression réduite(50k Pa) pour donner 6,2g d'un solide qui est purifié par chromatographie flash (éluant CH2Cl2 / acétone 75/25 en volume). On obtient ainsi 3,57 g de 4-(1-pyrrolyl) benzyl acétamidomalonate de diéthyle sous forme d'un solide beige fondant à 110°C.

### EXEMPLE 41: Préparation de dérivés de phénylalanine par la méthode H

### 41-1: 4-Ethylthiométhyl phénylalanine (RS)

On place dans un tricol maintenu sous azote, 300ml de méthanol anhydre puis on ajoute sous agitation 1, 72g de méthylate de sodium puis 5,55ml d'éthyl mercaptan. Le solvant est concentré sous pression réduite à 40°C pour donner 8,5 g du sel de sodium de l'éthylmercaptan qui est mis en solution dans 100ml de THF anhydre. On ajoute à température ambiante 3,6g de 4-chlorométhyl phénylalanine (RS) puis le mélange est chauffé au reflux pendant 18h. Le solvant est évaporé sous pression réduite à 40°C et le résidu repris par 100ml d'eau distillée. La solution trouble obtenue est acidifiée par 5 ml d'acide acétique. Le précipité obtenu est filtré, rincé à l'eau distillée puis séché à 60)C sous pression réduite pour donner 3,6g d'un solide qui est purifié par chromatographie flash (éluant AcOEt 60, AcOH 12, eau 10). On obtient ainsi 256 mg de 4-éthylthiométhyl phénylalanine (RS), sous forme d'un solide blanc fondant à 251°C.

La 4-chlorométhyl phénylalanine (RS) peut être obtenue par analogie avec la 4-chlorométhyl phénylalanine (S) décrite dans : RGonzalez-Muniz, F. Cornille, F. Bergemn, D. Ficheux, J. Pothier, C. Durieux and B. Roques, Int. J. Pept. Protein. Res., 1991,37 (41), 331-340.

### EXEMPLE 42: Préparation de dérivés de phénylalanine par la méthode I

### 42-1:4-O-(2-Chloroéthyl) tyrosine (S), chlorhydrate

On place dans un ballon, 5g de N-tert-butoxy carbonyl-4-O-(2-chloroéthyl) tyrosine (S) en solution dans 50ml de dioxane chlorhydrique. Après 28h d'agitation, le mélange est concentré à sec sous pression réduite. Le résidu obtenu est repris par 50ml d'éther, agité puis filtré. Le solide obtenu est lavé par 2 fois 25 ml d'éther, séché sous pression réduite pour donner 1,58g de chlorhydrate de 4-O-(2-chloroéthyl) tyrosine (S) sous forme d'un solide blanc fondant à 260°C.

### 42-2: N-tert-butoxy carbonyl-4-O-(2-chloroéthyl) tyrosine (S)

On place dans un tricol sous atmosphère d'azote, 14g de N-tert-butoxycarbonyl tyrosine (S), en solution dans 140ml de DMF. On ajoute 4,8g d'hydrure de sodium à 50% dans l'huile lentement à la spatule. Après 2h d'agitation à température ambiante, on ajoute 16,87g de 1-tosyl 2-chloroéthanol . Après 2 jours d'agitation on ajoute 2,4g d'hydrure de sodium à 50% dans l'huile et 8.4ml supplémentaires de 1-tosyl 2-chloroéthanol . La même opération est effectuée après 24h et l'agitation est poursuivie 24h supplémentaires. La réaction est arrétée par addition de 100ml d'eau distillée et le mélange réactionnel est concentré à sec sous pression réduite. Le résidu obtenu est repris par 100ml d'eau distillée puis extrait par 3 fois 100ml d'acétate d'éthyle. La phase aqueuse est décantée, acidifiée par 50ml d'HCl 1N jusqu'à pH3 et le produit est extrait par 3 fois 100ml d'acétate d'éthyle. Les phases organiques sont réunies, lavées par 2 fois 50ml d'eau, décantées , séchées sur sulfate de magnésium, filtrées puis concentrées à sec sous pression réduite pour donner 13,51g de N-tert-butoxy carbonyl-4-O-(2-chloroéthyl) tyrosine (S) sous forme d'une huile marron (Rf 0,5, toluène 70% / méthanol 20% / diéthylamine 10%) utilisée telle quelle dans l'étape suivante.

**TABLEAU V**

| **MICROORGANISMES** | **ANTIBIOTIQUES** |
|---|---|
| **CHAMPIGNONS** | |
| Micromonospora sp. | Vernamycines |
| | |

| **STREPTOMYCES** | |
|---|---|
| | |
| S. alborectus | Virginiamycines |
| S. conganensis (ATCC13528) | F1370 A, B |
| S. diastaticus | Plauracines, Streptograrnines |
| S. graminofasciens | Streptogramines |
| S. griseus (NRRL2426) | Viridogriséine (Etamycine) |
| S. griseoviridus | Griseoviridine |
| S. griseoviridus (FERMP3562) | Néoviridogriséines |
| S. lavendulae | Etamycines |
| S. loïdensis (ATCC11415) | Vernamycines |
| S. mitakaensis (ATCC15297) | Mikamycines |
| S. olivaceus (ATCC12019) | Synergistines (PA114 A, B) |
| S. ostréogriseus (ATCC27455) | Ostréogrycines |
| S. pristinaespiralis (ATCC25486) | Pristinamycines |
| S. virginiae (ATCC13161) | Virginiamycines (Staphylomycines) |
| | |

| **ACTINOMYCETES** | |
|---|---|
| | |
| A. auranticolor (ATCC31011) | Plauracines |
| A. azureus (ATCC31157) | Plauracines |
| A. daghestanicus | Etamycine |
| A. philippinensis | A-2315 A, B, C |
| Actinoplanes sp. (ATCC33002) | A15104 |
| Actinoplanes sp. | A17002 A, B, C, F |
| Actinomadura flava | Madumycines |

### Abréviations utilisées :

| | |
|---|---|
| AcOEt | acétate d'éthyle |
| ADN : | acide déoxyribonucléique |
| AMP : | adénosine 5'-monophosphate |
| CH₂Cl₂: | dichlorométhane |
| CLHP : | chromatographie liquide haute performance |
| dCTP | déoxy-cytosine 5'-triphosphate |
| DMF | diméthylformamide |
| DMPAPA | 4-diméthylamino-L-phénylalanine |
| HCl | acide chlorhydrique |
| HT7 | Hickey Tresner solid medium |
| 3-HPA | acide 3-hydroxypicolinique |
| IPTG | isopropyl β-D-thiogalactopyranoside |
| kb: | kilobase |
| LB: | Luria broth (milieu de croissance riche pour E. coli) |
| MeOH | méthanol |
| MMPAPA | 4-méthylamino-L-phénylalanine |
| NaOH | hydroxyde de sodium |
| PAPA | 4-amino-L-phénylalanine |
| PEG | Polyéthylène glycol |
| P I | Pristinamycine I |
| P II | Pristinamycine II |
| pb : | paire de base |
| SAM: | S-adénosylméthionine |
| TE : | tampon 10 mM Tris-HCl, 1 mM EDTA, pH 7,5 |
| THF | tétrahydrofuranne |
| Tris: | amino-2-hydroxylméthyl-2 propanediol-1,3 |
| U.V.: | rayons ultra-violets |
| X-gal: | 5-bromo-4-chloro-3-indoyl-β-D-galactoside |
| YEME: | yeast extract-malt extract medium (milieu de croissance riche pour Streptomyces) |

### Bibliographie:

- Bibb M. J., Findlay P. R. et Johnson M. W. (1984) Gene, 30 : 157-166.
- Bibb M. J., Janssen G. R., et Ward J. M. (1985) Gene, 38 : 215-226.
- Cocito C. G. (1979) Microbiol Rev, 43 : 145-198.
- Cocito C. G. (1983) In Antibiotics, 6 : (Ed. F. E. Hahn), 296-332.
- Dessen P. C., Fondrat C., Valencien C. et Mugnier C. (1990) Comp. Appl. in Biosciences, 6 : 355-356.
- Di Giambattista M., Chinali G. et Cocito C. G. (1989) J. Antim. Chemother., 24 : 485-507.
- Gibson TJ. (1984) Ph.D. thesis, Cambridge University, England.
- Hillemann D., Pülher A. et Wohlleben W. (1991) Nucl. Acids Res., 19 : 727-731.
- Hopwood D. A., Bibb M. J., Chater K. F., Kieser T., Bruton C. J., Kieser H. M., Lydiate D. J., Smith C. P., Ward J. M. et Scrempf H. (1985) A laboratory manual., The John Innes Fondation, Norwich, England.
- Hudson G. S. et Davidson B. E. (1984) J. Mol. Biol., 180 : 1023-1051.
- Kuhstoss S., Richardson M. A., et Rao R. N. (1991) Gene 97 : 143-146.
- Maniatis T., Fritsh E. F. et Sambrook J. (1989) Molecular cloning : a laboratory manual. Cold Spring Harbor, N. Y.,
- Messing J., Crea R. et Seeburg P. H. (1981) Nucleic Acids Res., 9 : 309.
- Molinero A. A., Kingston D. G. I., et Reed J. W. (1989) J. Nat. Prod., 52 : 99-108.
- Omer C. A., Lenstra R., Litle P. J., Dean J., Tepperman J. M., Leto K. J., Romesser J. A., et O'Keefe D. P. (1990) J. Bact. 172 : 3335-3345.
- Reed J. W., Purvis M. B., Kingston D. G. I., Biot A., et Gosselé F. (1989) J. Org. Chem. 54 : 1161-1165.
- Staden R. et McLachlan A. D. (1982) Nucleic Acids Res., 1O : 141-156.
- Schindler U., Sans N., et SchrôderJ. (1989) J. Bact. 171 : 847-854.
- Thorson J. S.; Lo S. F., et Liu H-W. (1993) J. Am. Chem. Soc. 115 : 6993-6994.
- Videau D. (1982) Path. Biol., 30 : 529-534.

## Revendications

1. Composé **caractérisé en ce qu'**il est représenté par la formule générale I 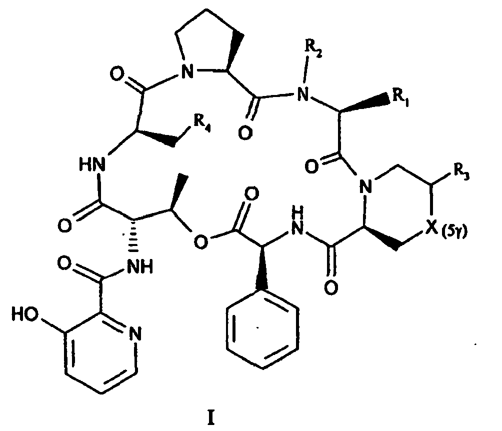 dans laquelle:
- R₂ et R₄ représentent, indépendamment l'un de l'autre, un atome d'hydrogène ou un groupement méthyle,
- R₃ représente un atome d'hydrogène ou un groupement hydroxyle,
- X représente un groupement CO, CHOH ou CH₂ et
- R₁ représente: 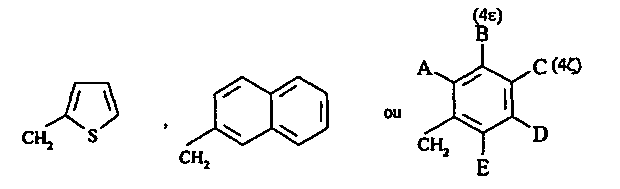
avec
- pour les dérivés méta :
A, C, D et E représentant un atome d'hydrogène et
B pouvant représenter:
- un halogène et de préférence un atome de fluor,
- un groupement monoalkylamino ou dialkylamino avec alkyle représentant de préférence un groupement méthyle ou éthyle,
- un groupement éther.
- un groupement thioéther,
- un groupement alkyle en C₁ à C₃ ou
- un groupement trihalogénométhyle et de préférence le trifluorométhyle.
- pour les dérivés para :
A, B, D et E représentant un atome d'hydrogène et
C pouvant représenter.
- un halogène,
- un groupement NR₅R₆ avec R₅ et R₆ représentant indépendamment l'un de l'autre un groupement choisi parmi
- l'hydrogène,
- un groupement alkyle en C₁ à C₄ linéaire ou ramifié avec lorsque l'un des substituants R₅ ou R₆, représente un groupement méthyle, l'autre représente obligatoirement un groupement éthyle,
- un groupement alkyl-cycloalkylméthyle avec un cycloalkyl en C₃ à C₄
- un groupement cycloalkyle en C₃ à C₄ éventuellement substitué,
- un groupement alcényle en C₁ à C₄ linéaire ou ramifié avec lorsque l'un des substituants R₅ ou R₆, représente un groupement alcényle, l'autre est différent d'un groupement méthyle ou d'un groupement cycloalkyle en C3 à C6,
- un groupement N-pyrrolidinyle substitué ou non,
- un groupement éther,
- un groupement thioéther,
- un groupement acyle ou alcoxycarbonyle,
- un groupement alkyle en C₁ à C₆, linéaire ou ramifié, et de préférence choisi parmi les groupements méthyle, isopropyle et tert-butyle,
- un groupement alkylthiométhyle,
- un groupement aryle et de préférence un phényle ou
- un groupement trihalogénométhyle et de préférence le trifluorométhyle et
- pour les dérivés disubstitués méta-para :
A, D et E représentant un atome d'hydrogène et
B pouvant représenter:
- un halogène et de préférence un atome de fluor,
- un groupement monoalkylamino ou dialkylamino avec alkyle représentant de préférence un groupement méthyle ou éthyle,
- un groupement éther,
- un groupement thioéther,
- un groupement alkyle en C₁ à C₃ et
C pouvant représenter.
- un halogène et de préférence un atome de fluor,
- un groupement amino, monoalkylamino ou dialkylamino avec alkyle représentant de préférence un groupement méthyle à la condition que B soit différent d'un atome de brome ou de chlore, ou un groupement allyle substitué ou non,
- un groupement éther,
- un groupement thioéther,
- un groupement alkyle en C₁ à C₆ ou
- un groupement trihalogénométhyle et de préférence le trifluorométhyle et
- pour les dérivés disubstitués ortho-para :
B, E et D représentant un atome d'hydrogène et A et C, un groupement méthyle.

2. Composé selon la revendication 1 **caractérisé en ce qu'**il s'agit de préférence de:
la 4ζ-méthylthio-dés(4ζ-diméthylamino) pristinamycine I_{A},
la 4ζ-méthylthio-dés(4ζ-diméthylamino) pristinamycine I_{H},
la 5 γ-hydroxy 4ζ-méthylthio-dés(4ζ-diméthylamino) pristinamycine I_{H},
la 4ζ-méthyl-dés(4ζ-diméthylamino) pristinamycine I_{A},
la 4ζ-méthyl-dés(4ζ-diméthylamino) pristinamycine I_{H},
la 4ζ-méthoxy-dés(4ζ-diméthylamino) pristinamycine I_{A},
la 4ζ-méthoxycarbonyl-dés(4ζ-diméthylamino) pristinamycine I_{A},
la 4ζ-chloro-dés(4ζ-iméthylamino) pristinamycine I_{A},
la 4ζ-bromo-dés(4ζ-diméthylamino) pristinamycine I_{A},
la 4ζ-bromo-dés(4ζ-diméthylamino) pristinamycine I_{H},
la 4ζ-iodo-dés(4ζ-diméthylamino) pristinamycine I_{A},
la 4ζ-iodo-dés(4ζ-diméthylamino) pristinamycine I_{H},
la 4ζ-trifluorométhyl-dés(4ζ-diméthylamino) pristinamycine I_{A},
la 4ζ-trifluorométhyl-dés(4ζ-diméthylamino) pristinamycine I_{H},
la 4ζ-tert-butyl-dés(4ζ-diméthylamino) pristinamycine I_{A},
la 4ζ-isopropyl-dés(4ζ-diméthylamino) pristinamycine I_{A},
la 4ζ-isopropyl-dés(4ζ-diméthylamino) pristinamycine I_{E},
la 4ε-méthylamino-dés(4ζ-diméthylamino) pristinamycine I_{A},
la 4ε-méthoxy-dés(4ζ-diméthylamino) pristinamycine I A,
la 4ε-méthoxy-dés(4ζ-diméthylamino) pristinamycine I H,
la 4ε-fluoro 4ζ-méthyl-dés(4ζ-diméthylamino) pristinamycine I A
la 4ζ-amino-dés(4ζ-diméthylamino) pristinamycine I A
la 4ζ-éthylamino-dés(4ζ-diméthylamino) pristinamycine I_{A}
la 4ζ-diéthylamino-dés(4ζ-diméthylamino) pristinamycine I_{A}
la 4ζ-allylamino-dés(4ζ-diméthylamino) pristinamycine I_{A}
la 4ζ-diallylamino-dés(4ζ-diméthylamino) pristinamycine I_{A}
la 4ζ-allyl éthylamino-dés(4ζ-diméthylamino) pristinamycine I_{A}
la 4ζ-éthyl propylamino-dés(4ζ-diméthylamino) pristinamycine I_{A}
la 4ζ-éthyl isoprcpylamino-dés(4ζ-diméthylamino) pristinamycine I_{A}
la 4ζ-éthyl méthylcyclopropylamino-dés(4ζ-diméthylamino) pristinamycine I_{A}
la 4ζ-(1-pyrrolidinyl) -dés(4ζ-diméthylamino) pristinamycine I_{A}
la 4ζ-trifluorométhoxy-dés(4ζ-diméthylamino) pristinamycine I_{A}
la 4ζ-allyloxy-dés(4ζ-diméthylamino) pristinamycine I_{A}
la 4ζ-éthoxy-dés(4ζ-diméthylamino) pristinamycine I_{A}
la 4ζ-éthylthio-dés(4ζ-diméthylamino) pristinamycine I_{A}
la 4ζ-méthylthiométhyl-dés(4ζ-diméthylamino) pristinamycine I_{A}
la 4ζ-(2-chloroéthoxy)-dés(4ζ-diraéthylamino) pristinamycine I_{A}
la 4ζ-acétyl-dés(4ζ-diméthylamino) pristinamycine I_{A}
la 4ζ-éthyl-dés(4ζ-diméthylamino) pristinamycine I_{A}
la 4ζ-éthyl-dés(4ζ-diméthylamino) pristinamycine I_{H}
la 4ε-diméthylamino-dés(4ζ-diméthylamino) pristinamycine I_{A}
la 4ε-méthylthio-dés(4ζ-diméthylamino) pristinamycine I_{A} et
la 4ε-éthoxy-dés(4ζ-diméthylamino) pristinamycine I_{A}.

3. Composition pharmaceutique **caractérisée en ce qu'**elle contient au moins un composé selon la revendication 1 ou 2 en association ou non avec une streptogramine du groupe A.

## Claims

1. Compound **characterized in that** it is represented by the general formula I 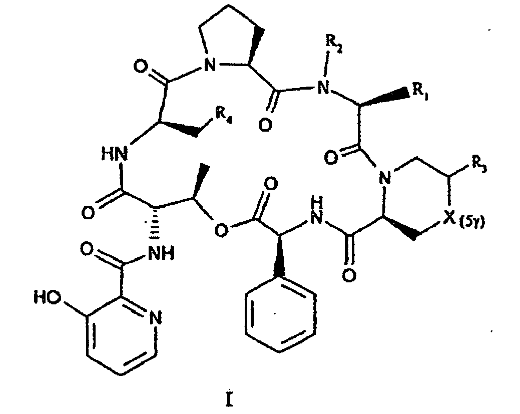 in which:
- R₂ and R₄ represent, independently of each other, a hydrogen atom or a methyl group,
- R₃ represents a hydrogen atom or a hydroxyl group,
- X represents a CO, CHOH or CH₂ group, and
- R₁ represents: 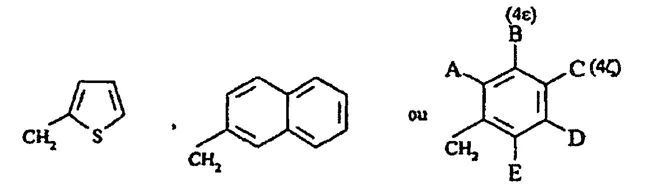
with
- for the meta derivatives:
A, C, D and E representing a hydrogen atom, and
B being able to represent:
- a halogen, and preferably a fluorine atom,
- a monoalkylamino or dialkylamino group, with alkyl preferably representing a methyl or ethyl group,
- an ether group,
- a thioether group,
- a C₁ to C₃ alkyl group, or
- a trihalogenomethyl group, preferably trifluoromethyl.
- for the para derivatives:
A, B, D and E representing a hydrogen atom, and
C being able to represent:
- a halogen,
- an NR₅R₆ group with R₅ and R₆ representing, independently of each other, a group selected from among
- hydrogen,
- a straight-chain or branched C₁ to C₄ alkyl group where, when one of the substituents R₅ or R₆ represents a methyl group, the other necessarily represents an ethyl group,
- an alkyl-cycloalkylmethyl group with a C₃ to C₄ cycloalkyl,
- an optionally substituted C₃ to C₄ cycloalkyl group,
- a straight-chain or branched C₁ to C₄ alkenyl group where, when one of the substituents R₅ or R₆ represents an alkenyl group, the other is different from a methyl group or a C3 to C6 cycloalkyl group,
- a substituted or unsubstituted N-pyrrolidinyl group,
- an ether group,
- a thioether group,
- an acyl or alkoxycarbonyl group,
- a C₁ to C₆ alkyl group which is straight-chain or branched and which is preferably selected from among the methyl, isopropyl and tert-butyl groups,
- an alkylthiomethyl group,
- an aryl group, preferably a phenyl group, or
- a trihalogenomethyl group, preferably trifluoromethyl and
- for the meta-para disubstituted derivatives:
A, D and E representing a hydrogen atom, and
B being able to represent:
- a halogen, preferably a fluorine atom,
- a monoalkylamino or dialkylamino group with alkyl preferably representing a methyl or ethyl group,
- an ether group,
- a thioether group,
- a C₁ to C₃ alkyl group, and
C being able to represent:
- a halogen, and preferably a fluorine atom,
- an amino, monoalkylamino or dialkylamino group with alkyl preferably representing a methyl group with the proviso that B is different from a bromine or chlorine atom, or a substituted or unsubstituted allyl group,
- an ether group,
- a thioether group,
- a C₁ to C₆ alkyl group, or
- a trihalogenomethyl group, preferably trifluoromethyl, and
- for the ortho-para disubstituted derivatives:
B, E and D representing a hydrogen atom and A and C a methyl group.

2. Compound according to Claim 1, **characterized in that** it is preferably:
4ζ-methylthio-de(4ζ-dimethylamino)pristinamycin I_{A},
4ζ-methylthio-de(4ζ-dimethylamino)pristinamycin I_{H},
5γ-hydroxy-4ζ-methylthio-de(4ζ-dimethylamino)pristinamycin I_{H},
4ζ-methyl-de(4ζ-dimethylamino)pristinamycin I_{A},
4ζ-methyl-de(4ζ-dimethylamino)pristinamycin I_{H},
4ζ-methoxy-de(4ζ-dimethylamino)pristinamycin I_{A},
4ζ-methoxycarbonyl-de(4ζ-dimethylamino)pristinamycin I_{A},
4ζ-chloro-de(4ζ-dimethylamino)pristinamycin I_{A},
4ζ-bromo-de(4ζ-dimethylamino)pristinamycin I_{A},
4ζ-bromo-de(4ζ-dimethylamino)pristinamycin I_{H},
4ζ-iodo-de(4ζ-dimethylamino)pristinamycin I_{A},
4ζ-iodo-de(4ζ-dimethylamino)pristinamycin I_{H},
4ζ-trifluoromethyl-de(4ζ-dimethylamino)-pristinamycin I_{A},
4ζ-trifluoromethyl-de(4ζ-dimethylamino)-pristinamycin I_{H},
4ζ-tert-butyl-de(4ζ-dimethylamino)-pristinamycin I_{A},
4ζ-isopropyl-de(4ζ-dimethylamino)-pristinamycin I_{A},
4ζ-isopropyl-de(4ζ-dimethylamino)-pristinamycin I_{E},
4ε-methylamino-de(4ζ-dimethylamino)-pristinamycin I_{A},
4ε-methoxy-de(4ζ-dimethylamino)pristinamycin IA,
4ε-methoxy-de(4ζ-dimethylamino)pristinamycin IH,
4ε-fluoro 4ζ-methyl-de(4ζ-dimethylamino)-pristinamycin IA,
4ζ-amino-de(4ζ-dimethylamino)pristinamycin IA,
4ζ-ethylamino-de(4ζ-dimethylamino)-pristinamycin I_{A},
4ζ-diethylamino-de(4ζ-dimethylamino)-pristinamycin I_{A},
4ζ-allylamino-de(4ζ-dimethylamino)-pristinamycin I_{A},
4ζ-diallylamino-de(4ζ-dimethylamino)-pristinamycin I_{A},
4ζ-allylethylamino-de(4ζ-dimethylamino)-pristinamycin I_{A},
4ζ-ethylpropylamino-de(4ζ-dimethylamino)-pristinamycin I_{A},
4ζ-ethylisopropylamino-de(4ζ-dimethylamino)-pristinamycin I_{A},
4ζ-ethylmethylcyclopropylamino-de(4ζ-dimethylamino)pristinamycin I_{A},
4ζ-(1-pyrrolidinyl)-de(4ζ-dimethylamino)-pristinamycin I_{A},
4ζ-trifluoromethoxy-de(4ζ-dimethylamino)-pristinamycin I_{A},
4ζ-allyloxy-de(4ζ-dimethylamino)pristinamycin I_{A},
4ζ-ethoxy-de(4ζ-dimethylamino)pristinamycin I_{A},
4ζ-ethylthio-de(4ζ-dimethylamino)-pristinamycin I_{A},
4ζ-methylthiomethyl-de(4ζ-dimethylamino)-pristinamycin I_{A},
4ζ-(2-chloroethoxy)-de(4ζ-dimethylamino)-pristinamycin I_{A},
4ζ-acetyl-de(4ζ-dimethylamino)pristinamycin I_{A},
4ζ-ethyl-de(4ζ-dimethylamino)pristinamycin I_{A},
4ζ-ethyl-de(4ζ-dimethylamino)pristinamycin I_{H},
4ε-dimethylamino-de(4ζ-dimethylamino)-pristinamycin I_{A},
4ε-methylthio-de(4ζ-dimethylamino)-pristinamycin I_{A}, and
4ε-ethoxy-de(4ζ-dimethylamino)pristinamycin I_{A}.

3. Pharmaceutical composition, **characterized in that** it contains at least one compound according to Claim 1 or 2 which may or may not be associated with a group A streptogramin.

## Patentansprüche

1. Verbindung, **dadurch gekennzeichnet, daß** sie durch die allgemeine Formel I 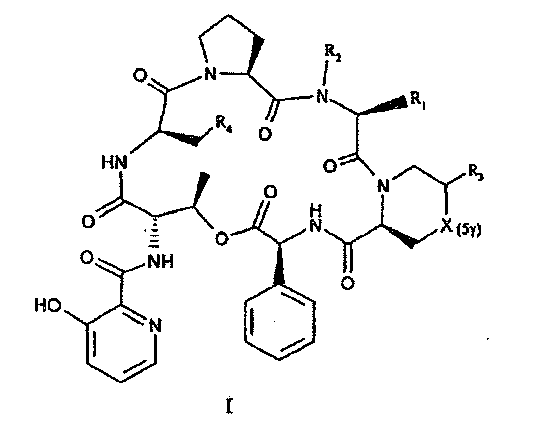 wiedergegeben wird, in der:
- R₂ und R₄ unabhängig voneinander für ein Wasserstoffatom oder eine Methylgruppe stehen,
- R₃ für ein Wasserstoffatom oder eine Hydroxylgruppe steht,
- X für eine CO-, CHOH- oder CH₂-Gruppe steht und
- R₁ für 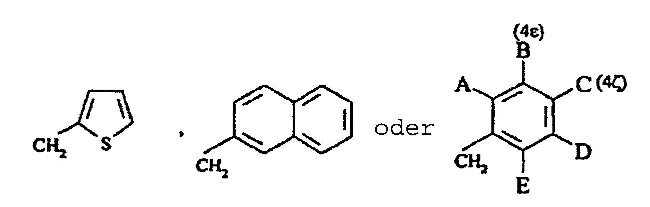
steht, wobei
- für die meta-Derivate:
A, C, D und E für ein Wasserstoffatom stehen und
B für
- ein Halogen und vorzugsweise ein Fluoratom,
- eine Monoalkylamino- oder Dialkylaminogruppe, wobei Alkyl vorzugsweise für eine Methyl- oder Ethylgruppe steht,
- eine Ethergruppe,
- eine Thioethergruppe,
- eine C₁- bis C₃-Alkylgruppe oder
- eine Trihalogenmethylgruppe und vorzugsweise Trifluormethyl
stehen kann;
- für die para-Derivate:
A, B, D und E für ein Wasserstoffatom stehen und
C für
- ein Halogen,
- eine Gruppe NR₅R₆, wobei R₅ und R₆ unabhängig voneinander für eine unter
- Wasserstoff,
- einer linearen oder verzweigten C₁- bis C₄-Alkylgruppe, wobei dann, wenn einer der Substituenten R₅ oder R₆ für eine Methylgruppe steht, die andere zwingend für eine Ethylgruppe steht,
- einer Alkylcycloalkylmethylgruppe mit einem C₃- bis C₄-Cycloalkyl,
- einer gegebenenfalls substituierten C₃- bis C₄-Cycloalkylgruppe,
- einer linearen oder verzweigten C₁- bis C₄-Alkenylgruppe, wobei dann, wenn einer der Substituenten R₅ oder R₆ für eine Alkenylgruppe steht, die andere von einer Methylgruppe oder einer C₃- bis C₆-Cycloalkylgruppe verschieden ist, ausgewählte Gruppe stehen,
- eine gegebenenfalls substituierte N-Pyrrolidinylgruppe,
- eine Ethergruppe,
- eine Thioethergruppe,
- eine Acyl- oder Alkoxycarbonylgruppe,
- eine lineare oder verzweigte C₁- bis C₆-Alkylgruppe, die vorzugsweise unter Methyl-, Isopropyl und tert.-Butylgruppen ausgewählt ist,
- eine Alkylthiomethylgruppe,
- eine Arylgruppe und vorzugsweise ein Phenyl oder
- eine Trihalogenmethylgruppe und vorzugsweise Trifluormethyl
stehen kann; und
- für die meta-para-disubstituierten Derivate:
A, D und E für ein Wasserstoffatom stehen und
B für
- ein Halogen und vorzugsweise ein Fluoratom,
- eine Monoalkylamino- oder Dialkylaminogruppe, wobei Alkyl vorzugsweise für eine Methyl- oder Ethylgruppe steht,
- eine Ethergruppe,
- eine Thioethergruppe,
- eine C₁- bis C₃-Alkylgruppe
stehen kann und
C für
- ein Halogen und vorzugsweise ein Fluoratom,
- eine Amino-, Monoalkylamino- oder Dialkylaminogruppe, wobei Alkyl vorzugsweise für eine Methylgruppe steht, mit der Maßgabe, daß B von einem Brom- oder Chloratom oder einer gegebenenfalls substituierten Allylgruppe verschieden ist,
- eine Ethergruppe,
- eine Thioethergruppe,
- eine C₁- bis C₆-Alkylgruppe oder
- eine Trihalogenmethylgruppe und vorzugsweise Trifluormethyl
stehen kann; und
- für die ortho-para-disubstituierten Derivate:
B, E und D für ein Wasserstoffatom stehen und A und C für eine Methylgruppe stehen.

2. Verbindung nach Anspruch 1, **dadurch gekennzeichnet, daß** es sich dabei vorzugsweise um
4ζ-Methylthio-des(4ζ-dimethylamino)pristinamycin I_{A},
4ζ-Methylthio-des(4ζ-dimethylamino)pristinamycin I_{H},
5γ-Hydroxy-4ζ-methylthio-des(4ζ-dimethylamino)-pristinamycin I_{H},
4ζ-Methyl-des(4ζ-dimethylamino)pristinamycin I_{A},
4ζ-Methyl-des(4ζ-dimethylamino)pristinamycin I_{H},
4ζ-Methoxy-des(4ζ-dimethylamino)pristinamycin I_{A},
4ζ-Methoxycarbonyl-des(4ζ-dimethylamino)-pristinamycin I_{A},
4ζ-Chlor-des(4ζ-dimethylamino)pristinamycin I_{A},
4ζ-Brom-des(4ζ-dimethylamino)pristinamycin I_{A},
4ζ-Brom-des(4ζ-dimethylamino)pristinamycin I_{H},
4ζ-Iod-des(4ζ-dimethylamino)pristinamycin I_{A},
4ζ-Iod-des(4ζ-dimethylamino)pristinamycin I_{H},
4ζ-Trifluormethyl-des(4ζ-dimethylamino)-pristinamycin I_{A},
4ζ-Trifluormethyl-des(Aζ-dimethylamino)-pristinamycin I_{H},
4ζ-tert.-Butyl-des(4ζ-dimethylamino)-pristinamycin I_{A},
4ζ-Isopropyl-des(4ζ-dimethylamino)-pristinamycin I_{A},
4ζ-Isopropyl-des(4ζ-dimethylamino)-pristinamycin I_{E},
4ε-Methylamino-des(4ζ-dimethylamino)-pristinamycin I_{A},
4ε-Methoxy-des(4ζ-dimethylamino)pristinamycin IA,
4ε-Methoxy-des(4ζ-dimethylamino)pristinamycin IH,
4ε-Fluor-4ζ-methyl-des(4ζ-dimethylamino)-pristinamycin IA,
4ζ-Amino-des(4ζ-dimethylamino)pristinamycin IA,
4ζ-Ethylamino-des(4ζ-dimethylamino)-pristinamycin I_{A},
4ζ-Diethylamino-des(4ζ-dimethylamino)-pristinamycin I_{A},
4ζ-Allylamino-des(4ζ-dimethylamino)-pristinamycin I_{A},
4ζ-Diallylamino-des(4ζ-dimethylamino)-pristinamycin I_{A},
4ζ-Allylethylamino-des(4ζ-dimethylamino)-pristinamycin I_{A},
4ζ-Ethylpropylamino-des(4ζ-dimethylamino)-pristinamycin I_{A},
4ζ-Ethylisopropylamino-des(4ζ-dimethylamino)-pristinamycin I_{A},
4ζ-Ethylmethylcyclopropylamino-des(4ζ-dimethylamino)pristinamycin I_{A},
4ζ-(1-Pyrrolidinyl)-des(4ζ-dimethylamino)-pristinamycin I_{A},
4ζ-Trifluormethoxy-des(4ζ-dimethylamino)-pristinamycin I_{A},
4ζ-Allyloxy-des(4ζ-dimethylamino)pristinamycin I_{A},
4ζ-Ethoxy-des(4ζ-dimethylamino)pristinamycin I_{A},
4ζ-Ethylthio-des(4ζ-dimethylamino)-pristinamycin I_{A},
4ζ-Methylthiomethyl-des(4ζ-dimethylamino)-pristinamycin I_{A},
4ζ-(2-Chlorethoxy)-des(4ζ-dimethylamino)-pristinamycin I_{A},
4ζ-Acetyl-des(4ζ-dimethylamino)pristinamycin I_{A},
4ζ-Ethyl-des(4ζ-dimethylamino)pristinamycin I_{A},
4ζ-Ethyl-des(4ζ-dimethylamino)pristinamycin I_{H},
4ε-Dimethylamino-des(4ζ-dimethylamino)-pristinamycin I_{A},
4ε-Methylthio-des(4ζ-dimethylamino)pristinamycin I_{A} und
4ε-Ethoxy-des(4ζ-dimethylamino)pristinamycin I_{A} handelt.

3. Pharmazeutische Zusammensetzung, **dadurch gekennzeichnet, daß** sie mindestens eine Verbindung nach Anspruch 1 oder 2, gegebenenfalls in Kombination mit einem Gruppe-A-Streptogramin, enthält.


### LISTE DE SEOUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION : NOUVELLES STREPTOGRAMINES ET PROCEDE DE PREPARATION DE STREPTOGRAMINES PAR MUTASYNTHESE.
   (iii) NOMBRE DE SEQUENCES: 8
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 2888 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 1:
(3) INFORMATION POUR LA SEQ ID NO: 2:
   (i) CARACTERISTIQUES DE LA SEQUENCE:
      (A) LONGUEUR: 888 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 2
   ATG AGG GGT GGT TCG GTG TTC GGG CGT TGT GTG GTG GTG GGC GGG GCC GGT GCG     54
   Met Arg Gly Gly Ser Val Phe Gly Arg Cys Val Val Val Gly Gly Ala Gly Ala     18
   GTG GGC CGC ATG TTC AGC CAC TGG CTG GTG CGT TCG GGG GTG GCG GTG ACC TGG    108
   Val Gly Arg Met Phe Ser His Trp Leu Val Arg Ser Gly Val Ala Val Thr Trp     36
   CTG GAC GTG GCC GGG GCC GGT GCG GCG GAC GGG GTG CGG GTG GTG GCC GGT GAT    162
   Leu Asp Val Ala Gly Ala Gly Ala Ala Asp Gly Val Arg Val Val Ala Gly Asp     54
   GTG CGG CGG CCG GGG CCG GAG GCG GTC GCG GCG CTG GCG GCG GCG GAC GTG GTG    216
   Val Arg Arg Pro Gly Pro Glu Ala Val Ala Ala Leu Ala Ala Ala Asp Val Val     72
   GTG CTG GCG GTG CCG GAG CCG GTG GCG TGG GAG GCG GTG GAG GTG CTG GCG GGG    270
   Val Leu Ala Val Pro Glu Pro Val Ala Trp Glu Ala Val Glu Val Leu Ala Gly     90
   GTG ATG CGG CCC GGT GCG GTG CTC GCG GAC ACC TTG TCG GTC AAG AGC CGG ATC    324
   Val Met Arg Pro Gly Ala Val Leu Ala Asp Thr Leu Ser Val Lys Ser Arg Ile    108
   GCC GGG CGG CTG CGT GAG GCG GCG CCG GGG CTG CAG GCG GTG GGG CTG AAC CCG    378
   Ala Gly Arg Leu Arg Glu Ala Ala Pro Gly Leu Gln Ala Val Gly Leu Asn Pro    126
   ATG TTC GCC CCC TCG CTG GGT CTT CAG GGG CGG CCG GTG GCG GCG GTG GTG GTC    432
   Met Phe Ala Pro Ser Leu Gly Leu Gln Gly Arg Pro Val Ala Ala Val Val Val    144
   ACC GAC GGG CCC GGT GTG CGG GCC CTG GTG GAG CTG GTG GCC GGG TGG GGG GCC    486
   Thr Asp Gly Pro Gly Val Arg Ala Leu Val Glu Leu Val Ala Gly Trp Gly Ala    162
   CGG GTG GTG GAG ATG CCG GCG CGG CGG CAC GAC GAG CTG ACC GCC GCG CAG CAG    540
   Arg Val Val Glu Met Pro Ala Arg Arg His Asp Glu Leu Thr Ala Ala Gln Gln    180
   GCC GCC ACG CAT GCC GCG GTG CTG GCC TTC GGG CTG GGC CTG GGT GAG CTG TCG    594
   Ala Ala Thr His Ala Ala Val Leu Ala Phe Gly Leu Gly Leu Gly Glu Leu Ser    198
   GTG GAC GTG GGG GCG CTG CGG GAC AGT GCC CCG CCG CCG CAT CTG GCG ATG CTG    648
   Val Asp Val Gly Ala Leu Arg Asp Ser Ala Pro Pro Pro His Leu Ala Met Leu    216
   GCG CTG CTG GCC CGG ATC GCC GGC GGG ACG CCG GAG GTG TAT TTC GAC ATC CAG    702
   Ala Leu Leu Ala Arg Ile Ala Gly Gly Thr Pro Glu Val Tyr Phe Asp Ile Gln    234
   GCC GCC AAC CCC GGC GCG CCG GCC GCG CGG CAG GCG CTG GGC CGC GGC CTG GTG    756
   Ala Ala Asn Pro Gly Ala Pro Ala Ala Arg Gln Ala Leu Gly Arg Gly Leu Val    252
   CGG CTG GGG CAG GCC GTC GAG AGG GGC GAC GAG GAG ACG TTC GCC GCC CTG TTC    810
   Arg Leu Gly Gln Ala Val Glu Arg Gly Asp Glu Glu Thr Phe Ala Ala Leu Phe    270
   GCC GAA CTG CGC GGT GTG CTG GGC GAG CAC GGC GCG GAG CTG GAA CGG CTG TGC    864
   Ala Glu Leu Arg Gly Val Leu Gly Glu His Gly Ala Glu Leu Glu Arg Leu Cys    288
   GCG CGG ATG TTC ACC GCC CTG CAC                                            888
   Ala Arg Met Phe Thr Ala Leu His                                            296
(4) INFORMATION POUR LA SEQ ID NO: 3:
   (i) CARACTERISTIQUES DE LA SEQUENCE:
      (A) LONGUEUR: 387 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 3
   ATG ACC CCG CCC GCC ATC CCC GCC GCC CCG CCC GCC ACC GGG CCC GCC CCC GCC     54
   Met Thr Pro Pro Ala Ile Pro Ala Ala Pro Pro Ala Thr Gly Pro Ala Ala Ala     18
   ACC GAC CCC CTC GAC GCG CTG CGC GCC CGC CTG GAC GCC GCG GAC GCC GCC CTG    108
   Thr Asp Pro Leu Asp Ala Leu Arg Ala Arg Leu Asp Ala Ala Asp Ala Ala Leu     36
   CTG GAC GCC GTC CGC ACA CGC CTG GAC ATC TGC CTG CGC ATC GGC GAG TAC AAG    162
   Leu Asp Ala Val Arg Thr Arg Leu Asp Ile Cys Leu Arg Ile Gly Glu Tyr Lys     54
   CGC CTC CAC CAG GTG CCG ATG ATG CAG CCC CAC CGG ATC GCC CAG GTC CAC GCC    216
   Arg Leu His Gln Val Pro Met Met Gln Pro His Arg Ile Ala Gln Val His Ala     72
   AAC GCC GCC CGC TAC GCC GCC GAC CAC GGC ATC GAC CCC GCC TTC CTG CGC ACC   270
   Asn Ala Ala Arg Tyr Ala Ala Asp His Gly Ile Asp Pro Ala Phe Leu Arg Thr    90
   CTG TAC GAC ACG ATC ATC ACC GAG ACC TGC CGC CTC GAG GAC GAG TGG ATC GCC   324
   Leu Tyr Asp Thr Ile Ile Thr Glu Thr Cys Arg Leu Glu Asp Glu Trp Ile Ala   108
   TCC GGC GGC GCC CCC GTC CCC ACG CCC GTG CAC GCG TCC GCG TCC GCG CGG GGG   378
   Ser Gly Gly Ala Pro Val Pro Thr Pro Val His Ala Ser Ala Ser Ala Arg Gly   126
   GCC GTG TCG                                                               387
   Ala Val Ser                                                               129
(5) INFORMATION POUR LA SEQ ID NO: 4:
   (i) CARACTERISTIQUES DE LA SEQUENCE:
      (A) LONGUEUR: 4496 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 4
(6) INFORMATION POUR LA SEQ ID NO: 5:
   (i) CARACTERISTIQUES DE LA SEQUENCE:
      (A) LONGUEUR: 1065 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 5
   ATG GAG ACC TGG GTC CTG GGC CGG CGC GAC GTC GCC GAG GTG GTG GCC GCC GTC 54
   Met Glu Thr Trp Val Leu Gly Arg Arg Asp Val Ala Glu Val Val Ala Ala Val 18
   GGC CGC GAC GAA CTC ATG CGC CGC ATC ATC GAC CGC CTC ACC GGC GGA CTG GCC 108
   Gly Arg Asp Glu Leu Met Arg Arg Ile Ile Asp Arg Leu Thr Gly Gly Leu Ala 36
   GAG ATC GGC CGC GGC GAG CGG CAC CTG TCC CCG CTG CGC GGC GGA CTG GAA CGC 162
   Glu Ile Gly Arg Gly Glu Arg His Leu Ser Pro Leu Arg Gly Gly Leu Glu Arg 54
   AGC GAA CCC GTG CCC GGC ATC TGG GAA TGG ATG CCG CAC CGC GAA CCC GGC GAC 216
   Ser Glu Pro Val Pro Gly Ile Trp Glu Trp Met Pro His Arg Glu Pro Gly Asp 72
   CAC ATC ACC CTC AAG ACC GTC GGC TAC AGC CCC GCC AAC CCC GGC CGC TTC GGC 270
   His Ile Thr Leu Lys Thr Val Gly Tyr Ser Pro Ala Asn Pro Gly Arg Phe Gly 90
   CTG CCG ACC ATC CTG GGC ACC GTC GCC CGC TAC GAC GAC ACC ACC GGC GCC CTG 324
   Leu Pro Thr Ile Leu Gly Thr Val Ala Arg Tyr Asp Asp Thr Thr Gly Ala Leu 108
   ACC GCC CTG ATG GAC GGC GTG CTG CTC ACC GCC CTG CGC ACC GGC GCC GCC TCC 378
   Thr Ala Leu Met Asp Gly Val Leu Leu Thr Ala Leu Arg Thr Gly Ala Ala Ser 126
   GCC GTC GCC TCC CGC CTG CTG GCC CGC CCC GAC AGC CAC ACC CTG GGA CTG ATC 432
   Ala Val Ala Ser Arg Leu Leu Ala Arg Pro Asp Ser His Thr Leu Gly Leu Ile 144
   GGC ACC GGC GCC CAG GCC GTC ACC CAA CTG CAC GCC CTG TCC CTG GTA CTG CCC 486
   Gly Thr Gly Ala Gln Ala Val Thr Gln Leu His Ala Leu Ser Leu Val Leu Pro 162
   CTG CAA CGG GCC CTG GTG TGG GAC ACC GAC CCC GCC CAC CGG GAA AGC TTC GCC    540
   Leu Gln Arg Ala Leu Val Trp Asp Thr Asp Pro Ala His Arg Glu Ser Phe Ala   180
   CGG CGC GCC GCG TTC ACC GGC GTC AGC GTC GAG ATC GCC GAG CCC GCC CGG ATC    594
   Arg Arg Ala Ala Phe Thr Gly Val Ser Val Glu Ile Ala Glu Pro Ala Arg Ile   198
   GCC GCC GAG GCC GAC GTC ATC TCC ACC GCC ACC TCG GTA GCC GTC GGC CAG GGC    648
   Ala Ala Glu Ala Asp Val Ile Ser Thr Ala Thr Ser Val Ala Val Gly Gln Gly   216
   CCG GTC CTG CCC GAC ACC GGC GTC CGC GAG CAC CTG CAC ATC AAC GCC GTC GGC    702
   Pro Val Leu Pro Asp Thr Gly Val Arg Glu His Leu His Ile Asn Ala Val Gly   234
   GCG GAC CTC GTC GGC AAG ACG GAA CTG CCG CTC GGC CTG CTC GAG CGG GCG TTC    756
   Ala Asp Leu Val Gly Lys Thr Glu Leu Pro Leu Gly Leu Leu Glu Arg Ala Phe   252
   GTC ACC GCC GAC CAC CCC GAG CAG GCG CTG CGC GAG GGC GAG TGC CAG CAA CTC    810
   Val Thr Ala Asp His Pro Glu Gln Ala Leu Arg Glu Gly Glu Cys Gln Gln Leu   270
   TCC GCC GAC CGG CTC GGC CCG CAG CTG GCC CAC CTG TGC GCC GAC CCG GCG GCC    864
   Ser Ala Asp Arg Leu Gly Pro Gln Leu Ala His Leu Cys Ala Asp Pro Ala Ala   288
   GCC GCC GGC CGG CAG GAC ACC CTG AGC GTC TTC GAC TCC ACC GGC TTC GCC TTC    918
   Ala Ala Gly Arg Gln Asp Thr Leu Ser Val Phe Asp Ser Thr Gly Phe Ala Phe   306
   GAG GAC GCC CTG GCG ATG GAA GTG TTC CTC GAG GCC GCC GCC GAA CGG GAC CTG    972
   Glu Asp Ala Leu Ala Met Glu Val Phe Leu Glu Ala Ala Ala Glu Arg Asp Leu   324
   GGC ATC CGG GTG GGC ATC GAA CAC CAC CCC GGC GAC GCC CTG GAC CCC TAC GCC   1026
   Gly Ile Arg Val Gly Ile Glu His His Pro Gly Asp Ala Leu Asp Pro Tyr Ala   342
   CTC CAG CCC CTG CCC CTG CCC CTG GCC GCC CCC GCC CAC                       1065
   Leu Gln Pro Leu Pro Leu Pro Leu Ala Ala Pro Ala His                        355
(7) INFORMATION POUR LA SEQ ID NO: 6:
   (i) CARACTERISTIQUES DE LA SEQUENCE:
      (A) LONGUEUR: 1194 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 6
   ATG CCC CCC ACC CCC CGG CCC ACC ACC GAC GAC GGC GGC CGT GAA CTG CTC GCC 54
   Met Pro Pro Thr Pro Arg Pro Thr Thr Asp Asp Gly Gly Arg Glu Leu Leu Ala 18
   TGG CTG CGC GAG ATG CGC CAC CAC CAC CCC GTC CAC GAG GAC GAA TAC GGT GCC 108
   Trp Leu Arg Glu Met Arg His His His Pro Val His Glu Asp Glu Tyr Gly Ala 36
   TTC CAC GTC TTC CGG CAC GCC GAC GTC CTC ACC GTC GCC TCC GAC CCC GGC GTC 162
   Phe His Val Phe Arg His Ala Asp Val Leu Thr Val Ala Ser Asp Pro Gly Val 54
   TAC TCC TCC CAG CTC AGC CGG CTA CGG CCC GGC TCC CAG GCG TTG AGC GAA CAG 216
   Tyr Ser Ser Gln Leu Ser Arg Leu Arg Pro Gly Ser Gln Ala Leu Ser Glu Gln 72
   ATC CTG TCG GTC ATC GAC CCG CCG ATG CAC CGC ACC CTG CGC CGC CTG GTC AGC 270
   Ile Leu Ser Val Ile Asp Pro Pro Met His Arg Thr Leu Arg Arg Leu Val Ser 90
   CAG GCC TTC ACC CCC CGC ACC GTC GCC GAC CTC GAA CCA CGC GTC ACC GAA CTG 324
   Gln Ala Phe Thr Pro Arg Thr Val Ala Asp Leu Glu Pro Arg Val Thr Glu Leu 108
   GCC GGG CAA CTG CTC GAC GCC GTC GAC GGC GAC ACG TTC GAC CTC GTC GCC GAC 378
   Ala Gly Gln Leu Leu Asp Ala Val Asp Gly Asp Thr Phe Asp Leu Val Ala Asp 126
   TTC GCC TAC CCG CTG CCC GTG ATC GTG ATC GCC GAA CTC CTC GGC GTG CCG CCC 432
   Phe Ala Tyr Pro Leu Pro Val Ile Val Ile Ala Glu Leu Leu Gly Val Pro Pro 144
   GCC GAC CGC ACC CTG TTC CGC TCC TGG TCC GAC CGG ATG CTG CAG ATG CAG GTC 486
   Ala Asp Arg Thr Leu Phe Arg Ser Trp Ser Asp Arg Met Leu Gln Met Gln Val 162
   GCC GAC CCG GCG GAC ATG CAG TTC GGC GAC GAC GCC GAC GAG GAC TAC CAA CGC 540
   Ala Asp Pro Ala Asp Met Gln Phe Gly Asp Asp Ala Asp Glu Asp Tyr Gln Arg 180
   CTC GTC AAA GAA CCC ATG CGC GCC ATG CAC GCC TAC CTC CAC GAC CAC GTC ACC 594
   Leu Val Lys Glu Pro Met Arg Ala Met His Ala Tyr Leu His Asp His Val Thr 198
   GAC CGC CGC GCC CGC CCC GCG AAC GAC CTG ATC TCC GCA CTC GTC GCC GCC CGC 648
   Asp Arg Arg Ala Arg Pro Ala Asn Asp Leu Ile Ser Ala Leu Val Ala Ala Arg 216
   GTG GAG GGC GAA CGA CTC ACC GAC GAG CAG ATC GTC GAA TTC GGG GCG CTG CTG 702
   Val Glu Gly Glu Arg Leu Thr Asp Glu Gln Ile Val Glu Phe Gly Ala Leu Leu 234
   CTG ATG GCC GGC CAC GTC TCC ACC TCC ATG CTG CTC GGC AAC ACC GTG CTG TGC 756
   Leu Met Ala Gly His Val Ser Thr Ser Met Leu Leu Gly Asn Thr Val Leu Cys 252
   CTG AAG GAC CAC CCC CGG GCC GAG GCC GCC GCC CGC GCC GAC CGG TCC CTG ATC 810
   Leu Lys Asp His Pro Arg Ala Glu Ala Ala Ala Arg Ala Asp Arg Ser Leu Ile 270
   CCC GCC CTG ATC GAA GAA GTA CTG CGG CTG CGG CCG CCG ATC ACC GTC ATG GCC 864
   Pro Ala Leu Ile Glu Glu Val Leu Arg Leu Arg Pro Pro Ile Thr Val Met Ala 288
   CGC GTC ACC ACC AAG GAC ACC GTC CTC GCC GGC ACC ACC ATC CCC GCC GGA CGC    918
   Arg Val Thr Thr Lys Asp Thr Val Leu Ala Gly Thr Thr Ile Pro Ala Gly Arg    306
   ATG GTC GTG CCC TCC CTG CTG TCC GCC AAC CAC GAC GAA CAG GTC TTC ACC GAC    972
   Met Val Val Pro Ser Leu Leu Ser Ala Asn His Asp Glu Gln Val Phe Thr Asp    324
   CCC GAC CAC CTC GAC CTC GCC CGC GAA GGC CGC CAG ATC GCC TTC GGC CAC GGC   1026
   Pro Asp His Leu Asp Leu Ala Arg Glu Gly Arg Gln Ile Ala Phe Gly His Gly    342
   ATC CAC TAC TGC CTG GGC GCC CCG CTC GCC CGC CTG GAG GGC CGC ATC GCC CTG   1080
   Ile His Tyr Cys Leu Gly Ala Pro Leu Ala Arg Leu Glu Gly Arg Ile Ala Leu    360
   GAA GCC CTC TTC GAC CGA TTC CCC GAC TTC TCG CCC ACC GAC GGC GCA AAA CTG   1134
   Glu Ala Leu Phe Asp Arg Phe Pro Asp Phe Ser Pro Thr Asp Gly Ala Lys Leu    378
   CGC TAC CAC CGC GAC GGA CTG TTC GGC GTC AAG AAC CTG CCG CTG ACC GTA CGG   1188
   Arg Tyr His Arg Asp Gly Leu Phe Gly Val Lys Asn Leu Pro Leu Thr Val Arg    396
   CGC GGC                                                                   1194
   Arg Gly                                                                    398
(8) INFORMATION POUR LA SEQ ID NO: 7:
   (i) CARACTERISTIQUES DE LA SEQUENCE:
      (A) LONGUEUR: 1561 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 7
(9) INFORMATION POUR LA SEQ ID NO: 8:
   (i) CARACTERISTIQUES DE LA SEQUENCE:
      (A) LONGUEUR: 1233 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Streptomyces pristinaespiralis
   (xi) DESCRIPTION DE LA SEQUENCE: SEQ ID NO: 8
   GTG CCG TTC GCG CGA CGC GGT TCG GTG GTG GGC GAG GCG GAC CTG GCG GCG CTG     54
   Val Pro Phe Ala Arg Arg Gly Ser Val Val Gly Glu Ala Asp Leu Ala Ala Leu     18
   GGC GAA CTG GTC CGC TCG GGC CGG TCG CTG ACG TCG GGG GTG TGG CGG GAG CGG    108
   Gly Glu Leu Val Arg Ser Gly Arg Ser Leu Thr Ser Gly Val Trp Arg Glu Arg     36
   TTC GAG GAA CAG TTC GCC CGC CTG ACC GGC GCC CGG CAC GCG CTC AGT GTC ACC    162
   Phe Glu Glu Gln Phe Ala Arg Leu Thr Gly Ala Arg His Ala Leu Ser Val Thr     54
   AGC GGC ACC GTC GCG CTG GAA CTG GCG GTG CGG ATG CTG GAC CTG GCG CCG GGC    216
   Ser Gly Thr Val Ala Leu Glu Leu Ala Val Arg Met Leu Asp Leu Ala Pro Gly     72
   GAC GAG GTG ATC GCC ACC CCG CAG ACG TTC CAG GCG ACG GTG CAG CCG CTG CTC    270
   Asp Glu Val Ile Ala Thr Pro Gln Thr Phe Gln Ala Thr Val Gln Pro Leu Leu     90
   GAC CAC GAC GTG CGG CTG CGG TTC TGC GAC ATC GAC CCG GAC ACC CTC AAC CTC    324
   Asp His Asp Val Arg Leu Arg Phe Cys Asp Ile Asp Pro Asp Thr Leu Asn Leu    108
   GAC CCG GCG GTG CTG GAG ACG CTG ATC ACC GAC CGC ACC CGG GCG ATC CTG CTC    378
   Asp Pro Ala Val Leu Glu Thr Leu Ile Thr Asp Arg Thr Arg Ala Ile Leu Leu    126
   GTC CAC TAC GGC GGC AAC CCG GCC GAC ATG GAC CGC ATC ATG GCC CTG GCC CGC    432
   Val His Tyr Gly Gly Asn Pro Ala Asp Met Asp Arg Ile Met Ala Leu Ala Arg    144
   AAG CGC GGC ATC ATC GTC GTC GAG GAC AGC GCG CAC GCG CTG GGC GCC GTG TAC    486
   Lys Arg Gly Ile Ile Val Val Glu Asp Ser Ala His Ala Leu Gly Ala Val Tyr    162
   CGG GGG CGG CGG CCG GGG GCA CTG GCG GAC ATC GGC TGC TTC ACT TTC CAC TCC    540
   Arg Gly Arg Arg Pro Gly Ala Leu Ala Asp Ile Gly Cys Phe Thr Phe His Ser    180
   ACG AAG AAC ATC ACC ACC CTC GGC GAG GGC GGC ATG ATC ACC CTG TCG CGT GAC    594
   Thr Lys Asn Ile Thr Thr Leu Gly Glu Gly Gly Met Ile Thr Leu Ser Arg Asp    198
   GAG TGG GCC CAG CGG GTG GGA CGT ATC CGC GAC AAC GAG GCC GAC GGC GTG TAC    648
   Glu Trp Ala Gln Arg Val Gly Arg Ile Arg Asp Asn Glu Ala Asp Gly Val Tyr    216
   GCG GCG CTG CCG GAC TCC GCG CGG GCG GGT GCT CCG GCG CTG CTG CCG TGG ATG    702
   Ala Ala Leu Pro Asp Ser Ala Arg Ala Gly Ala Pro Ala Leu Leu Pro Trp Met    234
   AAG TTC GCG GAG GGT GTG TAC GGT CAC CGG GCG GTC GGG GTC CGC GGG GCG GGC    756
   Lys Phe Ala Glu Gly Val Tyr Gly His Arg Ala Val Gly Val Arg Gly Ala Gly    252
   ACG AAC GCG ACG ATG TCG GAG GCG GCG GCG GCG GTG GGC GTG GTG CAA CTG GCG    810
   Thr Asn Ala Thr Met Ser Glu Ala Ala Ala Ala Val Gly Val Val Gln Leu Ala    270
   TCG CTG GAG CGG TTC GTG GCC CGG CGC CGG AGC ATC GCG CAG CGG CTG GAC GAG    864
   Ser Leu Glu Arg Phe Val Ala Arg Arg Arg Ser Ile Ala Gln Arg Leu Asp Glu    288
   GCC GTG GCC TCG GTG GCC GGC ACC CGG CTG CAC CGG GCG GCG GCG GAC AGT CTG    918
   Ala Val Ala Ser Val Ala Gly Thr Arg Leu His Arg Ala Ala Ala Asp Ser Leu    306
   CAC GCC TAC CAC CTG TAC ACG TTC TTC CTC ACC GGC GGC CGG CAG GTG CGG GAG    972
   His Ala Tyr His Leu Tyr Thr Phe Phe Leu Thr Gly Gly Arg Gln Val Arg Glu    324
   CGG TTC GTG CGC GCC CTG GAC CGG CTG GGT GTG GAG GTC CAG TTG CGG TAC TTC   1026
   Arg Phe Val Arg Ala Leu Asp Arg Leu Gly Val Glu Val Gln Leu Arg Tyr Phe    342
   CCG CTC CAT CTG TCG CCC GAG TGG CGG CTG CGC GGC CAC GGG CCG GGC GAG TGT   1080
   Pro Leu His Leu Ser Pro Glu Trp Arg Leu Arg Gly His Gly Pro Gly Glu Cys    360
   CCG ACG GCC GAA CGG GTC TGG TTC GAG GAG CAC ATG AAC CTG CCG TGC CAT CCC   1134
   Pro Thr Ala Glu Arg Val Trp Phe Glu Glu His Met Asn Leu Pro Cys His Pro    378
   GGT CTG AGT GAC GGC CAG GTC GAC TAC ATG GTC GAG GCG GTC ACC CGC GCC CTG   1188
   Gly Leu Ser Asp Gly Gln Val Asp Tyr Met Val Glu Ala Val Thr Arg Ala Leu    396
   CAC GAG GCC CAC GGC ACG GGG ACG CGG GTG GCG GCC GGG CAC CTG               1233
   His Glu Ala His Gly Thr Gly Thr Arg Val Ala Ala Gly His Leu                411